# EUROPEAN PATENT SPECIFICATION

(11) **EP 2 935 191 B1**
(45) Date of publication and mention of the grant of the patent: **01.08.2018**
(21) Application number: 13811206.5
(22) Date of filing: 18.12.2013
(51) Int. Cl.: C07C 45/82, C07C 45/62, C07C 45/67, C07C 45/74, C07C 29/42, C07C 29/17, C07C 29/40, C07C 45/51, C07C 45/48, C07C 43/313, C07D 311/72, C07D 319/06

(54) **PREPARATION OF (6R,10R)-6,10,14-TRIMETYLPENTADECAN-2-ONE FROM 3,7-DIMETYLOCT-2-ENAL OR 3,7-DIMETYLOCTA-2,6-DIENAL**
HERSTELLUNG VON (6R,10R)-6,10,14-TRIMETHYLPENTADECAN-2-ON AUS 3,7-DIMETYLOCT-2-ENAL ODER 3,7-DIMETYLOCTA-2,6-DIENAL
PRÉPARATION DE (6R,10R)-6,10,14-TRIMÉTYLPENTADÉCAN-2-ONE À PARTIR DE 6,10,3,7-TRIMÉTYLPENTADÉCA-5,9,13-TRIÉN-2-ONE OU DE 3,7-DIMÉTYLOCTA-5,9-DIÉN-2-AL

(30) Priority: 18.12.2012 EP 12197819
(43) Date of publication of application: 28.10.2015
(73) Proprietor: DSM IP Assets B.V., 6411 TE Heerlen (NL)
(72) Inventor: BONRATH, Werner, 4303 Kaiseraugst (CH); NETSCHER, Thomas, 4303 Kaiseraugst (CH); MEDLOCK, Jonathan Alan, 4303 Kaiseraugst (CH); STEMMLER, René Tobias, 4303 Kaiseraugst (CH); VERZIJL, Gerardus Karel Maria, 4303 Kaiseraugst (CH); VRIES, DE, Andreas Hendrikus Maria, 4303 Kaiseraugst (CH); SORIANO, Domenico, 4303 Kaiseraugst (CH)
(74) Representative: Dux, Roland
(86) International application number: PCT/EP2013/077184
(87) International publication number: WO 2014/096063

(56) References cited:
- US-A- 4 292 459
- TAKANO ET AL.: "An Enantiocontrolled Synthesis of Phytol by Reiterative Application of the Chiral 3-Hydroxyalkyne Formation Reaction", SYNLETT, no. 4, 1991, pages 279-282, XP002695186,
- MIYAKOSHI ET AL: "A convenient synthesis of isophytol by cross-coupling reaction of a Grignard reagent and a protected allylic chloride", ORGANIC PREPARATIONS AND PROCEDURES INTERNATIONAL, TAYLOR & FRANCIS, GB, vol. 24, no. 2, 1 January 1992 (1992-01-01), pages 135-141, XP008161078, ISSN: 1945-5453
- A. VASIL'EV ET AL.: "Novel Preparation of alpha,beta-Unsaturated Aldehydes. Benzeneselenolate Promotes Elimination of HBr from alpha-Bromoacetals", J. ORG. CHEM., vol. 65, 2000, pages 2151-2162, XP002695218,

## Description

The present invention relates to a process for the preparation of (6R,10R)-6,10,14-trimethylpentadecan-2-one and certain intermediates thereof.

### Background of the invention

(6R,10R)-6,10,14-Trimethylpentadecan-2-one is an important intermediate, particularly for the synthesis of (R,R)-isophytol [= (3RS,7R,11R)-3,7,11,15-tetramethylhexadec-1-en-3-ol], (R,R)-phytol and tocopherols.

Isophytol, phytol and tocopherols are chiral substances, the latter two of which occur in nature in the form of the "all-R" stereoisomer. Phytol contains 2 stereocentres and in addition a trisubstituted carbon-carbon double bond which gives rise to E/Z-steroisomers, while isophytol and tocopherols have 3 stereocentres. Therefore, there are multiple isomers.

It has been shown that of the naturally occurring stereoisomers of tocopherols, (2R,4'R,8'R)-tocopherols, particularly (2R,4'R,8'R)-α-tocopherol, have the highest bioactivity (biopotency).

As natural sources of (2R,4'R,8'R)-tocopherols and (R,R)-phytol, however, are very limited, the market has a strong need for an effective synthesis of (2R,4'R,8'R)-tocopherols and (R,R)-isophytol and (6R,10R)-6,10,14-trimethylpentadecan-2-one, the starting material of these products, which is useful for industrial scale application.

As, furthermore, higher bioactivity (biopotency) has been shown, for example by H. Weiser et al. in J. Nutr. 1996, 126(10), 2539-49, to occur in general by tocopherols having the R-configuration at the chiral centre situated next to the ether oxygen atom in the ring of the molecule (i.e. 2R-configuration), as compared to the corresponding isomers having S-configuration, there is a strong need for an effective and industrial scale synthesis of (2R,4'R,8'R)-tocopherols, particularly (2R,4'R,8'R)-alpha-tocopherol.

Takano et al., Synlett, no.4, 1991, 279-282 discloses an enantiocontrolled synthesis of phytol by reiterative application of chiral 3-hydroxyalkylene formation reaction.

### Summary of the invention

Therefore, the problem to be solved by the present invention is to offer a process for the manufacturing (6R,10R)-6,10,14-trimethylpentadecan-2-one.

Surprisingly, it has been found that the process according to claim 1 is able to solve this problem. It has been shown that it is possible to obtain one specific isomer of interest from a mixture of isomers of starting material, i. e. mixture of (E)-3,7-dimethyloct-2-enal and (Z)-3,7-dimethyloct-2-enal or a mixture of (E)-3,7-dimethylocta-2,6-dienal and (Z)-3,7-dimethylocta-2,6-dienal.

Preferred embodiments of the inventions allow making use of the non-desired isomers, by using a cis/trans-isomerization. The asymmetric hydrogenation, which is one of the key elements of this invention, can be improved in quality and speed by acetalization of the aldehydes or ketalization of the ketones to be asymmetrically hydrogenated as well as by the use of specific additives.

The process of the invention allows the production of the target molecules efficiently in a high quality from isomeric mixtures, allowing it to be used for industrial scale production. The process is very advantageous in that it forms the desired chiral product from a mixture of stereoisomers of the starting product in an efficient way.

Further aspects of the invention are subject of further independent claims. Particularly preferred embodiments are subject of dependent claims.

### Detailed description of the invention

In a first aspect the present invention relates to a process of manufacturing (6R,10R)-6,10,14-trimethylpentadecan-2-one in a multistep synthesis from 3,7-dimethyloct-2-enal or 3,7-dimethylocta-2,6-dienal comprising the steps
a) providing a mixture of (E)-3,7-dimethyloct-2-enal and (Z)-3,7-dimethyloct-2-enal or a mixture of (E)-3,7-dimethylocta-2,6-dienal and (Z)-3,7-dimethylocta-2,6-dienal;
b) separating one isomer of 3,7-dimethyloct-2-enal or 3,7-dimethylocta-2,6-dienal from the mixture of step a)
c) asymmetric hydrogenation using molecular hydrogen in the presence of a chiral iridium complex and yielding (R)-3,7-dimethyloctanal ;
d) chemically transforming (R)-3,7-dimethyloctanal to a mixture of (R,E)-6,10,14-trimethylpentadec-5-en-2-one and (R,Z)-6,10,14-trimethylpentadec-5-en-2-one ;
e) separating one isomer of (R)-6,10,14-trimethylpentadec-5-en-2-one from the mixture obtained in step d)
f) asymmetric hydrogenation using molecular hydrogen in the presence of a chiral iridium complex and yielding (6R,10R)-6,10,14-trimethylpentadecan-2-one ;
wherein the steps a)-f) are in the order a,b,c,d,e,f.

The term "independently from each other" in this document means, in the context of substituents, moieties, or groups, that identically designated substituents, moieties, or groups can occur simultaneously with a different meaning in the same molecule.

A "C_{x-y}-alkyl" group is an alkyl group comprising x to y carbon atoms, i.e., for example, a C₁₋₃-alkyl group is an alkyl group comprising 1 to 3 carbon atoms. The alkyl group can be linear or branched. For example -CH(CH₃)-CH₂-CH₃ is considered as a C₄-alkyl group.

A "C_{x-y}-alkylene" group is an alkylene group comprising x to y carbon atoms, i.e., for example C₂-C₆ alkylene group is an alkyl group comprising 2 to 6 carbon atoms. The alkylene group can be linear or branched. For example the group -CH(CH₃)-CH₂- is considered as a C₃-alkylene group.

A "phenolic alcohol" means in this document an alcohol which has a hydroxyl group which is bound directly to an aromatic group.

The term "(R,R)-isophytol" used in this document means (3RS,7R,11R)-3,7,11,15-tetramethylhexadec-1-en-3-ol).

The term "(R,R)-phytol" used in this document means (2E,7R,11R)-3,7,11,15-tetramethyl-2-hexadecen-1-ol).

Substance names starting with "poly" such as polythiol as used in the present document refer to substances formally containing two or more of the corresponding functional groups per molecule.

The term "stereogenic centre" as used in this document is an atom, bearing groups such that interchanging of any two of the groups leads to a stereoisomer. Stereoisomers are isomeric molecules that have the same molecular formula and sequence of bonded atoms (constitution), but that differ in the three-dimensional orientations of their atoms in space.

The configuration at a stereogenic centre is defined to be either R or S. The R/S-concept and rules for the determination of the absolute configuration in stereochemistry is known to the person skilled in the art.

In the present document a carbon-carbon double bond is defined as being "prochiral" if addition of molecular hydrogen to said carbon-carbon double bond leads to the formation of a stereogenic carbon centre.

Cis/trans isomers are configurational isomers having different orientation at the double bond. In this document the term "cis" is equivalently used for "Z" and vice versa as well as "trans" for "E" and vice versa. Therefore, for example the term "cis/trans isomerization catalyst" is equivalent to the term "E/Z isomerization catalyst".

A "cis/trans isomerization catalyst" is a catalyst which is able to isomerize a cis isomer (Z-isomer) to a cis/trans isomer mixture (E/Z isomer mixture) or to isomerize a trans isomer (E-isomer) to a cis/trans isomer (E/Z isomer mixture).

The terms "E/Z", "cis/trans" and "R/S" denote mixtures of E and Z, of cis and trans, and of R and S, respectively.

In case identical labels for symbols or groups are present in several formulae, in the present document, the definition of said group or symbol made in the context of one specific formula applies also to other formulae which comprises said same label.

In the present document any single dotted line represents the bond by which a substituent is bound to the rest of a molecule.

3,7-dimethyloct-2-enal is a known substance. 3,7-dimethyloct-2-enal is a mixture of (E)-3,7-dimethyloct-2-enal and (Z)-3,7-dimethyloct-2-enal.

3,7-dimethylocta-2,6-dienal is a known substance and commercially available and is a mixture of mixture of (E)-3,7-dimethylocta-2,6-dienal and (Z)-3,7-dimethylocta-2,6-dienal.

In a first step (step a) of the process a mixture of mixture of (E)-3,7-dimethyloct-2-enal and (Z)-3,7-dimethyloct-2-enal or a mixture of (E)-3,7-dimethylocta-2,6-dienal and (Z)-3,7-dimethylocta-2,6-dienal is provided.

### Separation

Steps b) and e) relate to the separation of one of the isomers of 3,7-dimethyloct-2-enal and 3,7-dimethylocta-2,6-dienal from the mixture of step a) and to the separation of one of the isomers of (R)-6,10,14-trimethylpentadec-5-en-2-one from the mixture obtained in step d), respectively.

This separation of isomers in step b) and/or e) can be done in different ways. A first possibility is the separation by means of chromatography. A further and preferred way of separation is that the separation of isomers in step b) and/or e) is done by distillation. The separation is possible by the fact that the isomers have different boiling points. In order to minimize thermal degradation of the isomers it is advisable to distil under reduced pressure and by means of a distillation column.

As the isomers to be separated have different boiling points (see table 1) the isomers can be separated by distillation. Using specific distillation techniques and equipment it is possible to separate the isomer having the higher or the lower boiling point.

**Table 1. Boiling points of isomers.**

| **Substance** | **Boiling point** |
|---|---|
| (E)-3,7-dimethyloct-2-enal | 112 °C at 5 mbar |
| (E)-6,10-dimethylundec-5-en-2-one | 99 °C at 10 mbar |
| (Z)-6,10-dimethylundec-5-en-2-one | 95 °C at 10 mbar |
| (R,E)-6,10,14-trimethylpentadec-5-en-2-one | 122 °C at 2 mbar |
| (R,Z)-6,10,14-trimethylpentadec-5-en-2-one | 119 °C at 2 mbar |

### Cis/trans isomerization

In a preferred embodiment the distillation is done in the presence of a cis/trans isomerization catalyst.

Cis/trans isomerization catalysts are catalysts which isomerize the carbon carbon double bonds. It has been found that for the purpose of this invention said cis/trans isomerization catalysing the cis/trans isomerization of the double bonds is particularly nitrogen monoxide (NO) or an organic sulphur compound, particularly a polythiol.

Particularly suitable as cis/trans isomerization catalysts are polythiols of formula (X) or aromatic polythiols 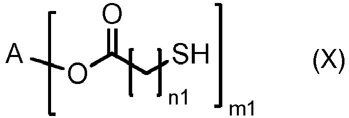
wherein n1 represents an integer from 1 to 4, particularly 2,
and m1 represents an integer from 2 to 8, particularly 3 or 4, preferably 4;
and A represents an aliphatic m1-valent hydrocarbon group of the molecular weight of between 28 g/mol and 400 g/mol, particularly between 90 and 150 g/mol.

The polythiols pentaerythritol tetra(mercaptoacetate), trimethylolpropane tris(mercaptoacetate), glycol dimercaptoacetate, pentaerythritol tetra-(3-mercaptopropionate), trimethylolpropanetri-(3-mercaptopropionate)(= 2-ethyl-2-(((3-mercaptopropanoyl)oxy)methyl)propane-1,3-diyl bis(3-mercaptopropanoate)) and glycol di-(3-mercaptopropionate) have been shown to be highly preferred polythiols of formula (X) and are the preferred polythiols of all the above mentioned polythiols.

Particularly preferred as aromatic polythiols are 4,4'-dimercaptobiphenyl or 4,4'-thiodibenzenethiol.

The use of polythiols of formula (X) as cis/trans isomerization catalysts is very advantageous in that polythiols have generally very low vapor pressures (i.e. high boiling points) allowing them to be used at elevated temperatures, e.g. while distilling the low boiling isomer. Furthermore, the polythiols bear a high density of thiol-functionalities per molecular weight, which is very advantageous, in that only little catalyst needs to be added.

The use of polythiol as cis/trans isomerization catalysts is very advantageous as they allow a very fast isomerization.

Nitrogen monoxide (NO) is a gas and can be introduced to the ketone or ketal to be isomerized as such or in the form of a gas mixture, particularly in combination with at least one inert gas, particularly with nitrogen. In the case a gas mixture is used the amount of nitrogen monoxide in the gas mixture is preferably in the range of 1 - 99 %, particularly of 5 -95 %, by weight of the gas mixture. Particularly, in view of corrosion and toxicity, the amount of nitrogen monoxide in the gas mixture is preferably in the range of 10 - 60 % by weight of the gas mixture.

The use of nitrogen monoxide as cis/trans isomerization catalysts is very advantageous in that the isomerization catalyst can be removed very easily from the ketone or ketal to be isomerized.

Nitrogen monoxide is preferably introduced to the ketone or ketal at atmospheric pressure or up to 1 MPa over-pressure. The over-pressure preferably amounts to 10 to 300 kPa.

Nitrogen monoxide (NO) or a mixture of nitrogen monoxide (NO) with other gases is preferably introduced in a continuous way by means of a tube and bubbled through the ketone or ketal to be isomerized.

The use of cis/trans isomerization allows the transformation of a pure cis or trans isomer or any mixtures of the isomers to yield a thermodynamically equilibrated mixture of the cis and trans isomer. Overall, this enables the separation of the desired isomer by distillation and transformation (isomerization) of the non-preferred isomer (residual isomer) into the desired isomer.

The distillation can be performed in the presence of the cis/trans isomerization catalyst (*one-pot isomerization* or *in-situ isomerization*), so that the desired isomer is re-formed continuously and can be separated by distillation.

Furthermore, the cis/trans isomerization can occur in a separate vessel in which the cis/trans isomerization catalyst is added to the remainder of the distillation. Hence, the residual isomer is isomerized by means of a cis/trans isomerization catalyst and subsequently added to the corresponding mixture of isomers provided in steps a) and d), respectively.

The use of the cis/trans isomerization in step b) and/or e) allows a high yield in the desired isomer. In preferred cases, it can be achieved that essentially all of the undesired isomer is overall isomerized to the desired isomer.

Preferably, particularly in the case where the isomerization catalyst is not nitrogen monoxide, more preferably in the case of polythiols as isomerization catalysts, the isomerization is undertaken at temperatures higher than 20° C, particularly at a temperature of between 20 °C and the boiling point of the desired isomer, particularly between 50 °C and the boiling point of the desired isomer. The isomerization can occur at ambient pressure or at reduced pressure. In case of the *one-pot isomerization* the isomerization is preferably undertaken under reduced pressure.

Particularly for the case of nitrogen monoxide being cis/trans isomerization catalyst the isomerization is undertaken at ambient or over-pressure.

It further has been observed that in the isomerization with polythiols addition of polar solvents such as amides, pyrrolidones, sulfones, sulfoxides, ionic liquids, particularly N,N-dimethylformide (DMF) or N-methyl-2-pyrrolidone (NMP), sulfolane, dimethylsulfoxide (DMSO) and 1-butyl-3-methylimidazolium bromide has an accelarating effect on the isomerization .

Therefore, it is preferred that the process of a cis/trans isomerization is undertaken in the presence of a polar solvent, particularly a polar solvent which is selected from the group consisting of ionic liquids, particularly 1-butyl-3-methylimidazolium bromide, N,N-dimethylformide (DMF), N-methyl-2-pyrrolidone (NMP), sulfolane and dimethylsulfoxide (DMSO).

More preferred it is that the process of a cis/trans isomerization is undertaken in the presence of a polar solvent, particularly a polar solvent which is selected from the group consisting of ionic liquids, particularly 1-butyl-3-methylimidazolium bromide, N,N-dimethylformide (DMF), N-methyl-2-pyrrolidone (NMP) and dimethylsulfoxide (DMSO).

The amount of cis/trans isomerization catalyst is preferably between 1 and 20 % by weight in relation to the amount of the isomers of 3,7-dimethyloct-2-enal or 3,7-dimethylocta-2,6-dienal, respectively to (R)-6,10,14-trimethylpentadec-5-en-2-one .

### Ketal acetal formation

In a further embodiment before the step c) a step cₒ) takes place
cₒ) forming an acetal of the isomer of 3,7-dimethyloct-2-enal or of the acetal of the isomer of 3,7-dimethylocta-2,6-dienal separated in step b)

Hence, in step c) the acetal of 3,7-dimethyloct-2-enal or the acetal of 3,7-dimethylocta-2,6-dienal is asymmetrically hydrogenated and after the asymmetric hydrogenation the hydrogenated acetal is hydrolysed to the ketone and yielding (R)-3,7-dimethyloctanal .

In a further embodiment before the step f) a step fₒ) takes place
fₒ) forming a ketal of the isomer of (R)-6,10,14-trimethylpentadec-5-en-2-one separated in step e)

Hence, in step f) the ketal of (R)-6,10,14-trimethylpentadec-5-en-2-one is asymmetrically hydrogenated and after the asymmetric hydrogenation the hydrogenated ketal is hydrolysed to the ketone and yielding (6R,10R)-6,10,14-trimethylpentadecan-2-one .

The formation of an acetal or ketal from an aldehyde or a ketone, per se, is known to the person skilled in the art.

The acetal of an unsaturated aldehyde can be preferably formed from the above mentioned unsaturated aldehyde and an alcohol.

The ketal of an unsaturated ketone can be preferably formed from the above mentioned unsaturated ketone and an alcohol.

It is known to the person skilled in the art that there are alternative routes of synthesis for acetals or ketals. In principle, the acetal or ketal can also be formed by treating an aldehyde or a ketone with ortho-esters or by trans-ketalization or transacetalization such as disclosed for example in Pério et al., Tetrahedron Letters 1997, 38 (45), 7867-7870, or in Lorette and Howard, J. Org. Chem. 1960, 521-525. Preferably the acetal or ketal is formed from the above mentioned unsaturated ketone or aldehyde and an alcohol.

The alcohol can comprise one or more hydroxyl groups. The alcohol may be a phenolic alcohol or an aliphatic or cycloaliphatic alcohol. Preferably the alcohol has one or two hydroxyl groups.

In case the alcohol has one hydroxyl group, the alcohol is preferably an alcohol which has 1 to 12 carbon atoms. Particularly, the alcohol having one hydroxyl group is selected from the group consisting of methanol, ethanol, 1-propanol, 2-propanol, 1-butanol, 2-methyl-1-propanol, 2-butanol, pentane-1-ol, 3-methylbutane-1-ol, 2-methylbutane-1-ol, 2,2-dimethylpropan-1-ol, pentane-3-ol, pentane-2-ol, 3-methylbutane-2-ol, 2-methylbutan-2-ol, hexane-1-ol, hexane-2-ol, hexane-3-ol, 2-methyl-1-pentanol, 3-methyl-1-pentanol, 4-methyl-1-pentanol, 3-methyl-2-pentanol, 4-methyl-2-pentanol, 2-methyl-3-pentanol, 2,2-dimethyl-1-butanol, 2,3-dimethyl-1-butanol, 3,3-dimethyl-1-butanol, 3,3-dimethyl-2-butanol, 2-ethyl-1-butanol, and all structural isomers of heptanol, octanol and halogenated C₁-C₈-alkyl alcohols, particularly 2,2,2-trifluoroethanol. Particularly suitable are primary or secondary alcohols. Preferably primary alcohols are used as alcohols with one hydroxyl group. Particularly methanol, ethanol, 1-propanol, 2-propanol, 1-butanol, 2-butanol or 2,2,2-trifluoroethanol, preferably methanol, ethanol, 1-propanol, 1-butanol or 2,2,2-trifluoroethanol, are used as alcohols with one hydroxyl group.

In another embodiment the alcohol is a diol, hence has two hydroxyl groups. Preferably the diol is selected from the group consisting of ethane-1,2-diol, propane-1,2-diol, propane-1,3-diol, butane-1,4-diol, butane-1,3-diol, butane-1,2-diol, butane-2,3-diol, 2-methylpropane-1,2-diol, 2,2-dimethylpropane-1,3-diol, 1,2-dimethylpropane-1,3-diol, benzene-1,2-diol and cyclohexane-1,2-diols. From two cyclohexane-1,2-diols the preferred stereoisomer is syn-cyclohexane-1,2-diol (=*cis*-cyclohexane-1,2-diol).

The two hydroxyl group are in one embodiment bound to two adjacent carbon atoms, hence these diols are vicinal diols. Vicinal diols form a 5 membered ring in a ketal.

Particularly suitable are vicinal diols which are selected from the group consisting of ethane-1,2-diol, propane-1,2-diol, butane-1,2-diol, butane-2,3-diol, 2-methylpropane-1,2-diol, benzene-1,2-diol and *syn*-cyclohexane-1,2-diol, particularly ethane-1,2-diol.

Other particularly suitable are diols, in which the hydroxyl groups are separated by 3 carbon atoms, and, hence, form a very stable 6 membered ring in a ketal. Particularly suitable diols of this type are propane-1,3-diol, butane-1,3-diol, 2-methylpropane-1,3-diol, 2-methylbutane-1,3-diol, 2,2-dimethylpropane-1,3-diol, 1,2-dimethylpropane-1,3-diol, 3-methylpentane-2,4-diol and 2-(hydroxymethyl)-cyclohexanol.

Preferably primary alcohols are used as diols.

The reaction conditions and stoichiometry used for the acetal or ketal formation are known to the person skilled in the art.

The preferred acetals or ketals have the formula (XI) or (XII) 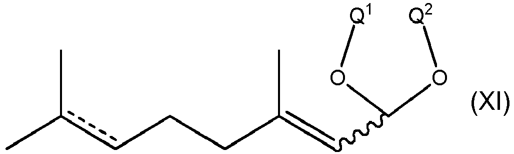 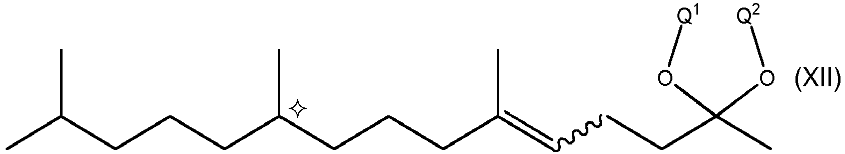
wherein a wavy line represents a carbon-carbon bond which is linked to the adjacent carbon-carbon double bond so as to have said carbon-carbon double bond either in the Z or in the E-configuration;
and wherein the double bond having dotted lines (------) in formula represent either a single carbon-carbon bond or a double carbon-carbon bond;
and wherein ✧ represents a stereogenic centre;
and wherein
Q¹ and Q²
   stand either individually or both for a C₁-C₁₀ alkyl group or a halogenated C₁-C₁₀ alkyl group;
   or form together a C₂-C₆ alkylene group or a C₆-C₈ cycloalkylene group.
   Q¹ and Q² stand particularly for
   either a linear C₁-C₁₀ alkyl group or fluorinated linear C₁-C₁₀ alkyl group, preferably a linear C₁-C₄ alkyl group or a -CH₂CF₃ group
   or a group of formula 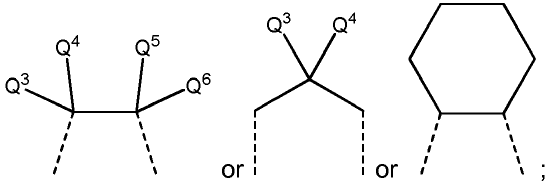
   in which Q³, Q⁴, Q⁵ and Q⁶ are independently from each other hydrogen atoms or methyl or ethyl groups.

Particularly Q¹ and Q² stand both for a fluorinated linear C₁-C₁₀ alkyl group, preferably a linear C₁-C₄ alkyl group or a -CH₂CF₃ group or form together the alkylene group CH₂-C(CH₃)₂-CH₂.

Hence the preferred acetals or ketals to be asymmetrically hydrogenated are selected from the group consisting of 1,1-dimethoxy-3,7-dimethyloct-2-ene, 1,1-diethoxy-3,7-dimethyloct-2-ene, 1,1-dipropoxy-3,7-dimethyloct-2-ene, 1,1-diisopropoxy-3,7-dimethyloct-2-ene, 1,1-di-sec-butoxy-3,7-dimethyloct-2-ene, 3,7-dimethyl-1,1-bis(pentan-2-yloxy)oct-2-ene, 1,1-bis(isopentyloxy)-3,7-dimethyloct-2-ene, 1,1-bis(pentyloxy)-3,7-dimethyloct-2-ene, 1,1-bis(hexyloxy)-3,7-dimethyl-oct-2-ene, 2-(2,6-dimethylhept-1-en-1-yl)-1,3-dioxolane, 2-(2,6-dimethylhept-1-en-1-yl)-4-methyl-1,3-dioxolane, 2-(2,6-dimethylhept-1-en-1-yl)-4,5-dimethyl-1,3-di-oxolane, (3aR,7aS)-2-(2,6-dimethylhept-1-en-1-yl)hexahydrobenzo[d][1,3]dioxole, 2-(2,6-dimethylhept-1-en-1-yl)-1,3-dioxane, 2-(2,6-dimethylhept-1-en-1-yl)-5-methyl-1,3-dioxane, 2-(2,6-dimethylhept-1-en-1-yl)-5,5-dimethyl-1,3-dioxane, 3,7-dimethyl-1,1-bis(2,2,2-trifluoroethoxy)octa-2,6-diene, 3,7-dimethyl-1,1-bis(2,2,2-trifluoroethoxy)oct-2-ene and all the E- and Z-isomers thereof;
or the group consisting of 1,1-dimethoxy-3,7-dimethylocta-2,6-diene, 2-(2,6-dimethylhepta-1,5-dien-1-yl)-1,3-dioxolane, 2-(2,6-dimethylhepta-1,5-dien-1-yl)-4-methyl-1,3-dioxolane, 2-(2,6-dimethylhepta-1,5-dien-1-yl)-1,3-dioxane, 2-(2,6-dimethylhepta-1,5-dien-1-yl)-5-methyl-1,3-dioxane, 2-(2,6-dimethylhepta-1,5-dien-1-yl)-5,5-dimethyl-1,3-dioxane, 3,7-dimethyl-1,1-bis(2,2,2-trifluoroethoxy)-octa-2,6-diene and all the E- and Z-isomers thereof;
or the group consisting of 2,5,5-trimethyl-2-(4,8,12-trimethyltridec-3-en-1-yl)-1,3-dioxane, 6,10,14-trimethyl-2,2-bis(2,2,2-trifluoroethoxy)pentadec-5-ene, (R)-2,5,5-trimethyl-2-(4,8,12-trimethyltridec-3-en-1-yl)-1,3-dioxane, (R)-6,10,14-trimethyl-2,2-bis(2,2,2-trifluoroethoxy)pentadec-5-ene and all the E- and Z-isomers thereof.

The hydrolysis of the hydrogenated ketal to the corresponding ketone is known to the person skilled in the art. Particularly suitable is the hydrolysis by means of an acid and isolation of the ketone formed, particularly by means of extraction.

### Asymmetric hydrogenation

Steps c) and f) involve asymmetric hydrogenations by molecular hydrogen in the presence of a chiral iridium complex.

Chiral iridium complexes are compounds having organic ligands being coordinated to a central iridium atom. The chirality of chiral iridium complexes is due to either the chirality of the ligands or the spacial arrangements of the ligands. This concept of chirality is well known from complex chemistry. Ligands can be monodentate or polydentate. Preferably, the ligands bound to the iridium central atom are chelating ligands. For the present invention, it has been shown that particularly chiral iridium complexes having an organic ligand bearing a stereogenic centre are very suitable.

It is preferred that the chiral iridium complex is bound to a chelating organic ligand having N and P as coordinating atoms and to either two olefins or to a diene having two carbon-carbon double bonds, and that, hence, the chiral iridium complex has preferably the following formula (III-0) 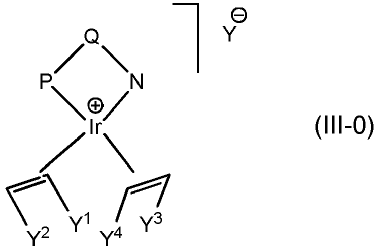 wherein
P-Q-N stands for a chelating organic ligand comprising a stereogenic centre or has planar or axial chirality and has a nitrogen and phosphorous atom as binding site to the iridium centre of the complex;
Y¹, Y², Y³ and Y⁴ are independently from each other hydrogen atoms, C₁₋₁₂-alkyl, C₅₋₁₀-cycloalkyl, or aromatic group; or at least two of them form together at least a two-valent bridged group of at least 2 carbon atoms; with the proviso that Y¹, Y², Y³ and Y⁴ are not all hydrogen atoms; and
Y^{⊝} is an anion, particularly selected from the group consisting of halide, PF₆⁻, SbF₆⁻, tetra(3,5-bis(trifluoromethyl)phenyl)borate (BAr_{F}⁻), BF₄⁻, perfluorinated sulfonates, preferably F₃C-SO₃⁻ or F₉C₄-SO₃⁻; ClO₄⁻, Al(OC₆F₅)₄⁻, Al(OC(CF₃)₃)₄⁻, N(SO₂CF₃)₂⁻ N(SO₂C₄F₉)₂⁻ and B(C₆F₅)₄⁻.

The nitrogen and the phosphorous atom are preferably separated by 2 to 5, preferably 3, atoms in the chemical formula of the ligand P-Q-N.

The chelating organic ligand P-Q-N is preferably selected from the formulae (III-N1), (III-N2), (III-N3), (III-N4), (III-N5), (III-N6), (III-N7), (III-N8) and (III-N9) 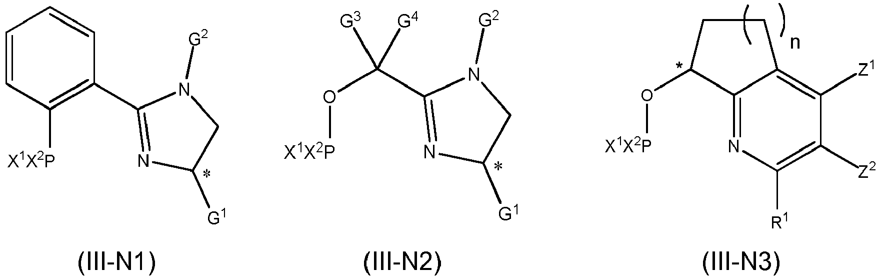 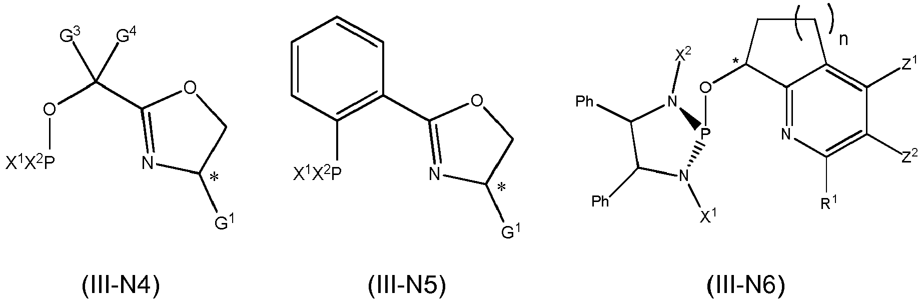 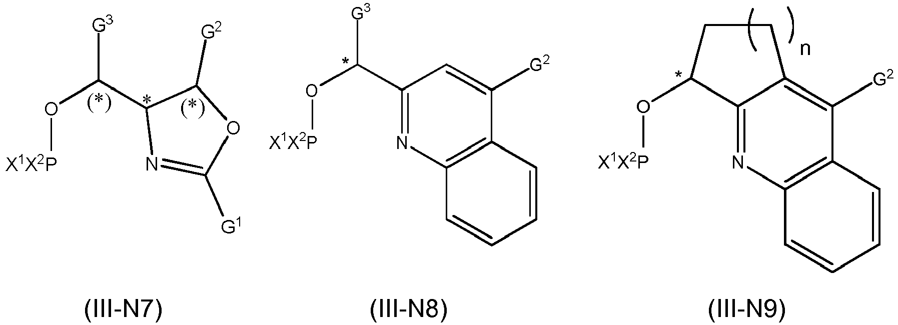
wherein G¹ represents either a C₁-C₄-alkyl, C₅₋₇-Cycloalkyl, adamantyl, phenyl (optionally substituted with one to three C₁₋₅- alkyl, C₁₋₄-alkoxy, C₁₋₄-perfluoroalkyl groups and/or one to five halogen atoms)), benzyl, 1-naphthyl, 2-naphthyl, 2-furyl group;
G², G³ and G⁴ represent independently from each other hydrogen atoms or a C₁-C₄-alkyl, C₅₋₇-cycloalkyl, adamantyl, phenyl (optionally substituted with one to three C₁₋₅-, C₁₋₄-alkoxy, C₁₋₄-perfluoroalkyl groups and/or one to five halogen atoms)), benzyl, 1-naphthyl, 2-naphthyl, 2-furyl group;
X¹ and X² are independently from each other hydrogen atoms, C₁₋₄-alkyl, C₅₋₇-Cycloalkyl, adamantyl, phenyl (optionally substituted with one to three C₁₋₅-alkyl, C₁₋₄-alkoxy, C₁₋₄-perfluoroalkyl groups and/or one to five halogen atoms)), benzyl, 1-naphthyl, 2-naphthyl, 2-furyl or ferrocenyl;
Ph stands for phenyl;
n is 1 or 2 or 3, preferred 1 or 2;
and R¹, Z¹ and Z² are as defined later for formula (III)

In case Y¹ and Y² and/or Y³ and Y⁴ form an olefin of the formula Y¹-=-Y² and/or of the Formula Y³-=-Y⁴, this olefin is or these olefins are preferably selected from the group consisting of ethene, prop-1-ene, 2-methylprop-1-ene, 2-methyl-but-2-ene, 2,3-dimethylbut-2-ene, (Z)-cyclooctene, cyclohexene, cycloheptene, cyclopentene and norbornene.

In case Y¹, Y², Y³ and Y⁴ are forming a diene, it is either cyclic (double bond in a cycle) or acyclic (double bond not in a cycle).

The two carbon-carbon double bonds of the diene are preferably linked by two carbon bonds, i.e. the dienes preferably comprise the substructure C=C-C-C-C=C.

Examples of preferred acylic dienes are hexa-1,5-diene, hepta-1,5-diene, octa-1,5-diene, octa-2,6-diene, 2,4-dialkyl-2,7-octadiene, 3,6-dialkylocta-2,6-diene, 1,2-divinylcyclohexane and 1,3-butadiene.

Examples for cyclic dienes are cycloocta-1,5-diene, cyclohexa-1,4-diene, cyclohexa-1,3-diene, 3,4,7,8-tetraalkylcycloocta-1,5-diene, 3,4,7-trialkylcycloocta-1,5-diene, 3,4-di alkylcycloocta-1,5-diene, 3,7-di-alkylcycloocta-1,5-diene, 3,8-di alkylcycloocta-1,5-diene, 3-alkylcycloocta-1,5-diene; norbornadiene, 1-alkylnorbornadiene, 2-alkylnorbornadiene, 7-alkylnorbornadiene, dicyclopentadiene, cyclopentadiene and (1s,4s)-bicyclo[2.2.2]octa-2,5-diene.

Preferred diene is cycloocta-1,5-diene.

A highly preferred class of chiral iridium complexes are chiral iridium complexes of formula (III) 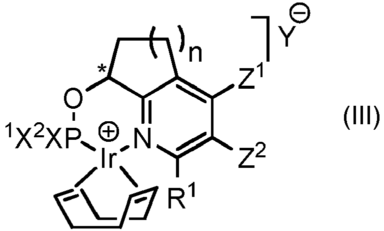 wherein
n is 1 or 2 or 3, preferred 1 or 2;
X¹ and X² are independently from each other hydrogen atoms, C₁₋₄-alkyl, C₅₋₇-cycloalkyl, adamantyl, phenyl (optionally substituted with one to three C₁₋₅-, C₁₋₄-alkoxy, C₁₋₄-perfluoroalkyl groups and/or one to five halogen atoms)), benzyl, 1-naphthyl, 2-naphthyl, 2-furyl or ferrocenyl;
Z¹ and Z² are independently from each other hydrogen atoms, C₁₋₅-alkyl or C₁₋₅-alkoxy groups;
or Z¹ and Z² stand together for a bridging group forming a 5 to 6 membered ring;
Y^{⊝} is an anion, particularly selected from the group consisting of halide, PF₆⁻, SbF₆⁻, tetra(3,5-bis(trifluoromethyl)phenyl)borate (BAr_{F}⁻), BF₄⁻, perfluorinated sulfonates, preferably F₃C-SO₃⁻ or F₉C₄-SO₃⁻; ClO₄⁻, Al(OC₆F₅)₄⁻, Al(OC(CF₃)₃)₄⁻, N(SO₂CF₃)₂⁻ N(SO₂C₄F₉)₂⁻ and B(C₆F₅)₄⁻;
R¹ represents either phenyl or o-tolyl or m-tolyl or p-tolyl or a group of formula (IVa) or (IVb) or (IVc) 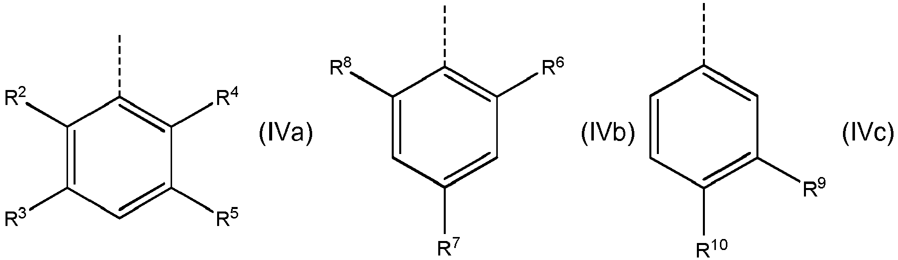
   wherein R² and R³ represent either both H or a C₁-C₄-alkyl group or a halogenated C₁-C₄-alkyl group or represent a divalent group forming together a 6-membered cycloaliphatic or an aromatic ring which optionally is substituted by halogen atoms or by C₁-C₄-alkyl groups or by C₁-C₄-alkoxy groups
   R⁴ and R⁵ represent either both H or a C₁-C₄-alkyl group or a halogenated C₁-C₄-alkyl group or a divalent group forming together a 6-membered cycloaliphatic or an aromatic ring which optionally is substituted by halogen atoms or by C₁-C₄-alkyl groups or by C₁-C₄-alkoxy groups;
   R⁶ and R⁷ and R⁸ represent each a C₁-C₄-alkyl group or a halogenated C₁-C₄-alkyl group;
   R⁹ and R¹⁰ represent either both H or a C₁-C₄-alkyl group or a halogenated C₁-C₄-alkyl group or a divalent group forming together a 6-membered cycloaliphatic or an aromatic ring which optionally is substituted by halogen atoms or by C₁-C₄-alkyl groups or by C₁-C₄-alkoxy groups;
and wherein * represents a stereogenic centre of the complex of formula (III).

The complex of formula (III) is neutral, i.e. the complex consists of a complex cation of formula (III') and anion Y as defined before.

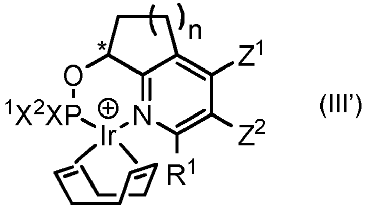

The person skilled in the art knows that anions and cations may be dissociated.

X¹ and/or X² represent preferably hydrogen atoms, methyl, ethyl, n-propyl, iso-propyl, n-butyl, iso-butyl, tert-butyl, n-pentyl, iso-pentyl, neopentyl, cyclopentyl, cyclohexyl, cycloheptyl, adamantly, phenyl, benzyl, o-tolyl, m-tolyl, p-tolyl, 4-methoxyphenyl, 4-trifluoromethylphenyl, 3,5-di-tert-butylphenyl, 3,5-dimethoxyphenyl, 1-naphthyl, naphthyl, 2-furyl, ferrocenyl or a phenyl group which is substituted with one to five halogen atoms.

In case of X¹ and/or X² representing phenyl groups which are substituted with one to five halogen atoms, the phenyl groups substituted by fluorine atoms are particularly useful, i.e. C₆H₄F, C₆H₃F₂, C₆H₂F₃, C₆HF₄ or C₆F₅.

In case of X¹ and/or X² representing phenyl groups which are substituted with one to three C₁₋₄-alkyl, the phenyl groups substituted by methyl group(s) are particularly useful, particularly ortho-tolyl and para-tolyl.

Preferably both X¹ and X² represent the same substituent.

Most preferred both X¹ and X² are phenyl or ortho-tolyl groups.

It is preferred that the C₁-C₄-alkyl or alkoxy groups used in the definition of R², R³, R⁴, R⁵ R⁶, R⁷, R⁸, R⁹ and R¹⁰ above are primary or secondary, preferably primary, alkyl or alkoxy groups.

A particularly suited substituent R¹ of formula (IVa) is the 9-anthryl or 1-naphthyl group.

A further particularly suited substituent R¹ of formula (IVb) is the mesityl group.

A further particularly suited substituent R¹ of formula (IVc) is the 2-naphthyl group.

Preferably R¹ is represented by phenyl (abbreviated as "Ph") or formula (IV-1) or (IV-2) or (IV-3), particularly (IV-1) or (IV-3).

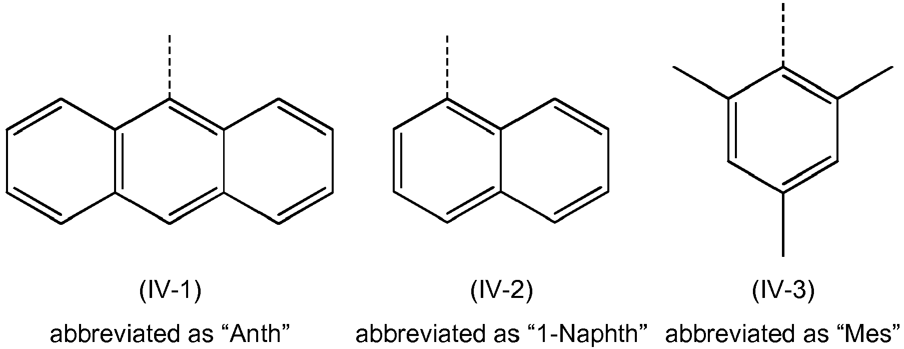

It has been found that the most preferred substituent R¹ is either 9-anthryl or phenyl.

The preferred chiral iridium complexes of formula (III) are the complexes of formulae (III-A), (III-B), (III-C), (III-D), (III-E) and (III-F).

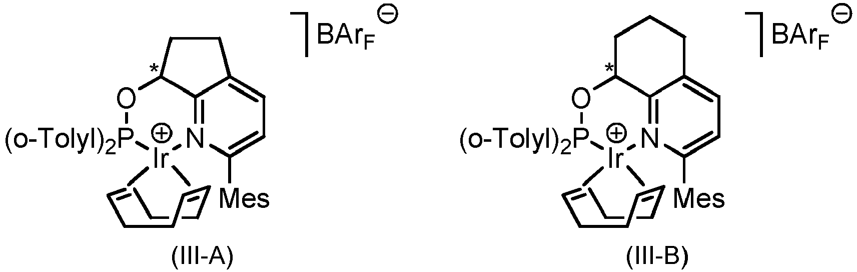

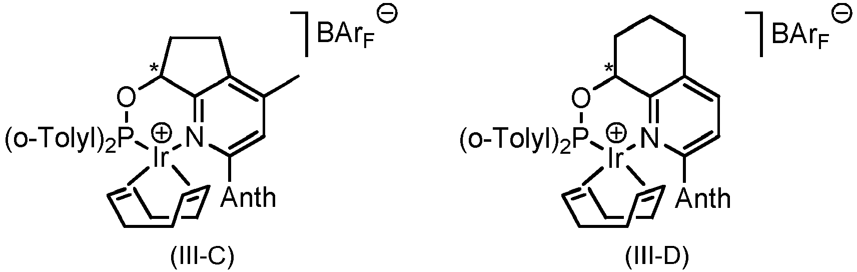

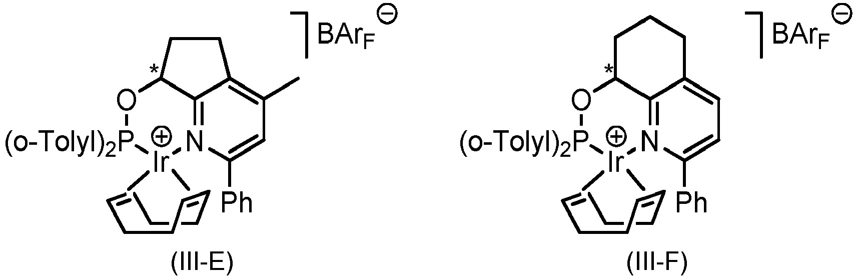

Most preferred as chiral iridium complexes of formula (III) are the complexes of formulae (III-C) and (III-D) and (III-F), particularly the one of formula (III-C) or (III-F).

The chiral iridium complexes of formula (III) can be synthesized accordingly as described in detail in Chem. Sci., 2010, 1, 72 - 78. The iridium complex of formula (III) is chiral. The chirality at said chiral centre marked by the asterisk is either S or R, i.e. there exist two enantiomers (IIIa) and (IIIb) of the chiral complex of formula (III):

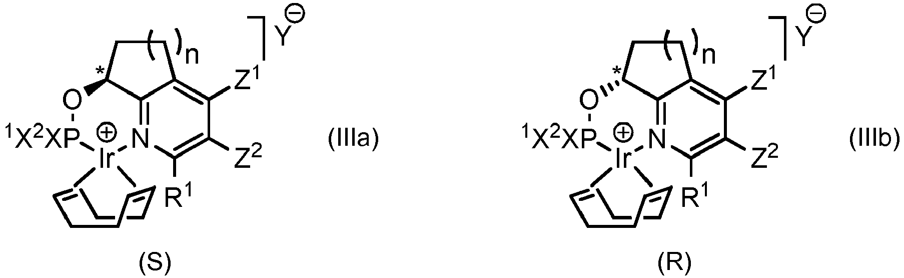

The individual enantiomers of the complex of formula (III) could be principally separated after the complexation step from a racemic mixture. However, as Chem. Sci., 2010, 1, 72 - 78 discloses, the synthesis of the complex of formula (III) comprises a reaction involving a non-racemic chiral alcohol. As it is known that the further reaction steps do not modify the chirality of the complex its isomeric purity (S:R-ratio) is governed therefore by the enantiomeric purity of said alcohol. As said corresponding alcohol can be obtained in a R/S ratio of more than 99 % resp. lower than 1 %, the complex of formula (III) can be obtained in extremely high enantiomeric purities, particularly in a R/S ratio of more than 99 % resp. lower than 1 %.

The chiral iridium complex is preferably used in an excess of one enantiomer.

Particularly, it is preferred that the ratio of the molar amounts of the individual enantiomers R:S of the chiral iridium complex of formula (III) is more than 90 : 10 or less than 10 : 90, preferably in the range of 100 : 0 to 98 : 2 or 0 : 100 to 2 : 98. Most preferred is that this ratio is about 100 : 0 resp. about 0 : 100. The ultimately preferred ratio is 100 : 0 resp. 0 : 100.

In one embodiment the stereogenic centre indicated by * has the R-configuration.

In another embodiment the stereogenic centre indicated by * has the S-configuration.

The hydrogenating agent is molecular hydrogen (H₂).

The amount of chiral iridium complex is preferably from about 0.0001 to about 5 mol %, preferably from about 0.001 to about 2 mol %, more preferably from about 0.01 to about 1 mol %, based on the amount of the ketone resp. ketal.

The hydrogenation can be carried out in substance or in an inert carrier, particularly in an inert solvent, or a mixture of inert solvents. The hydrogenation is preferably carried out in substance (neat).

Preferred suitable solvents are halogenated hydrocarbons, hydrocarbons, carbonates, ethers and halogenated alcohols.

Particularly preferred solvents are hydrocarbons, fluorinated alcohols and halogenated hydrocarbons, particularly halogenated aliphatic hydrocarbons.

Preferred examples of hydrocarbons are hexane, heptane, toluene, xylene and benzene, particularly toluene and heptane.

Preferred ethers are dialkylethers. Particularly useful ethers are dialklyethers with less than 8 carbon atoms. Most preferred ether is methyl *tert.-*butyl ether (CH₃-O-C(CH₃)₃).

Preferred halogenated alcohols are fluorinated alcohols. A particularly preferred fluorinated alcohol is 2,2,2-trifluoroethanol.

One preferred group of halogenated hydrocarbon are halogenated aromatic compounds, particularly chlorobenzene.

Preferred examples of halogenated aliphatic hydrocarbons are mono- or polyhalogenated linear or branched or cyclic C₁- to C₁₅-alkanes. Especially preferred examples are mono- or polychlorinated or -brominated linear or branched or cyclic C₁- to C₁₅-alkanes. More preferred are mono- or polychlorinated linear or branched or cyclic C₁- to C₁₅ -alkanes. Most preferred are dichloromethane, 1,2-dichloroethane, 1,1,1-trichloroethane, chloroform, and methylene bromide.

The most preferred solvent for the hydrogenation is dichloromethane.

The amount of solvent used is not very critical. However, it has been shown that the concentration of the ketone or ketal or aldehyde or acetal to be hydrogenated is preferably between 0.05 and 1 M, particularly between 0.2 and 0.7 M.

The hydrogenation reaction is conveniently carried out at an absolute pressure of molecular hydrogen from about 1 to about 100 bar, preferably at an absolute pressure of molecular hydrogen from about 20 to about 75 bar. The reaction temperature is conveniently between about 0 to about 100°C, preferably between about 10 to about 60°C.

The sequence of addition of the reactants and solvent is not critical.

The technique and apparatus suitable for the hydrogenation is principally known to the person skilled in the art.

By the asymmetric hydrogenation a prochiral carbon-carbon double bond is hydrogenated to form a chiral stereogenic centre at one or both of the carbon atoms.

In step c) either 3,7-dimethyloct-2-enal or 3,7-dimethylocta-2,6-dienal or an acetal of 3,7-dimethyloct-2-enal or an acetal of 3,7-dimethylocta-2,6-dienal is asymmetrically hydrogenated.

In step f) either 6,10,14-trimethylpentadec-5-en-2-one or a ketal of 6,10,14-trimethylpentadec-5-en-2-one is hydrogenated.

In case an acetal or ketal is asymmetrically hydrogenated, after the asymmetric hydrogenation the asymmetrically hydrogenated ketal has preferably the formula (XV) or (XVI). 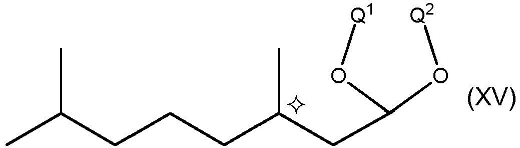 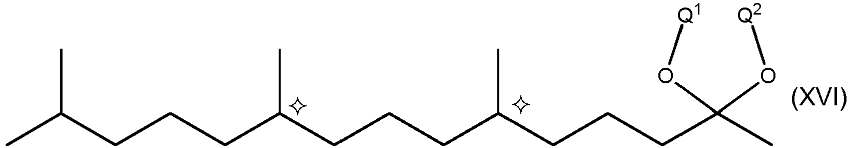
wherein ✧ represents a stereogenic centre;
and wherein Q¹ and Q² are as defined for formula (XI) and (XII).

Hence the preferred acetals or ketals which have been asymmetrically hydrogenated are preferably selected from the group consisting of
2-(2,6-dimethylheptyl)-5,5-dimethyl-1,3-dioxane,
3,7-dimethyl-1,1-bis(2,2,2-trifluoroethoxy)octane,
2,5,5-trimethyl-2-(4,8,12-trimethyltridecyl)-1,3-dioxane,
6,10,14-trimethyl-2,2-bis(2,2,2-trifluoroethoxy)pentadecane;
(R)-2-(2,6-dimethylheptyl)-5,5-dimethyl-1,3-dioxane,
(R)-3,7-dimethyl-1,1-bis(2,2,2-trifluoroethoxy)octane,
2,5,5-trimethyl-2-((4R,8R)-4,8,12-trimethyltridecyl)-1,3-dioxane and (6R,10R)-6,10,14-trimethyl-2,2-bis(2,2,2-trifluoroethoxy)pentadecane.

When these ketals or acetals are hydrolysed into the corresponding ketone, they yield 3,7-dimethyloctanal or 6,10,14-trimethylpentadecan-2-one, or (R)-3,7-dimethyloctanal or (6R,10R)-6,10,14-trimethylpentadecan-2-one, respectively.

Despite the fact that the asymmetric hydrogenation of 3,7-dimethyloct-2-enal or 3,7-dimethylocta-2,6-dienal and (R)-6,10,14-trimethylpentadec-5-en-2-one by means of molecular hydrogen in the presence of a chiral iridium complex, particularly those of formula (III), is already rather fast and efficient and shows high conversion rates as well as excellent selectivities, it has been observed that the asymmetric hydrogenation can even be improved when ketals of the corresponding ketones are asymmetrically hydrogenated.

It has been observed that the chiral iridium complex of a specific chirality (R or S) converts the starting material into a product bearing a specific stereogenic centre, which is formed as a result of the asymmetric hydrogenation.

As is in the present invention it is desired to produce products bearing a stereocentre with R-configuration by asymmetric hydrogenation i.e. (R)-3,7-dimethyloctanal in step c), and (6R,10R)-6,10,14-trimethylpentadecan-2-one in step f), respectively, the chirality of the chiral iridium complex needs to be selected depending on whether the olefin isomers being separated in step b) and step e), respectively, have the Z- or E-configuration.

It has been shown that when chiral iridium complexes of formula (III) having the S-configuration at the stereogenic centre indicated by * are used for the hydrogenation of E-isomers, i.e. (E)-3,7-dimethyloct-2-enal or (E)-3,7-dimethylocta-2,6-dienal and (R,E)-6,10,14-trimethylpentadec-5-en-2-one, respectively, the corresponding products, i.e. (R)-3,7-dimethyloctanal in step c), and (6R,10R)-6,10,14-trimethylpentadecan-2-one in step f), are obtained bearing the R-configuration at the newly formed stereogenic centre. Correspondingly, the hydrogenation of Z-isomers, i.e.

(Z)-3,7-dimethyloct-2-enal or (Z)-3,7-dimethylocta-2,6-dienal and (R,Z)-6,10,14-trimethylpentadec-5-en-2-one, respectively, in the presence of the chiral iridium complex of formula (III) having the R-configuration at the stereogenic centre indicated by * furnishes the same products, i.e. (R)-3,7-dimethyloctanal in step c), and (6R,10R)-6,10,14-trimethylpentadecan-2-one in step f), are obtained bearing the R-configuration at the newly formed stereogenic centre

Surprisingly, it has been found that this finding is independent from whether an aldehyde or ketone or an acetal or a ketal is used in step c) or f).

Therefore, the chiral iridium complex of formula (III) used in step c) and/or f) for the asymmetric hydrogenation preferably has the
S-configuration at the stereogenic centre indicated by * in case (E)-3,7-dimethyloct-2-enal or (E)-3,7-dimethylocta-2,6-dienal, or acetals thereof, or (R,E)-6,10,14-trimethylpentadec-5-en-2-one, or ketals thereof, is to be hydrogenated;
or has the
R-configuration at the stereogenic centre indicated by * in case (Z)-3,7-dimethyloct-2-enal or (Z)-3,7-dimethylocta-2,6-dienal, or acetals thereof, or (R,Z)-6,10,14-trimethylpentadec-5-en-2-one, or ketals thereof, is to be hydrogenated.

In a preferred embodiment of the invention the asymmetric hydrogenation in step c) and/or step f) takes place in the presence of an additive which is selected from the group consisting of organic sulfonic acids, transition metal salts of organic sulfonic acids, metal alkoxides, aluminoxanes, alkyl aluminoxanes and B(R)₍₃₋ᵥ₎(OZ)ᵥ; wherein v stands for 0, 1, 2 or 3 and R stands for F, a C₁₋₆-alkyl, a halogenated C₁₋₆-alkyl, an aryl or halogenated aryl group; and Z stands a C₁₋₆-alkyl, a halogenated C₁₋₆-alkyl, an aryl or halogenated aryl group.

Particularly suitable additives are selected from the group consisting of triflic acid, alkyl aluminoxanes, particularly methyl aluminoxane, ethyl aluminoxane, tetra alkoxy titanates, B(R)₍₃₋ᵥ₎(OZ)ᵥ; particularly tri-*iso*propylborate and triethylborane and BF₃, preferably in the form of a BF₃ etherate.

Particularly useful as the transition metal salts of organic sulfonic acids are scandium, indium, yttrium and zirconium salts of organic sulfonic acids.

Metal alkoxides are known to the person skilled in the art. This term particularly relates to the alkoxides of the elements of the group 4 and 13 of the periodic system. It is also known to the person skilled in the art that the metal alkoxides often do not form well-defined structures. Characteristically, metal alkoxides have hydrocarbyl group bound by an oxygen atom to a metal centre. A metal alkoxide may also have different metal centres which are bridged by oxygen or oxygen containing groups, such as for example (polynuclear) aluminium oxoalkoxides.

Particularly useful as metal alkoxides are titanium alkoxides (also being called alkoxy titanates) zirconium alkoxides (also being called alkoxy zirconates) or aluminium alkoxides.

A particularly preferred class of metal alkoxide is of the type of polynuclear aluminium oxoalkoxides such as disclosed in J. Chem. Soc., Dalton Trans., 2002, 259-266 or in Organometallics 1993, 12, 2429-2431.

Alkyl aluminoxanes, are known products which are particularly useful as co-catalysts for olefin polymerizations of the Ziegler-Natta type. They are prepared by controlled hydrolysis of trialkylaluminium compound, particularly trimethylaluminium or triethylaluminium. The hydrolysis can be achieved for example by hydrated metal salts (metal salts containing crystal water).

Preferably the additive is selected from the group consisting of triflic acid, alkyl aluminoxanes, particularly methyl aluminoxane, ethyl aluminoxane, tetra alkoxy titanates, B(R)₍₃₋ᵥ₎(OZ)ᵥ; particularly tri-*iso*propylborate and triethylborane and BF₃, preferably in the form of a BF₃ etherate.

More preferred are triflic acid, alkyl aluminoxanes, particularly methyl aluminoxane, ethyl aluminoxane, tetra alkoxy titanates, B(R)₍₃₋ᵥ₎(OZ)ᵥ; particularly tri-*iso*propylborate and triethylborane.

Especially good results have been obtained by an additive with has been obtained from trimethylaluminoxane and 2,2,2-trifluoroethanol or from trialkylaluminium and 2,2,2-trifluoroethanol.

It has been found that the quality and speed of the asymmetric hydrogenation using molecular hydrogen in the presence of a chiral iridium complex is enhanced significantly when the above mentioned additives are used.

It has been further observed that, most significantly, the efficiency of the asymmetric hydrogenation is maximized when the above mentioned additives are used with the corresponding acetals or ketasl of the aldehydes or ketones to be asymmetrically hydrogenated, i.e. ketals of 3,7-dimethyloct-2-enal or 3,7-dimethylocta-2,6-dienal or (R)-6,10,14-trimethylpentadec-5-en-2-one.

The increased efficiency has the effect that the amount of chiral iridium complex can be remarkably lowered by using an acetal or ketal of 3,7-dimethyloct-2-enal or 3,7-dimethylocta-2,6-dienal or (R)-6,10,14-trimethylpentadec-5-en-2-one. and/or addition of the mentioned additive(s), particularly in the combination with fluorinated alcohols, particularly 2,2,2-trfluoroethanol, to achieve a given yield and stereospecific hydrogenation in the asymmetric hydrogenation as compared to the corresponding asymmetric hydrogenation of 3,7-dimethyloct-2-enal or 3,7-dimethylocta-2,6-dienal or (R)-6,10,14-trimethylpentadec-5-en-2-one as such.

When the process comprises steps of cis/trans isomerization, as discussed above in detail, the process of invention is extremely interesting because for an optimal use of all starting material, it is not necessary to set up two parallel product lines for the separate asymmetric hydrogenation of each isomer using hydrogenation complexes of opposite chirality. Therefore, the *in-situ isomerization,* as discussed above, is much preferred.

### Chemical transformation

In step d) (R)-3,7-dimethyloctanal is chemically transformed to a mixture of (R,E)-6,10,14-trimethylpentadec-5-en-2-one and (R,Z)-6,10,14-trimethylpentadec-5-en-2-one ;

In a preferred method the chemical transformation of (R)-3,7-dimethyloctanal to a mixture of (R,E)-6,10,14-trimethylpentadec-5-en-2-one and (R,Z)-6,10,14-trimethylpentadec-5-en-2-one is done by the steps
d1) reacting with acetone to yield (R)-6,10-dimethylundec-3-en-2-one, i.e. mixture of (R,E)-6,10-dimethylundec-3-en-2-one and (R,Z)-6,10-dimethylundec-3-en-2-one;
d2) hydrogenation of (R)-6,10-dimethylundec-3-en-2-one with molecular hydrogen in the presence of a hydrogenation catalyst to yield (R)-6,10-dimethylundecan-2-one;
followed by
either
d3) ethynylation of (R)-6,10-dimethylundecan-2-one using ethyne in the presence of a basic substance to yield (7R)-3,7,11-trimethyldodec-1-yn-3-ol;
d4) hydrogenation of (7R)-3,7,11-trimethyldodec-1-yn-3-ol with molecular hydrogen in the presence of a Lindlar catalyst to yield (7R)-3,7,11-trimethyldodec-1-en-3-ol;
or
d3')vinylation of (R)-6,10-dimethylundecan-2-one by addition of a vinyl Grignard reagent to yield (7R)-3,7,11-trimethyldodec-1-en-3-ol;
followed by
either
d5) reacting (7R)-3,7,11-trimethyldodec-1-en-3-ol with 2-methoxyprop-1-ene to yield a mixture of (R,E)-6,10,14-trimethylpentadec-5-en-2-one and (R,Z)-6,10,14-trimethylpentadec-5-en-2-one;
or
d5')reacting (7R)-3,7,11-trimethyldodec-1-en-3-ol with an alkyl acetoacetate or diketene in the presence of a base and/or an acid yield a mixture of (R,E)-6,10,14-trimethylpentadec-5-en-2-one and (R,Z)-6,10,14-trimethylpentadec-5-en-2-one.

Details for the reaction type and conditions of the variant using steps d3) is disclosed in EP 1 532 092 B1, particularly in example 2, or WO 2003/029175 A1 (using a basic anion exchange resin). The hydrogenation with molecular hydrogen in the presence of a Lindlar catalyst in step d4) is known to the person skilled in the art. For example A. Ofner et al, Helv. Chim. Acta, 42,. 1959, 2577-2584 disclose the combination of steps d3) and d4). US 4,028,385 for example discloses details for the reaction type and conditions of the variant using step d3') as well as also for steps d3) and d4). Details for the reaction type and conditions of the variant using step d5), which is a Saucy-Marbet reaction, are disclosed for example in DE 1193490 and DE 196 49 564 A1. Details for the reaction type and conditions of the first variant in step d5'), which is a Carroll rearrangement, are disclosed for example in chapter 8 "The Carroll Rearrangement" in Hatcher et al., The Claisen Rearrangement; Hiersemann, M.; Nubbemeyer, U., Eds.; Wiley-VCH Verlag GmbH & Co. KGaA, 2007; 397-430. In the Carroll rearrangement the reaction occurs with an alkyl acetoacetate, preferably methyl acetoacetate or ethyl acetoacetate, in the presence of a base, preferably an alkali acetate such as sodium acetate.

The reaction type and conditions of the second variant in step d5') are disclosed for example in US 2,795,617 or in W. Kimel et al., J. Org. Chem. 1957, 22, 1611-1618. The reaction preferably occurs with diketene in the presence of preferably pyridine and acetic acid resp. in the presence of an alkoxide.

As mentioned earlier, (6R,10R)-6,10,14-trimethylpentadecan-2-one is an important intermediate and is particularly useful for the synthesis of (R,R)-isophytol, (2-*ambo*)-α-tocopherol or of (2R,4'R,8'R)-α-tocopherol.

Therefore, in a further aspect the invention relates to a process of manufacturing (R,R)-isophytol ((3RS,7R,11R)-3,7,11,15-tetramethylhexadec-1-en-3-ol) which comprises
the process of manufacturing (6R,10R)-6,10,14-trimethylpentadecan-2-one as described above in detail;
followed by the steps
either
g) ethynylation of (6R,10R)-6,10,14-trimethylpentadecan-2-one using ethyne in the presence of a base to yield (7R,11R)-3,7,11,15-tetramethyl-yn-3-ol;
h) hydrogenation of (7R,11R)-3,7,11,15-tetramethylhexadec-1-yn-3-ol with molecular hydrogen in the presence of a Lindlar catalyst to yield (R,R)-isophytol;
or
h') vinylation of (6R,10R)-6,10,14-trimethylpentadecan-2-one by addition of a vinyl Grignard reagent to yield (R,R)-isophytol.

The conditions for steps g) and h) and h') correspond to the ones described for the analogous steps d3) and d4) and d3') in step d).

In a further aspect the invention relates to a process of manufacturing compound of formula (V) comprising
the process of manufacturing (6R,10R)-6,10,14-trimethylpentadecan-2-one
as described above in detail;
followed by the steps
either
g) ethynylation of (6R,10R)-6,10,14-trimethylpentadecan-2-one using ethyne in the presence of a basic substance to yield (7R,11R)-3,7,11,15-tetramethylhexadec-1-yn-3-ol;
h) hydrogenation of (7R,11R)-3,7,11,15-tetramethylhexadec-1-yn-3-ol with molecular hydrogen in the presence of a Lindlar catalyst to yield (R,R)-isophytol;
or
h') vinylation of (6R,10R)-6,10,14-trimethylpentadecan-2-one by addition of a vinyl Grignard reagent to yield (R,R)-isophytol;
followed by the steps
m) condensing (R,R)-isophytol with compound of formula (VI) to yield compound of formula (V) being an isomeric mixture in view of the chirality at the centre indicated by #; 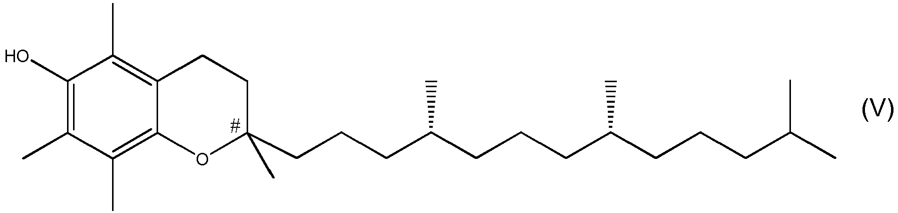 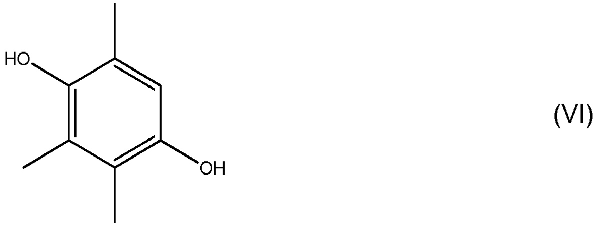 wherein # represents a stereogenic centre.

The conditions for steps g) and h) and h') correspond to the ones described for the analogous steps d1) and d2) and d1') in step d).

The condensation reaction of (R,R)-isophytol and compound of formula (VI), described as step m), is known by the person skilled in the art. For this condensation a series of catalysts may be used such as ZnCl₂/mineral acid, BF₃/AlCl₃, Fe/HCl, trifluoroacetic acid or boric acid/carboxylic acid as well as indium(III) or scandium(III) salts as disclosed in WO 2005/121115 A1. Furthermore, suitable catalysts are heteropoly acids, particularly 12-tungstophosphoric acid or 12-tungstosilicic acid such as disclosed in EP 0 970 953 A1.

The compounds of formula (V) represent (2-*ambo*)-α-tocopherol, i.e. a mixture of the corresponding (2R,4'R,8'R)-α-tocopherol and (2S,4'R,8'R)-α-tocopherol).

In a further aspect the invention relates to a process of manufacturing compound of formula (V-A) comprising
the process of manufacturing (6R,10R)-6,10,14-trimethylpentadecan-2-one as described above in detail;
followed by the steps
either
g) ethynylation of (6R,10R)-6,10,14-trimethylpentadecan-2-one using ethyne in the presence of a basic substance to yield (7R,11R)-3,7,11,15-tetramethylhexadec-1-yn-3-ol;
h) hydrogenation of (7R,11R)-3,7,11,15-tetramethylhexadec-1-yn-3-ol with molecular hydrogen in the presence of a Lindlar catalyst to yield (R,R)-isophytol;
or
h') vinylation of (6R,10R)-6,10,14-trimethylpentadecan-2-one by addition of a vinyl Grignard reagent to yield (R,R)-isophytol; followed by the steps
m) condensing (R,R)-isophytol with compound of formula (VI) to yield compound of formula (V) being an isomeric mixture in view of the chirality at the centre indicated by #; 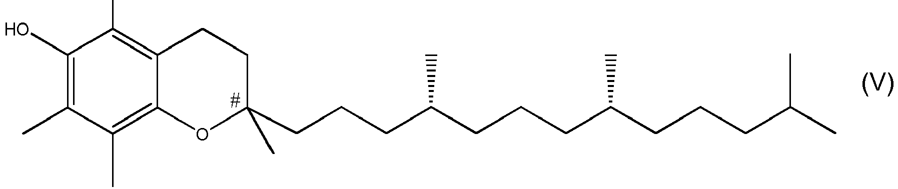 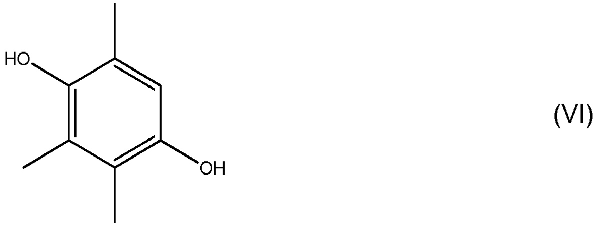 wherein # represents a stereogenic centre;
and
n) isolating compound of formula (V-A) from the isomeric mixture of formula (V)

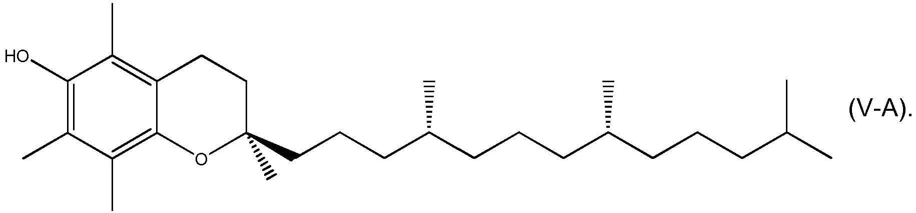

This process of manufacturing compound of formula (V-A) is the same as the process of manufacturing compound of formula (V) except for an additional step n).

The isolation of a (2R,4'R,8'R)-α-tocopherol from the corresponding (2-*ambo*)-*α*-tocopherol can be achieved by chromatographic separation by means of a chiral phase, particularly as described in WO2012/152779 A1. It is also preferred to enhance the yield in (2R,4'R,8'R)-α-tocopherol by means of epimerization of fractions enriched in (2S,4'R,8'R)-α-tocopherol as disclosed as step c) in WO2012/152779 A1.

The substances (E)-3,7-dimethyloct-2-enal, (Z)-3,7-dimethyloct-2-enal, (E)-3,7-dimethylocta-2,6-dienal (Z)-3,7-dimethylocta-2,6-dienal, (R,E)-3,7-dimethyloctanal, (R,Z)-3,7-dimethyloctanal; acetals of (E)-3,7-dimethyloct-2-enal, (Z)-3,7-dimethyloct-2-enal, (E)-3,7-dimethylocta-2,6-dienal (Z)-3,7-dimethylocta-2,6-dienal, (R,E)-3,7-dimethyloctanal, (R,Z)-3,7-dimethyloctanal; (R,E)-6,10,14-trimethylpentadec-5-en-2-one, (R,Z)-6,10,14-trimethylpentadec-5-en-2-one, ketals of (R,E)-6,10,14-trimethylpentadec-5-en-2-one, ketals of (R,Z)-6,10,14-trimethyl-pentadec-5-en-2-one, (6R,10R)-6,10,14-trimethylpentadecan-2-one, ketals of (6R,10R)-6,10,14-trimethylpentadecan-2-one, (7R)-3,7,11-trimethyldodec-1-yn-3-ol, (7R)-3,7,11-trimethyldodec-1-en-3-ol and (R,R)-isophytol
are important intermediates for the synthesis of tocopherols, vitamin K1, as well as for flavours and fragrances or for pharmaceutical products. The majority of them has a typical odour which makes them very attractive to be used as ingredients in products of the industry of flavours and fragrances such as in perfumes.

In a further aspect, the invention relates to a composition comprising
- at least one acetal or ketal of formula (XI) or (XII) and
- at least one chiral iridium complex;
   wherein ✧ represents a stereogenic centre which has the R-configuration.

The acetal or ketal of formula (XI) or (XII) and the chiral iridium complex, their ratios and as well their preferred embodiments, properties and effects have been discussed in this documents already in great detail.

In a final aspect, the invention relates to ketals of of formula (XX-A) or (XX-B) or (XX-C) or (XX-D) or (XX-E). 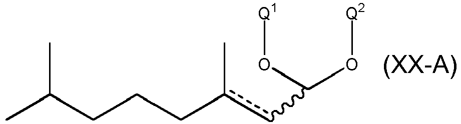 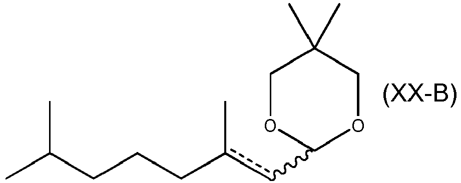 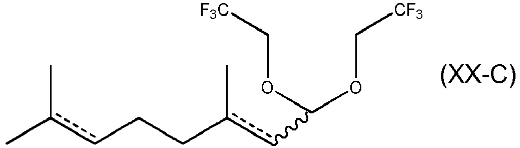 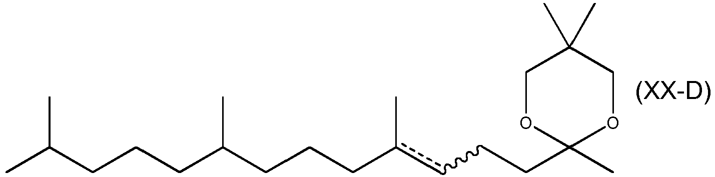 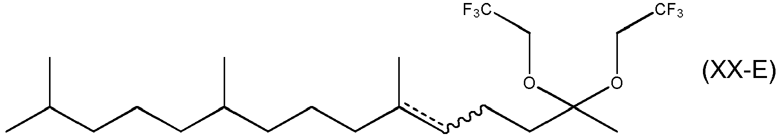
wherein the double bond having dotted lines (------) in the above formulae represents either a single carbon-carbon bond or a double carbon-carbon bond;and
wherein a wavy line represents a carbon-carbon bond which is linked to an adjacent single carbon bond (------ representing 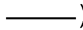 ) or to an adjacent carbon-carbon double bond (------ representing 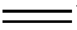 ) so as to have said carbon-carbon double bond either in the Z or in the E-configuration.
and wherein
Q¹ and Q²
   stand either individually or both for a C₁-C₁₀ alkyl group or a halogenated C₁-C₁₀ alkyl group;
   or form together a C₂-C₆ alkylene group or a C₆-C₈ cycloalkylene group; with the exception of 1,1-dimethoxy-3,7-dimethyloct-2-ene;
and wherein
the acetal or ketal of formula (XX-D) or (XX-E) is
   (R)-2,5,5-trimethyl-2-(4,8,12-trimethyltridec-3-en-1-yl)-1,3-dioxane
      or
   (R)-6,10,14-trimethyl-2,2-bis(2,2,2-trifluoroethoxy)pentadec-5-ene
      or
   2,5,5-trimethyl-2-((4R,8R)-4,8,12-trimethyltridecyl)-1,3-dioxane
      or
   (6R,10R)-6,10,14-trimethyl-2,2-bis(2,2,2-trifluoroethoxy)pentadecane.

Particularly the acetal or ketal is selected from
the group consisting of 1,1-diethoxy-3,7-dimethyloct-2-ene, 1,1-dipropoxy-3,7-dimethyloct-2-ene, 1,1-diisopropoxy-3,7-dimethyloct-2-ene, 1,1-di-sec-butoxy-3,7-dimethyloct-2-ene, 3,7-dimethyl-1,1-bis(pentan-2-yloxy)oct-2-ene, 1,1-bis(isopentyloxy)-3,7-dimethyloct-2-ene, 1,1-bis(pentyloxy)-3,7-dimethyloct-2-ene, 1,1-bis(hexyloxy)-3,7-dimethyloct-2-ene, 2-(2,6-dimethylhept-1-en-1-yl)-1,3-dioxolane, 2-(2,6-dimethylhept-1-en-1-yl)-4-methyl-1,3-dioxolane, 2-(2,6-dimethylhept-1-en-1-yl)-4,5-dimethyl-1,3-dioxolane, (3aR,7aS)-2-(2,6-dimethylhept-1-en-1-yl)hexahydrobenzo[d][1,3]dioxole, 2-(2,6-dimethylhept-1-en-1-yl)-1,3-dioxane, 2-(2,6-dimethylhept-1-en-1-yl)-5-methyl-1,3-dioxane, 2-(2,6-dimethylhept-1-en-1-yl)-5,5-dimethyl-1,3-dioxane, 3,7-dimethyl-1,1-bis(2,2,2-trifluoroethoxy)octa-2,6-diene, 3,7-dimethyl-1,1-bis(2,2,2-trifluoroethoxy)oct-2-ene and all the E- and Z-isomers thereof;
or from the group consisting of (E)-3,7-dimethyl-1,1-bis(2,2,2-trifluoro-ethoxy)octa-2,6-diene and (Z)-3,7-dimethyl-1,1-bis(2,2,2-trifluoroethoxy)octa-2,6-diene and all the E- and Z-isomers thereof;
or the group consisting of (R)-2,5,5-trimethyl-2-(4,8,12-trimethyltridec-3-en-1-yl)-1,3-dioxane, (R)-6,10,14-trimethyl-2,2-bis(2,2,2-trifluoroethoxy)pentadec-5-ene and all the E- and Z-isomers thereof.

All these acetals or ketals are particularly suited for the asymmetric hydrogenation as described above in detail or are the product of said asymmetric hydrogenation. As mentioned also before the unsaturated acetals or ketals behave extremely advantageously as compared to the corresponding ketones or aledhydes.

### Figures

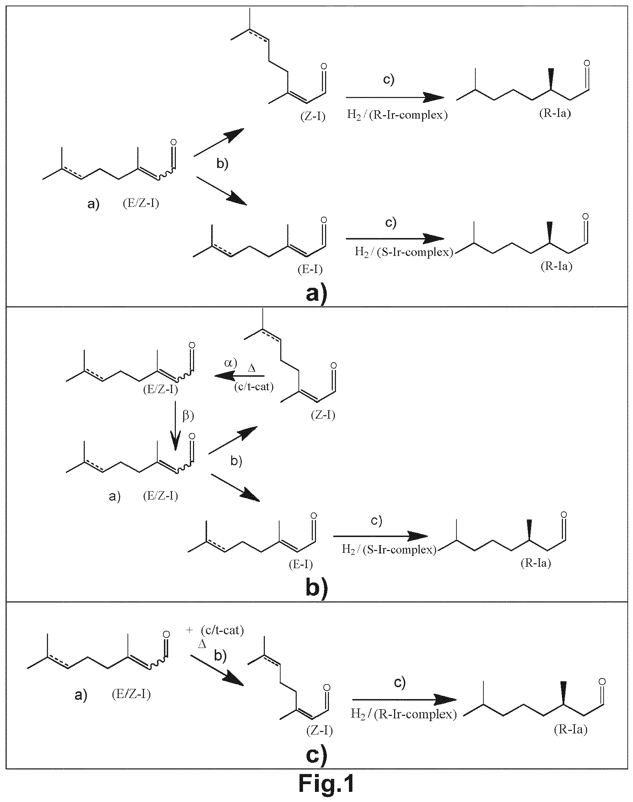

In the following paragraphs some preferred embodiments of the inventions are further discussed by means of schematic figures 1 to 7. This, however, is not to be understood as limiting the invention to the embodiments described here in the figures.

The reference signs in parentheses in the figures, such as (R-V) are used for identification purposes as described below and are not to be confused with the indication of formula such as (V) used in the rest of this document.

The figures 1 to 4 show the subsequent steps from both 3,7-dimethyloct-2-enal and 3,7-dimethylocta-2,6-dienal to (6R,10R)-6,10,14-trimethylpentadecan-2-one.

The figures 1 to 5 show the subsequent steps from both 3,7-dimethyloct-2-enal and 3,7-dimethylocta-2,6-dienal to (R,R)-isophytol, (2*-ambo*)-α-tocopherol and (2R,4'R,8'R)-α-tocopherol, respectively.

In figure 1, three different possibilities for the synthesis of (R)-3,7-dimethyloctanal (*R*-*Ia*) are schematically shown (Figure 1a), 1b), 1c)). As a first step a) for all possibilities shown in figure 1, a mixture of (E)-3,7-dimethyloct-2-enal and (Z)-3,7-dimethyloct-2-enal or a mixture of (E)-3,7-dimethylocta-2,6-dienal and (Z)-3,7-dimethylocta-2,6-dienal (*E*/*Z-I*) is provided. In figure 1a) the E-isomer (*E-I*) (i.e. (E)-3,7-dimethyloct-2-enal or (E)-3,7-dimethylocta-2,6-dienal, respectively) and the corresponding Z-isomer (*Z-I*) (i.e (Z)-3,7-dimethyloct-2-enal or (Z)-3,7-dimethylocta-2,6-dienal, respectively) are separated in step b) from the mixture provided in step a). The separation in step b) is preferably done by distillation over a column. In step c) the Z-isomer is asymmetrically hydrogenated with a specific chiral iridium complex, whereas the E-isomer is asymmetrically hydrogenated with the corresponding enantiomeric chiral iridium complex. A preferred chiral iridium complex is the one of formula (III). The E-isomer (*E-I*) is asymmetrically hydrogenated using molecular hydrogen in the presence of the chiral iridium complex of formula (IIIa) (*S-Ir-complex)* having the S-configuration at the stereogenic centre indicated by * in formula (III). The Z-isomer (*Z-I*), on the other hand, is asymmetrically hydrogenated using molecular hydrogen in the presence of the chiral iridium complex of formula (IIIb) (*R-Ir-complex*) having the R-configuration at the stereogenic centre indicated by * in formula (III). Both asymmetric hydrogenation routes furnish the same product, i.e. (R)-3,7-dimethyloctanal (*R-Ia*). The double bond at position 6 of (Z)-3,7-dimethylocta-2,6-dienal is also hydrogenated during the asymmetric hydrogenation. However, as this double bond is not prochiral, no chiral centre is formed at this position during the hydrogenation.

In figure 1b) only one of the isomers (E-isomer (*E-*I)) (here: desired isomer) is asymmetrically hydrogenated as described above for figure 1a). The other isomer (Z-isomer (*Z-I*)) (here: undesired isomer) is subjected to cis/trans isomerization in step α) by addition of a cis/trans isomerization catalyst (*c*/*t-cat*) and heating. The cis/trans isomerization catalyst preferably used is a polythiol, particularly of formula (X). By the action of the cis/trans isomerization catalyst the (Z-isomer (*Z-I*)) is isomerized to a mixture of the E-isomer and the Z-isomer (*E*/*Z-I*) which can be added in step β) to the mixture provided in step a). Figure 1b) shows the process in case the E-isomer is the desired isomer, i.e. the one which is asymmetrically hydrogenated. It is obvious that in case the Z-isomer is the desired isomer, i.e. the one which is asymmetrically hydrogenated, the isomerization process would apply in an analogous way to that of the E-isomer.

In figure 1c) only one of the isomers (Z-isomer (*Z-I*)) (here: desired isomer) is asymmetrically hydrogenated as described above for figure 1a). A cis/trans isomerization catalyst (*c*/*t*-*cat*) is added to the mixture of the E-isomer and the Z-isomer (*E*/*Z-I*) provided in step a). In step b) the separation of the (desired) isomer (Z-isomer (*Z-I*)) is done by distillation in the presence of the cis/trans isomerization catalyst in a (*one-pot isomerization* or *in-situ isomerization*). As the desired isomer is separated by distillation, the remainder, enriched in the higher boiling isomer, is isomerized so that in the distillation vessel a thermodynamic equilibrium between the Z- and E- isomers is formed continuously. This procedure may allow all of the undesired isomer being present in the isomer mixture at the beginning provided in step a) to be converted to the desired isomer.

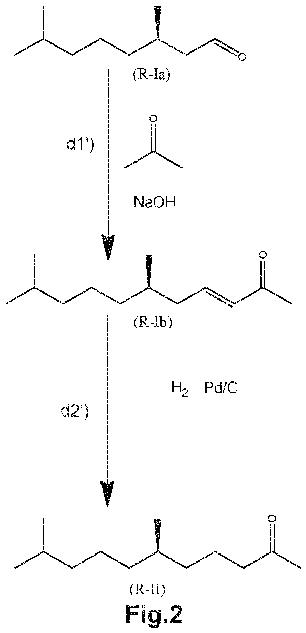

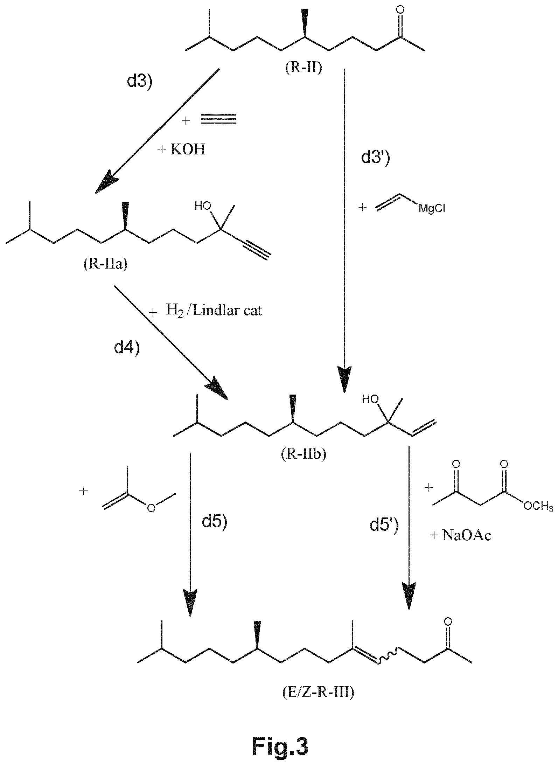

Figures 2 and 3 shows the chemical transformation of (R)-3,7-dimethyloctanal to a mixture of (R,E)-6,10,14-trimethylpentadec-5-en-2-one and (R,Z)-6,10,14-trimethylpentadec-5-en-2-one .

In figure 2 is the conversion of (R)-3,7-dimethyloctanal (*R-Ia*), followed by reaction in step d1') with acetone and a base schematically shown to yield (R)-6,10-dimethylundec-3-en-2-one (*R-Ib*), which is then hydrogenated in step d2') with molecular hydrogen in the presence of a hydrogenation catalyst (shown: a Pd/carbon hydrogenation catalyst) to yield (R)-6,10-dimethylundecan-2-one (*R-II*).

Figure 3 shows the subsequent steps of the chemical transformation step d): (R)-6,10-dimethylundecan-2-one (*R-II*) is chemically transformed to a mixture of (R,E)-6,10,14-trimethylpentadec-5-en-2-one and (R,Z)-6,10,14-trimethylpenta-dec-5-en-2-one (*E*/*Z-R-III*). Figure 3 also shows the preferred variants of such chemical transformations.

In one of the variants shown in figure 3, (7R)-3,7,11-trimethyldodec-1-en-3-ol (*R-IIb*) is formed from (R)-6,10-dimethylundecan-2-one (*R-II*) by reacting in a first step, i.e. step d3), (R)-6,10-dimethylundecan-2-one (*R-II*) with ethyne (acetylene) in the presence of a base (shown is KOH) to yield the intermediate (7R)-3,7,11-trimethyldodec-1-yn-3-ol (*R-IIa*) and then in second step, i.e. in step d4), reacting with molecular hydrogen in the presence of a Lindlar catalyst.

In another variant (7R)-3,7,11-trimethyldodec-1-en-3-ol (*R-IIb*) is formed directly by means of reaction with a Grignard reagent. In figure 3 vinylmagnesium chloride is shown as Grignard reagent.

Subsequently, two variants for the conversion of (7R)-3,7,11-trimethyldodec-1-en-3-ol (*R-IIb*) to the mixture of (R,E)-6,10,14-trimethylpentadec-5-en-2-one and (R,Z)-6,10,14-trimethylpentadec-5-en-2-one (*E*/*Z-R-III*) are shown in figure 3. In the first variant, (7R)-3,7,11-trimethyldodec-1-en-3-ol (*R-IIb*) is reacted in a Saucy-Marbet reaction (step d5)) with 2-methoxyprop-1-ene to yield a mixture of (R,E)-6,10,14-trimethylpentadec-5-en-2-one and (R,Z)-6,10,14-trimethylpentadec-5-en-2-one (*E*/*Z-R-III*). In the second variant, (7R)-3,7,11-trimethyldodec-1-en-3-ol (*R-IIb*) is reacted with alkyl acetoacetate, preferably methyl acetoacetate, in the presence of a base, preferably sodium acetate, to a mixture of (R,E)-6,10,14-trimethylpentadec-5-en-2-one and (R,Z)-6,10,14-trimethylpentadec-5-en-2-one (*E*/*Z-R-III*) (Carroll rearrangement).

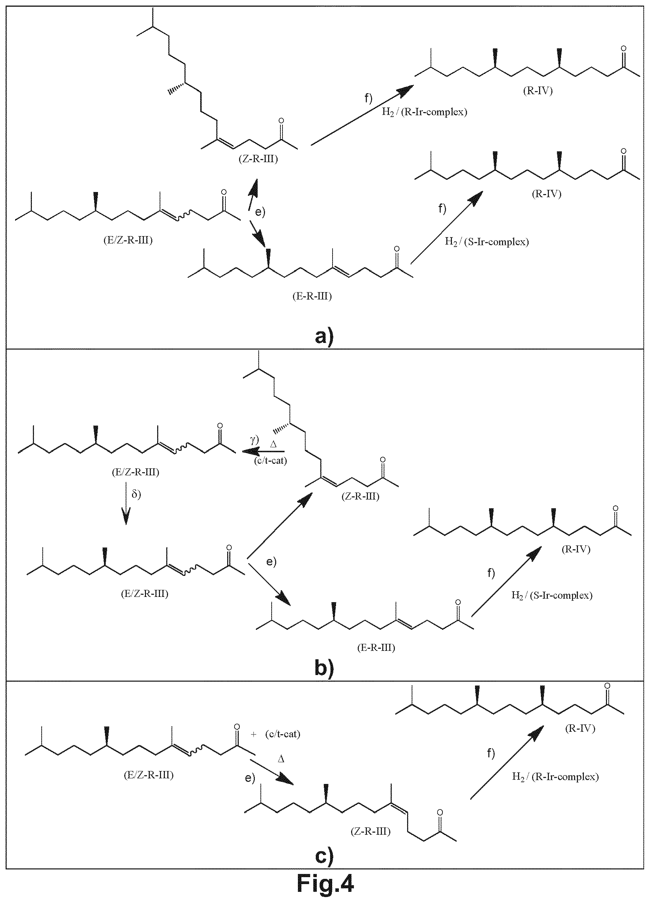

In figure 4, three different possibilities for the synthesis of (6R,10R)-6,10,14-trimethylpentadecan-2-one (*R-IV*) are schematically shown (Figure 4a), 4b), 4c)). As a first for all possibilities shown in figure 4, a mixture of (R,E)-6,10,14-trimethylpentadec-5-en-2-one and (R,Z)-6,10,14-trimethylpentadec-5-en-2-one (*E*/*Z-R-III*) is provided in step a). In figure 4a) the E-isomer (*E-R-III*) (i.e. (R,E)-6,10,14-trimethylpentadec-5-en-2-one) and the corresponding Z-isomer (*Z-R-III*) (i.e (R,E)-6,10,14-trimethylpentadec-5-en-2-one) are separated in step e) from the mixture. The separation in step e) is preferably done by distillation over a column. In step f) the Z-isomer is asymmetrically hydrogenated with a specific chiral iridium complex, whereas the E-isomer is asymmetrically hydrogenated with the corresponding enantiomeric chiral iridium complex. A preferred chiral iridium complex is the one of formula (III). The E-isomer (*E-R-III*) is asymmetrically hydrogenated using molecular hydrogen in the presence of the chiral iridium complex of formula (IIIa) (*S-Ir-complex)* having the S-configuration at the stereogenic centre indicated by * in formula (III). The Z-isomer (*Z-R-III*), on the other hand, is asymmetrically hydrogenated using molecular hydrogen in the presence of the chiral iridium complex of formula (IIIb) (*R-Ir-complex*) having the R-configuration at the stereogenic centre indicated by * in formula (III). Both asymmetric hydrogenation routes furnish the same product, i.e. (6R,10R)-6,10,14-trimethylpentadecan-2-one (*R-IV*).

In figure 4b) only one of the isomers (E-isomer (*E-R-III*)) (desired isomer) is asymmetrically hydrogenated as described above for figure 4a). The other isomer (Z-isomer (*Z-R-III*)) (undesired isomer) is subjected to cis/trans isomerization in step γ) by addition of a cis/trans isomerization catalyst (*c*/*t-cat*) and heating. The cis/trans isomerization catalyst preferably used is a polythiol, particularly of formula (X). By the action of the cis/trans isomerization catalyst the (Z-isomer (*Z-R-III*)) is isomerized to a mixture of the E-isomer and the Z-isomer (*E*/*Z-R-III*) which can be added in step δ) to the mixture. Figure 4b) shows the process in case the E-isomer is the desired isomer, i.e. the one which is asymmetrically hydrogenated. It is obvious that in case the Z-isomer is the desired isomer, i.e. the one which is asymmetrically hydrogenated, the isomerization process would apply in an analogous way to that of the E-isomer.

In figure 4c) only one of the isomers (Z-isomer (*Z-R-III*)) (desired isomer) is asymmetrically hydrogenated as described above for figure 4a). A cis/trans isomerization catalyst (*c*/*t-cat*) is added to the mixture of the E-isomer and the Z-isomer (*E*/*Z-R-III*). In step e) the separation of the (desired) isomer (Z-isomer (Z-*R-III*)) is done by distillation in the presence of the cis/trans isomerization catalyst in a (*one-pot isomerization* or *in-situ isomerization*). As the desired isomer is separated by distillation, the remainder, enriched in the higher boiling isomer, is isomerized so that in the distillation vessel a thermodynamic equilibrium between the Z- and E- isomers is formed continuously. This procedure may allow all of the undesired isomer being present in the isomer mixture at the beginning provided in step e) to be converted to the desired isomer.

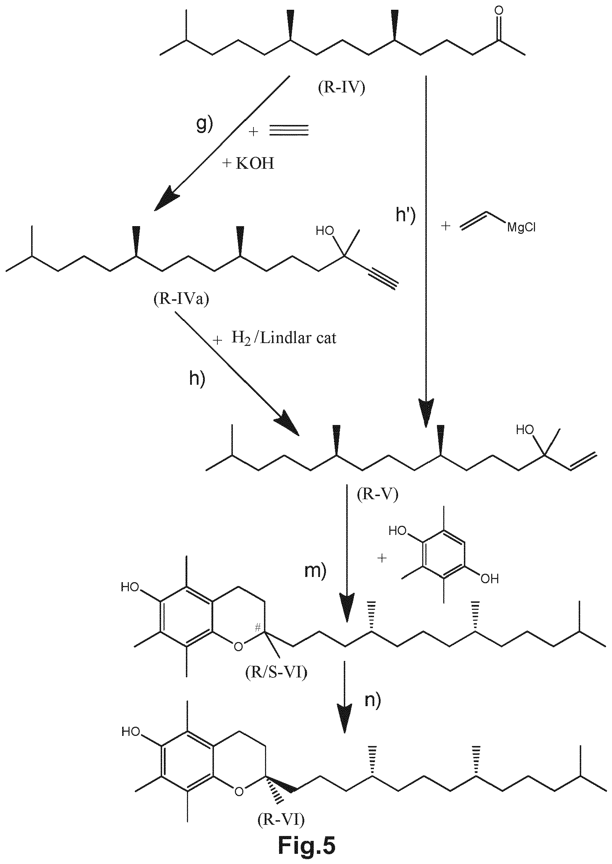

Figure 5 shows the subsequent steps from (6R,10R)-6,10,14-trimethylpentadecan-2-one to (R,R)-isophytol, (2-*ambo*)-α-tocopherol, and (2R,4'R,8'R)-α-tocopherol, respectively.

Figure 5 shows two variants for the conversion of (6R,10R)-6,10,14-trimethylpentadecan-2-one to (R,R)-isophytol. In the first variant, (R,R)-isophytol (*R-V*) is formed from (6R,10R)-6,10,14-trimethylpentadecan-2-one (*R-IV*) by reacting in a first step, i.e. step g), (6R,10R)-6,10,14-trimethylpentadecan-2-one (*R-IV*) with ethyne (acetylene) in the presence of a base (shown is KOH) to yield the intermediate (7R,11R)-3,7,11,15-tetramethylhexadec-1-yn-3-ol (*R-IVa*) and then in second step, i.e. in step h), reacting with molecular hydrogen in the presence of a Lindlar catalyst.

In the other variant shown, (R,R)-isophytol (*R-V*) is formed from (6R,10R)-6,10,14-trimethylpentadecan-2-one (*R-IV*) by means of reaction with a Grignard reagent, in step h'). In figure 5 vinylmagnesium chloride is shown as Grignard reagent.

(R,R)-isophytol (*R-V*) can further be condensed in step m) with 2,3,5-trimethylbenzene-1,4-diol to yield (2-*ambo*)-α-tocopherol (*R*/*S-VI*)).

In a further step n) (2R,4'R,8'R)-α-tocopherol (*R-VI*)) is isolated from the corresponding (2-*ambo*)-α-tocopherol (*R*/*S-VI*). The isolation is preferably done by chromatographic separation by means of a chiral phase.

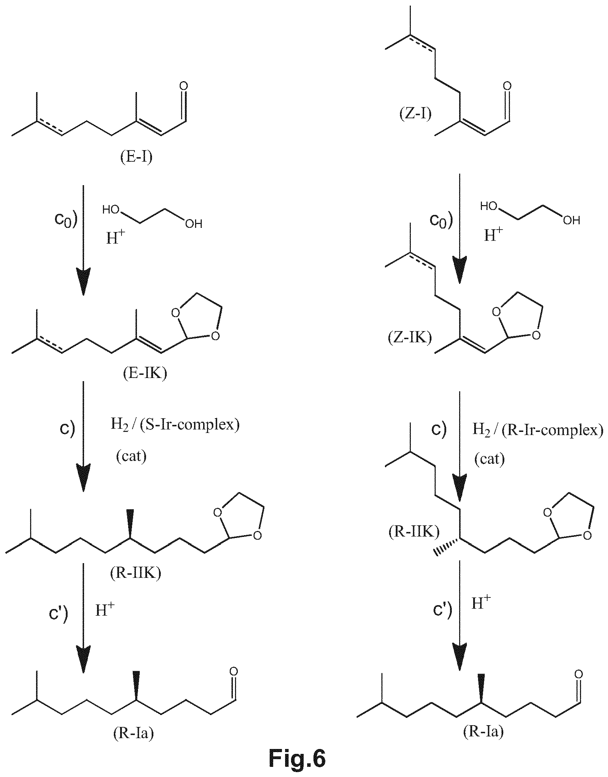

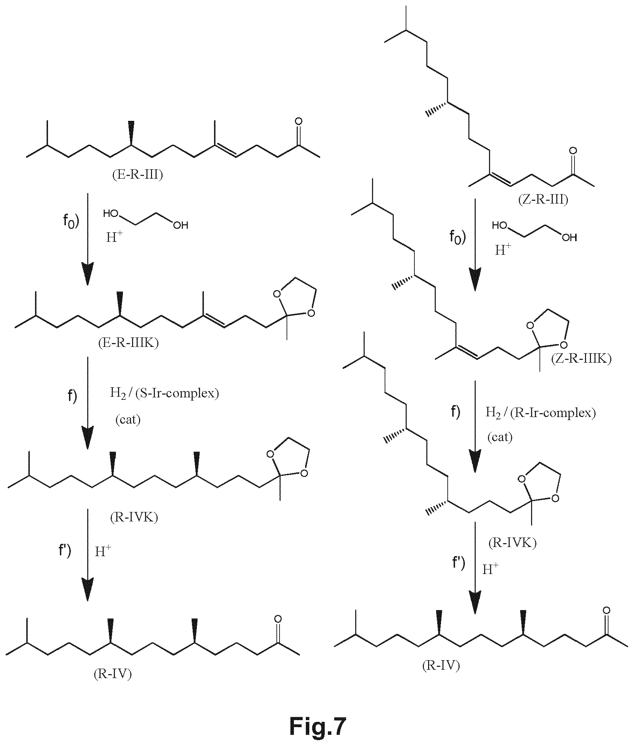

In figures 6 and 7 preferred embodiments of asymmetric hydrogenations are shown. Figure 6 refers to the process steps in figure 1 and figure 7 to the process steps of figure 4.

The left side of figure 6 shows in step c₀) the formation of ketals (*E-IK*) of (E)-3,7-dimethyloct-2-enal or (E)-3,7-dimethylocta-2,6-dienal (*E-I*), respectively (which are obtained after separation of the corresponding isomer mixture in step b)) using an alcohol (in figure 6 ethylene glycol is shown) in the presence of an acid. The ketal (*E-IK*) is then asymmetrically hydrogenated in step c) as discussed in figure 1. The direct product of this asymmetric hydrogenation is an asymmetrically hydrogenated ketal (*R-IIK*), which after acidic hydrolysis in step c') yields (R)-3,7-dimethyloctanal (*R-Ia*). On the right side of figure 6 the corresponding reaction scheme is shown for the Z-isomer, i.e. (Z)-3,7-dimethyloct-2-enal or (Z)-3,7-dimethylocta-2,6-dienal (*Z-I*), respectively, furnishing via the corresponding ketal intermediate (*Z-IK*) the same compound (R)-3,7-dimethyloctanal (*R-Ia*).

The left side of figure 7 shows in step fₒ) the formation of ketals (*E-R-IIIK*) of (R,E)-6,10,14-trimethylpentadec-5-en-2-one (*E-R-III*) obtained after isomer separation in step e) using an alcohol (in figure 7 ethylene glycol is shown) in the presence of an acid. The ketal (*E-R-IIIK*), preferably (R,E)-2-methyl-2-(4,8,12-trimethyltridec-3-en-1-yl)-1,3-dioxolane, is then asymmetrically hydrogenated in step f) as discussed in figure 4. The direct product of this asymmetric hydrogenation is an asymmetrically hydrogenated ketal, i.e. 2-methyl-2-((4R,8R)-4,8,12-trimethyltridecyl)-1,3-dioxolane (*R-IVK*), which after acidic hydrolysis in step f') yields (6R,10R)-6,10,14-trimethylpentadecan-2-one (*R-IV*). On the right side of figure 7 the corresponding reaction scheme is shown for the Z-isomer, i.e. (R,Z)-6,10,14-trimethylpentadec-5-en-2-one (*Z-R-III*), furnishing via the ketal intermediate, preferably (R,Z)-2-methyl-2-(4,8,12-trimethyltridec-3-en-1-yl)-1,3-dioxolane (*Z-R-IIIK*), the same compound (6R,10R)-6,10,14-trimethylpentadecan-2-one (*R-IV*).

### Examples

The present invention is further illustrated by the following experiments.

### Analytical Methods

### GC Determination of E/Z-ratio and/or purity of (R)-6,10,14-trimethylpentadec-5-en-2-one (R-THFA):

Agilent 6850, column DB-5HT (30 m, 0.25 mm diameter, 0.10 µm film thickness), 107 kPa helium carrier gas). The samples were injected as solutions in hexane, split ratio 300:1, injector temperature 200 °C, detector temperature 350 °C. Oven temperature program: 100 °C (8 min), 10 °C/min to 200 °C (1 min), 20 °C/min to 220 °C (4 min), runtime 24 min.

### GC Determination of purity of (6R, 10R)-6,10,14-trimethylpentadecan-2-one

Agilent 6850, column DB-5HT (30 m, 0.25 mm diameter, 0.10 µm film thickness), 115 kPa helium carrier gas). The samples were injected as solutions in hexane, split ratio 300:1, injector temperature 200 °C, detector temperature 350 °C. Oven temperature program: 120 °C (5 min), 14 °C/min to 260 °C (2 min), 20 °C/min to 280 °C (4 min), runtime 22 min.

### GC Determination of purity of (3RS,7R,11R)-3,7,11,15-tetramethylhexadec-1-en-3-ol((R,R)-Isophytol)

Agilent 6850 instrument equipped with FID. Agilent DB-5 column (30 m, 0.32 mm diameter, 0.25 µm film thickness) with 172.369 kPa (25 psi) molecular hydrogen carrier gas. The samples were injected as solutions in acetonitrile with a split ratio of 50:1. Injector temperature: 250 °C, detector temperature: 350 °C. Oven temperature program: 100 °C, 4 °C/min to 250 °C.

### GC Determination of E/Z-ratio and/or purity of (E)-3,7-dimethylocta-2,6-dienal (geranial) and ketals:

Agilent 6850 instrument, column Agilent DB-5 (123-5032E, 30 m x 0.32 mm, film 0.25 µm), the samples were injected as solutions in acetonitrile, split ratio 50:1, injector 250 °C, detector 350 °C. Oven temperature program: 100 °C, 4 °C/min until 250 °C, 37.5 min total runtime.

| *Retention times (t_{R}):* | min. |
|---|---|
| *Geranial* | 7.1 |
| *Geranial-neo* | 15.2 |
| *neral-neo* | 14.1 |
| *R,E-THFA-DM* | 24.2, pc¹ |
| *R,E-THFA-neo* | 29.1 |
| *R,Z-THFA-DM* | 23.1, pc¹ |
| *R,Z-THFA-neo* | 27.9 |
| *R-THGA-DM* | 13.1 |
| *R-Tetrahydrocitral-neo* | 12.6 |
| *R-THGA-neo* | 18.9 |
| *R-THGA-tfe* | 11.8 |
| *RR-C18-DM* | decomp.² |
| *RR-C18-neo* | 28.5 |
| *RR-C18-tfe* | 21.4 |

| | |
|---|---|
| *¹pc* = *partial decomposition* *²decomp.*= *decomposition during GC analysis* | |

### Analysis of the asymmetrically hydrogenated reaction products

The corresponding dimethyl, ethylene glycol, neopentyl and bis(trifluoroethyl) ketals were hydrolyzed to the ketones in the presence of aqueous acid and analyzed for conversion and their stereoisomer ratio using the following methods for ketones.

The conversion of the hydrogenation reaction was determined by gas chromatography using an achiral column.

### Method for Conversion:

Agilent 7890A GC equipped with FID. Agilent HP-5 column (30 m, 0.32 mm diameter, 0.25 µm film thickness) with 172.369 kPa (25 psi) molecular hydrogen carrier gas. The samples were injected as solutions in dichloromethane with a split ratio of 10:1. Injector temperature: 250 °C, detector temperature: 300 °C. Oven temperature program: 50 °C (2 min) then 15 °C/min to 300 °C, hold 5 min.

For the determination of the isomer ratio, the hydrogenated ketones can be reacted with either (+)-diisopropyl-*O*,*O*'-bis(trimethylsilyl)-L-tartrate or (-)-diisopropyl-*O*,*O*'-bis(trimethylsilyl)-D-tartrate in the presence of trimethylsilyl triflate [Si(CH₃)₃(OSO₂CF₃)] to form the diastereomeric ketals as described in A. Knierzinger, W. Walther, B. Weber, R. K. Müller, T. Netscher, Helv. Chim. Acta 1990, 73, 1087-1107. The ketals can be analysed by gas chromatography using an achiral column to determine the isomer ratios. For the hydrogenated ketone 6,10-dimethylundecan-2-one, either D-(-)- or L-(+)-diisopropyltartrate can be used. For 6,10,14-trimethylpentadecan-2-one, L-(+)-diisopropyltartrate can be used to measure the quantity of the (6*R*,10*R*)-isomer that was present. D-(-)-diisopropyltartrate can be used to determine the amount of the (6S,10S)-isomer. Thus the selectivity of the stereoselective hydrogenation can be determined indirectly.

### Method for determination of isomers:

Agilent 6890N GC with FID. Agilent CP-Sil88 for FAME column (60 m, 0.25 mm diameter, 0.20 µm film thickness) with 110.316 kPa (16 psi) molecular hydrogen carrier gas. The samples were injected as solutions in ethyl acetate with a split ratio of 5:1. Injector temperature: 250 °C, FID detector temperature: 250 °C. Oven temperature program: 165 °C (isothermal, 240 min)

The Ir complexes indicated in the following experiments are prepared according to the disclosure in Chem. Sci., 2010, 1, 72 - 78 .

### Experiment E1: Synthesis of (R)-6,10-dimethylundecan-2-one

### 1. Separation of 3,7-dimethylocta-2,6-dienal (step a/b)

(E)-3,7-dimethylocta-2,6-dienal (geranial) was separted from a mixture of geranial and neral by distillation.

### 2. Formation of neopentyl glycol ketals of (E)-3,7-dimethylocta-2,6-dienal (Geranial-neo) (step cₒ)

Tetramethyl orthocarbonate (0.675 g, 4.86 mmol, 98%, 1.48 eq.) was suspended in dichloromethane (10 mL) under argon at room temperature. 2,2-Dimethylpropane-1,3-diol (2.27 g, 21.8 mmol, 6.6 eq.) was added and the mixture was stirred for 2 h, after which a clear solution was obtained. Boron trifluoride etherate (0.372 mL, 0.422 g, 2.88 mmol, 97%, 0.90 eq.) was added and after stirring for 10 min at room temperature, the reaction was cooled to -78 °C. The reaction was diluted with dichloromethane (5 mL) and geranial (0.503 g, 3.29 mmol, 99.9%, 1.0 eq.) was added slowly and dropwise via a cooled syringe (using dry ice wrapped in a towel) over 10 min. Subsequently, the reaction was stirred at -78 °C or 1 h. GC analysis indicated full conversion. The reaction was quenched slowly with cooled triethylamine (4.0 mL, 2.91 g, 28.7 mmol, 8.7 eq.) at -78 °C. Subsequently, sat. aq. NaHCO₃ (6 mL) was added at -78 °C and the suspension was allowed to warm to room temperature. The reaction was diluted with pentane (50 mL) and the organic phase was washed with sat. aq. NaHCO₃ (2 x 50 mL), water (50 mL), dried over MgSO₄, filtered and concentrated in vacuo, furnishing (*E*)-2-(2,6-dimethylhepta-1,5-dien-1-yl)-5,5-dimethyl-1,3-dioxane (573 mg, 97.3% (GC), 71% yield, *E*/*Z* 97.6:2.4) as colorless liquid.

### Characterization data of (E)-2-(2,6-dimethylhepta-1,5-dien-1-yl)-5,5-dimethyl-1,3-dioxane (Geranial-neo):

¹H NMR (300 MHz, *CDCl₃*): δ 0.74 (s, 3 H), 1.23 (s, 3 H), 1.59 (s, 3 H), 1.68 (s, 3 H), 1.74 (d, *J* = 1.3 Hz, 3 H), 1.98-2.18 (m, 4 H), AB signal (δ_{A} = 3.51, δ_{B} = 3.65, *J*_{AB} = 11.0 Hz, 4 H), 5.06-5.15 (m, 1 H), 5.09 (d, *J* = 6.4 Hz, 1 H), 5.34 (dq, *J* = 6.2, 1.1 Hz, 1 H).
¹³C NMR (75 MHz, *CDCl₃*): δ 17.3 (1 C), 17.6 (1 C), 21.9 (1 C), 23.0 (1 C), 25.6 (1 C), 26.2 (1 C), 30.0 (1 C), 39.2 (1 C), 77.3 (2 C), 98.9 (1 C), 121.7 (1 C), 123.8 (1 C), 131.8 (1 C), 142.9 (1 C) ppm.
MS (EI, m/z): 238 (M⁺, 7), 237 [(M-H)⁺, 7], 223 [(M-CH₃)⁺, 5], 195 (15), 181 (6), 169 (30), 134 (12), 115 (30), 83 (26), 69 (100), 55 (30), 41 (57).
IR (cm⁻¹): 2953 (w), 2928 (w), 2845 (w), 1746 (w), 1680 (w), 1451 (w), 1393 (m), 1362 (w), 1305 (w), 1258 (w), 1232 (w), 1192 (w), 1142 (m), 1093 (s), 1041 (w), 1015 (m), 981 (s), 967 (s), 929 (m), 814 (w), 790 (w), 670 (w).

### 2. Asymmetric hydrogenations of geranial / geranial ketal (step c)

An autoclave was charged with 0.5 mmol (*E*)-2-(2,6-dimethylhepta-1,5-dien-1-yl)-5,5-dimethyl-1,3-dioxane or (*E*)-geranial, and with 4 g of dichloromethane and a solution of 2 mol% of the chiral iridium complex of formula (III-F) having the S-chirality at the centre indicated by * in said formula. The autoclave was closed and a pressure of 30 bar of molecular hydrogen was applied. The reaction mixture was stirred for 16 hours at 40 °C. Afterwards the pressure was released and the solvent was removed. In case of hydrogenation of (E)-2-(2,6-dimethylhepta-1,5-dien-1-yl)-5,5-dimethyl-1,3-dioxane analysis by GC showed full conversion and a hydrogenated acetal purity of 71%, while only 2% product was observed in the (E)-geranial case.

### Characterization data of (R)-2-(2,6-dimethylheptyl)-5,5-dimethyl-1,3-dioxane (R-Tetrahydrocitral-neo):

¹H NMR (300 MHz, *CDCl₃*): δ 0.72 (s, 3 H), 0.87 (d, *J* = 6.6 Hz, 6 H), 0.91 (d, *J* = 6.4 Hz, 3 H), 1.06-1.18 (m, 2 H), 1.20 (s, 3 H), 1.22-1.78 (m, 8 H), 3.43 (dd, *J* = 11.2, 1.4 Hz, 2 H), 3.61 (d, *J* = 11.2 Hz, 2 H), 4.48 (t, *J* = 5.2 Hz, 1 H) ppm.
¹³C NMR (75 MHz, *CDCl₃*): δ 19.7 (1 C), 21.9 (1 C), 22.6 (1 C), 22.7 (1 C), 23.1 (1 C), 24.5 (1 C), 27.9 (1 C), 28.6 (1 C), 29.7 (1 C), 30.1 (1 C), 37.5 (1 C), 39.2 (1 C), 42.1 (1 C), 77.3 (1 C), 101.4 (1 C) ppm.
MS (EI, m/z): 241.3 [(M-H)⁺, 10], 200 (1), 155 (4), 130 (4), 115 (100), 69 (35), 56 (21), 41 (19).
IR (cm⁻¹): 2953 (s), 2924 (s), 2854 (m), 1744 (w), 1645 (w), 1461 (m), 1406 (w), 1393 (w), 1378 (w), 1284 (m), 1252 (w), 1230 (w), 1162 (s), 1122 (s), 1083 (m), 1044 (w), 1017 (w), 974 (m), 932 (w), 921 (w), 890 (w), 837 (w), 735 (w), 666 (w).

### 3. Hydrolysis of (R)-2-(2,6-dimethylheptyl)-5,5-dimethyl-1,3-dioxane

A sample of the hydrogenated acetal (30 mg) ((*R)-2-(2,6-dimethylheptyl)-5,5-dimethyl-1,3-dioxane (R-Tetrahydrocitral-neo))* was dissolved in formic acid (3 mL) under argon, treated with sodium formate (50 mg) and heated to 70 °C for 1 h. After cooling, the reaction was diluted with water (10 mL) and EtOAc (20 mL). The organic phase was washed with brine (5 mL), dried over MgSO₄ and concentrated. (R)-3,7-dimethyloctanal (*R-Tetrahydrocitral*) was analyzed for its stereoisomer ratio.

### 4. Synthesis of (R)-6,10-dimethylundec-3-en-2-one (step d1')

A 25 ml round bottom flask, equipped with stir bar and a argon inlet was charged with (R)-3,7-dimethyloctanal (1.00 g, 6.08 mmol, 1.0 eq., 95%), acetone (4.48 g, 77.1 mmol, 12.7 eq.) and a solution of NaOH (43 mg, 1.07 mmol, 17 mol%) in distilled water (1.67 mL). The reaction was heated to 45°C under argon for 24 h. Subsequently, the reaction was allowed to cool to room temperature and neutralized with acetic acid (51 µL, 0.90 mmol, 15 mol%). The reaction was diluted with pentane (50 mL) and water (50 mL). The aqueous phase was extracted with pentane (2 x 50 mL) and the combined organic phases were washed with water (50 mL) and saturated NaCl solution (50 mL). The organic phase was dried over MgSO4 and concentrated. The crude material (1.13 g) was purified by distillation in a Kugelrohr apparatus at 120 °C/7.1·10⁻² mbar, furnishing (R)-6,10-dimethylundec-3-en-2-one as colorless oil (0.703 g, 88 a% by GC, 52% yield).

### 5. Synthesis of (R)-6,10-dimethylundecan-2-one (step d2')

Palladium on carbon (5%, 10 mg) was added to a solution of (R)-6,10-dimethylundec-3-en-2-one (150 mg, 88%) in heptane (2 g) in a glass liner. The liner was placed in the reactor, the reactor was sealed and purged 3 times with nitrogen (pressurised to 6 bar, then vent) and then purged 3 times with hydrogen (pressurised to 6 bar then vent). The reactor was heated to 25 °C under stirring at 500 rpm. Then the stirring rate was increased to 1000 rpm and the reactor pressurised with 6 bar hydrogen. The reactor was stirred until the desired hydrogen uptake was obtained, then cooled, vented and purged once with nitrogen. The mixture was filtered, and evaporated to give (R)-6,10-dimethylundecan-2-one (144 mg). GC analysis showed 89% purity.

Analysis of the isomer ratio of the product showed a 88:12 ratio (R):(S) which is consistent with the starting composition.

An identical reaction with palladium on alumina (5%, 10mg) gave a crude product (146 mg) with a GC purity of 90%. Analysis of the isomer ratio of the product showed a 88:12 ratio (R):(S) which is consistent with the starting composition.

### Experiment E3a: Vinylation of (R)-6,10-dimethylundecan-2-one (step d3')

A dried 100 mL four-necked flask equipped with overhead stirrer, thermometer, condenser and argon inlet was evacuated and purged with argon. Vinylmagnesium chloride (23.63 mL of a 1.6 M solution in THF, 37.8 mmol, 1.56 eq.) was added at room temperature. A solution (R)-6,10-dimethylundecan-2-one (5.01 g, 24.24 mmol, 96.0%, 1.0 eq.) in dry THF (20 mL) was added slowly within 20 min. The exothermic reaction was maintained between 25 and 30 °C internal temperature by cooling with an ice bath. After complete addition the reaction was allowed to stir at room temperature for 1 h. Saturated NH₄Cl solution (10 mL) was added carefully to quench excess Grignard reagent. Pentane (150 mL), water (150 mL) and brine (150 mL) was added. The organic phase was extracted with brine (2 x 150 mL) and the aqueous phase was back-extracted with pentane (2 x 150 mL). The combined organic phases were dried (MgSO₄) and concentrated in vacuo, resulting in a colorless oil (5.42 g). The crude product was purified by vacuum distillation in a Kugelrohr apparatus. The main fraction distilled at 143 °C/3.8⁻² mbar, furnishing (7R)-3,7,11-trimethyldodec-1-en-3-ol as colorless oil with a purity of 94.0% (5.15 g, 21.38 mmol, 88% yield).

### Experiment E3b: Ethynylation of (R)-6,10-dimethylundecan-2-one (step d3)

(R)-6,10-dimethylundecan-2-one (340 g, 1.70 mol, 1.0 eq., 99.0%) was added to an autoclave equipped with thermostat, dosing pump, acetylene inlet and ammonia inlet. The reactor was sealed, evacuated and flushed with nitrogen and cooled to 15 °C. Ammonia (632 g, 37.2 mol, 22.0 eq., 99.8%) was condensed into the reactor and cooled to 15 °C, resulting in a pressure of 8-9 bar. Acetylene was introduced until 12 bar pressure was reached, followed by a dosed addition of KOH (45 wt.-% in water, 6.6 g, 52.9 mmol, 3.1 mol%) at 15 °C. The reaction progress was monitored by GC. After 90 min the reaction mixture was neutralized with acetic acid, and the reactor was subsequently vented at 25 °C. The reaction mixture was washed and concentrated in vacuo and purified by distillation in vacuo furnishing 325 g of (7R)-3,7,11-trimethyldodec-1-yn-3-ol with a purity of 95% (81% yield).

### Experiment E3c: Hydrogenation of (7R)-3,7,11-trimethyldodec-1-yn-3-ol (step d4)

(7R)-3,7,11-trimethyldodec-1-yn-3-ol (910 g, 4.06 mol, 1.0 eq., 95%), Lindlar catalyst catalyst (850 mg) were placed into an autoclave. The reactor was sealed, evacuated, flushed with nitrogen and subsequently heated to 45 °C. The reactor was evacuated once more and flushed with hydrogen, pressurized to 2 bar. The reaction was stirred for approx. 2-3 hours at 45 °C until the calculated amount of molecular hydrogen had been consumed. After filtration 884 g of (7R)-3,7,11-trimethyldodec-1-en-3-ol was obtained with a purity of 91.5% (88 % yield).

### Experiment E4: Reaction of (7R)-3,7,11-trimethyldodec-1-en-3-ol with 2-methoxyprop-1-ene (step d5)

(7R)-3,7,11-trimethyldodec-1-en-3-ol (640 g, 2.59 mol, 1.0 eq., 91.5%), H₃PO₄ (20 wt% in water, 4.5 g, 8.2 mmol, 0.32 mol%) and isopropenyl methyl ether (600 g, 8.17 mol, 3.2 eq., 98.0%) were placed into a 2 L autoclave equipped with thermostat, overhead stirrer and 1.1 m distillation column (Sulzer packing). The reactor was sealed, evacuated and flushed with nitrogen and subsequently heated to 80 °C. The pressure that built up was slowly vented. The inner temperature was then gradually raised to 160 °C within 1 h. The reaction was stirred for 3 h at 160 °C. The reaction mixture was cooled to 30 °C, concentrated in vacuo and washed with water and NaHCO₃ solution. Then low boilers were removed by distillation (jacket temperature 150 °C, 1 mbar), furnishing 690 g of a mixture of (R,E)-6,10,14-trimethylpentadec-5-en-2-one and (R,Z)-6,10,14-trimethylpentadec-5-en-2-one as residue with a purity of 85% (85% yield). The mixture was analyzed by GC to be a mixture of 49 % E-isomer and 36 % Z-isomer.

### Experiment E5: Separation of E/Z isomer mixtures of (R,Z)-6,10,14-trimethyl-pentadec-5-en-2-one (step e)

The mixture of (R,E)-6,10,14-trimethylpentadec-5-en-2-one and (R,Z)-6,10,14-trimethylpentadec-5-en-2-one 1.94 kg, 36 % (R,Z)-6,10,14-trimethylpentadec-5-en-2-one and 49 % (R,E)-6,10,14-trimethylpentadec-5-en-2-one was fractionated using the separation equipment consisting of a still (volume: 9 litre) with a falling film evaporator, a rectifying column (70 mm inner diameter, height 5 m). The column was equipped with a very efficient structured packing (Sulzer). The rectification process was operated at a top pressure of approx. 2 mbar and at a column top temperature varied in the range from 95 to 122°C and the bottom temperature in the still was 165°C. The reflux ratio was adjusted to 20. Fractionation of the distillate stream furnished fractions containing (R,Z)-6,10,14-trimethylpentadec-5-en-2-one (content of Z-isomer = 97%). At the end (R,E)-6,10,14-trimethylpentadec-5-en-2-one was found left in the still (content of E-isomer = 94%). Both isomers were further purified by fractionation and furnished fractions of both E-isomer and Z-isomer each with a purity of 99.5%)

### Experiment E6: Asymmetric hydrogenations of (R,E)-6,10,14-trimethylpentadec-5-en-2-one and (R,Z)-6,10,14-trimethylpentadec-5-en-2-one (step f)

Both isomers (R,E)-6,10,14-trimethylpentadec-5-en-2-one) (*R,E-THFA*) (E/Z= 99.5/0.5, R/S= 92/8) and (R,Z)-6,10,14-trimethylpentadec-5-en-2-one (*R,Z-THFA*) (Z/E= 99.5/0.5, R/S= 92/8) were hydrogenated asymmetrically, separate from each other in the following manner:
A 125 mL autoclave was charged with 7.0 g (26 mmol) of the specific isomer, 50 mL of 2,2,2-trifluoroethanol and a solution of the chiral iridium complex of formula (III-F) having the chirality given in table 3 at the centre indicated by * in said formula (42 mg, 0.026 mmol, 0.1 mol%) in anhydrous dichloromethane (4 g). The autoclave was closed and a pressure of 50 bar of molecular hydrogen was applied. The reaction mixture was heated to 30 °C whilst stirring for 16 hours. Afterwards the pressure was released and the solvent removed. The product formed is (6R,10R)-6,10,14-trimethylpentadecan-2-one. The conversion as well as the amount of isomers formed is given in table 3.

The products of the two separate asymmetric hydrogenations have been combined.

In a further experiment in an autoclave 0.25 mmol of (R,E)-6,10,14-trimethylpentadec-5-en-2-one) (*R,E-THFA*) and 1 mol-%, of the Ir complex of the formula (III-D) and 1.25 ml of absolute (dry) dichloromethane were put. The autoclave was closed and a pressure of 50 bar of molecular hydrogen was applied. Under stirring the reaction solution was kept at room temperature for 14 hours. Afterwards the pressure was released and the solvent removed. For determining the conversion the crude product was analysed by achiral gas chromatography without any further purification. The amount for the isomers has been determined using the above method and given in table 3.

In a further experiment in an autoclave 0.5 mmol of (R,E)-6,10,14-trimethylpentadec-5-en-2-one) (*R,E-THFA*) and 1 mol-%, of the Ir complex of the formula as indicated in table 3' and 4 g solvent as indicated in table 3' were put. The autoclave was closed and a pressure of 30 bar of molecular hydrogen was applied. Under stirring the reaction solution was kept at room temperature for 16 hours. Afterwards the pressure was released and the solvent removed. For determining the conversion the crude product was analysed by achiral gas chromatography without any further purification. The amount for the isomers has been determined using the above method and given in table 3'.

**Table 3: Asymmetric hydrogenation of R,E-THFA and R,Z-THFA.**

| | ***1*** | ***2*** | ***3*** |
|---|---|---|---|
| | *R,Z- THFA* | *R,E- THFA* | *R,E- THFA* |
| Formula of Ir-complex | III-F | III-F | III-D |
| Configuration of chiral Ir-complex | R | S | S |
| Amount of chiral Ir complex [mol-%] | 0.1 | 0.1 | 1 |
| Conversion [%] | >99 | >99 | 100 |
| (6R,10R)-6,10,14-trimethylpentadecan-2-one [%] | 87.0 | 88.4 | 97.0 |
| (6S,10R)-6,10,14-trimethylpentadecan-2-one [%] | 5.3 | 3.7 | 1.8 |
| (6R,10S)-6,10,14-trimethylpentadecan-2-one [%] | 7.7 | 7.9 | 1.2* |
| (6S,10S)-6,10,14-trimethylpentadecan-2-one [%] | 0.0 | 0.0 | |

| | | | |
|---|---|---|---|
| *: (6R,10S) and (6S,10S) isomers determined as sum. | | | |

**Tab. 3': Asymmetric hydrogenation of R,E-THFA and R,Z-THFA with different Ir complexes.**

| | ***4*** | ***5*** | ***6*** | ***7*** |
|---|---|---|---|---|
| | *R,E-THFA* | *R,E-THFA* | *R,E-THFA* | *R,E-THFA* |
| Formula of Ir complex | III-C | III-D | III-D | III-A' ² |
| Configuration of chiral Ir complex at * | S | R | S | S |
| Amount of chiral Ir complex [mol-%] | 1 | 1 | 1 | 1 |
| Solvent¹ | DCM | TFE | DCM | DCM |
| Conversion [%] | >99 | >99 | >99 | >99 |

| Isomer-Distribution | | | | |
|---|---|---|---|---|
| (6R,10R)-6,10,14-trimethylpentadecan-2-one [%] | 97.3 | 0.9 | 96.9 | 96.6 |
| (6S,10R)-6,10,14-trimethylpentadecan-2-one [%] | 1.3 | 97.0 | 1.8 | 2.0 |
| (6R,10S)-6,10,14-trimethylpentadecan-2-one [%] | 1.4 | 0 | 1.3 | 1.4 |
| (6S,10S)-6, 10, 14-trimethylpentadecan-2-one [%] | 0 | 2.1 | 0 | 0 |

| | | | | |
|---|---|---|---|---|
| ¹TFE = 2,2,2-trifluoroethanol; DCM = dichloromethane ²chiral Ir complex of formula (III-A'): | | | | |

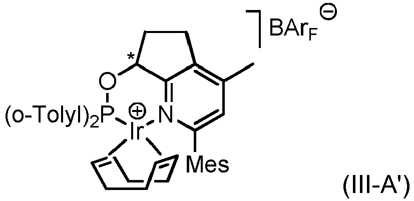

### Experiment E6a: Preparation of ketals of (R,E)-6,10,14-trimethylpentadec-5-en-2-one and (R,Z)-6,10,14-trimethylpentadec-5-en-2-one (step fₒ)

### a) Preparation of dimethyl ketals

(R,E)-6,10,14-trimethylpentadec-5-en-2-one or (R,Z)-6,10,14-trimethylpentadec-5-en-2-one (170.5 mmol) was added to trimethyl orthoformate (50.8 mL, 49.2 g, 451 mmol, 2.65 eq.) and cooled to 5 °C. Sulfuric acid (96%, 32.3 mg, 0.29 mmol, 0.2 mol%) in MeOH (16 mL) was added within 5 min. Subsequently, the reaction was heated to reflux (65 °C IT) for 3 h. After cooling, thin layer chromatography (TLC) analysis indicated full conversion. NaOMe (0.24 mL of a 25% solution in MeOH) was added to neutralize the acid. The mixture was concentrated in vacuo and subsequently diluted with hexane (50 mL). The developed precipitate was filtered off and the filtrate was concentrated. The crude product was purified by distillation, furnishing the desired dimethyl ketal.

### b) Preparation of neopentyl glycol ketals

(R,E)-6,10,14-trimethylpentadec-5-en-2-one or (R,Z)-6,10,14-trimethylpentadec-5-en-2-one (90.7 mmol), 2,2-dimethyl-1,3-propanediol (neopentylglycol, 32.4 g, 283 mmol, 3.4 eq.) and p-toluene sulfonic acid monohydrate (60 mg, 0.31 mmol, 0.3 mol%) were suspended in toluene (300 mL). The reaction was heated to 90 °C upon which a homogeneous solution formed. Subsequently, at 75 °C, vacuum was applied cautiously (first 63 mbar, then 24 mbar) in order to slowly distill toluene off (approx. 100 mL over 4 h). After 4 h, thin layer chromatography (TLC) analysis indicated full conversion of the ketone. The reaction was allowed to cool to room temperature and diluted with heptane (300 mL) upon which excess neopentylglycol precipitated. The precipitate was filtered off (17.4 g wet). The filtrate was treated with Et₃N (1 mL), subsequently washed with aqueous NaHCO₃ solution (2.4% w/w, 2 x 300 mL), dried over MgSO₄ and concentrated in vacuo. The crude product was purified by distillation, furnishing the desired neopentyl ketal.

**Tab. 3a: Preparation of dimethyl and neopentyl glycol ketals of 6,10,14-trimethylpentadec-5-en-2-one .**

| | *R-E-THFA-DM* | *R-Z-THFA-DM* | *R-E-THFA-neo* | *R-Z-THFA-neo* |
|---|---|---|---|---|
| Ketone | (R,E)-6,10,14-trimethylpentadec-5-en-2-one | (R,Z)-6,10,14-trimethylpentadec-5-en-2-one | (R,E)-6,10,14-trimethylpentadec-5-en-2-one | (R,Z)-6,10,14-trimethylpentadec-5-en-2-one |
| Ketal | (R,E)-2,2-dimethoxy-6,10,14-trimethylpentadec-5-ene | (R,Z)-2,2-dimethoxy-6,10,14-trimethylpentadec-5-ene | (R,E)-2,5,5-trimethyl-2-(4,8,12-trimethyltridec-3-en-1-yl)-1,3-dioxane | (R,Z)-2,5,5-trimethyl-2-(4,8,12-trimethyltridec-3-en-1-yl)-1,3-dioxane |
| Yield [%] | 92 | 87 | 83 | 86 |
| E/Z | 99.5/0.5 | 3.6/96.4 | 99.8/0.2 | 4/96 |

Characterization data:
(R,E)-2,2-dimethoxy-6,10,14-trimethylpentadec-5-ene (*R-E-THFA-DM)*
   ¹H NMR (300 MHz, CDCl₃): δ 0.84 (d, J = 6.6 Hz, 3 H), superimposed by 0.86 (d, J = 6.6 Hz, 6 H), 0.99-1.44 (m, 11 H), superimposed by 1.28 (s. 3H), 1.52 (tqq, J = 6.6, 6.6, 6.6 Hz, 1 H), 1.60 (s, 3 H), 1.60-1.66 (m, 2 H), 1.90-2.05 (m, 4 H), 3.18 (s, 6 H), 5.10 (tq, J = 7.1, 1.1 Hz, 1 H) ppm.
   ¹³C NMR (75 MHz, CDCl₃): δ 16.3 (1 C), 20.1 (1 C), 21.3 (1 C), 23.0 (1 C), 23.1 (1 C), 23.2 (1 C), 25.2 (1 C), 25.7 (1 C), 28.4 (1 C), 33.1 (1 C), 36.9 (1 C), 37.1 (1 C), 37.7 (1 C), 39.8 (1 C), 40.3 (1 C), 48.4 (2 C), 101.9 (1 C), 124.0 (1 C), 136.0 (1 C) ppm.
   MS (EI, m/z): No GC-MS was obtained due to decomposition on the column.
   IR (cm⁻¹): 2952 (m), 2927 (s), 2869 (m), 2828 (w), 1461 (m), 1377 (m), 1301 (w), 1262 (m), 1222 (m), 1197 (m), 1172 (m), 1120 (m), 1101 (m), 1076 (m), 1054 (s), 930 (w), 854 (m), 737 (w), 620 (w).
(R,Z)-2,2-dimethoxy-6,10,14-trimethylpentadec-5-ene (*R-Z-THFA-DM)*
   ¹H NMR (300 MHz, CDCl₃): δ 0.85 (d, J = 6.4 Hz, 3 H), superimposed by 0.87 (d, J = 6.4 Hz, 6 H), 1.01-1.27 (m, 7 H), 1.28 (s, 3 H), 1.29-1.44 (m, 4 H), 1.53 (dqq, J = 6.5, 6.5 Hz, 6.5 Hz, 1 H), 1.58-1.66 (m, 2 H), 1.68 (q, J = 1.1 Hz, 3 H), 1.91-2.08 (m, 4 H), 3.18 (s, 6 H), 5.11 (t, J = 6.8 Hz, 1 H) ppm.
   ¹³C NMR (75 MHz, CDCl₃): δ) 19.7 (1 C), 20.9 (1 C), 22.60 (1 C), 22.69 (1 C), 22.71 (1 C), 23.4 (1 C), 24.8 (1 C), 25.5 (1 C), 28.0 (1 C), 32.0 (1 C), 32.7 (1 C), 36.8 (1 C), 37.0 (1 C), 37.3 (1 C), 39.3 (1 C), 48.0 (2 C), 101.5 (1 C), 124.3 (1 C), 135.9 (1 C) ppm.
   MS (EI, m/z): No GC-MS was obtained due to decomposition on the column.
   IR (cm⁻¹): 2952 (m), 2927 (m), 2869 (m), 2828 (w), 1462 (m), 1376 (m), 1301 (w), 1261 (w), 1197 (w), 1172 (m), 1119 (m), 1098 (m), 1074 (m), 1054 (s), 1022 (w), 854 (m), 736 (w), 622 (w).
(R,E)-2,5,5-trimethyl-2-(4,8,12-trimethyltridec-3-en-1-yl)-1,3-dioxane (*R-E-THFA-neo)*
   ¹H NMR (300 MHz, CDCl₃): δ 0.84 (d, J = 6.4 Hz, 3 H), 0.86 (d, J = 6.6 Hz, 6 H), 0.92 (s, 3 H), 0.99 (s, 3 H), superimposed by 0.97-1.44 (m, 11 H), superimposed by 1.37 (s, 3 H), 1.52 (qqt, J = 6.9, 6.9, 6.9 Hz, 1 H), 1.60 (s, 3 H), 1.67-1.76 (m, 2 H), 1.93 (t, J = 7.4 Hz, 2 H), 2.03-2.18 (m, 2 H), AB signal (δ_{A} = 3.45, δ_{B} = 3.52, J_{AB} = 11.4 Hz, 4 H), 5.12 (tq, J = 7.2, 1.0 Hz, 1 H) ppm.
   ¹³C NMR (75 MHz, CDCl₃): δ 15.8 (1 C), 19.7 (1 C), 20.9 (1 C), 22.0 (1 C), 22.6 (2 C), 22.7 (2 C), 24.8 (1 C), 25.3 (1 C), 27.9 (1 C), 29.9 (1 C), 32.6 (1 C), 36.6 (1 C), 37.3 (1 C), 37.4 (1 C), 39.3 (1 C), 39.9 (1 C), 70.3 (2 C), 98.8 (1 C), 123.8 (1 C), 135.5 (1 C) ppm.
   MS (EI, m/z): 352 (M⁺, 4), 337 [(M-CH₃)⁺, 8), 265 (6), 129 (100), 95 (10), 69 (25), 43 (25).
   IR (cm⁻¹): 2953 (s), 2926 (s), 2867 (m), 1462 (m), 1394 (w), 1369 (m), 1270 (w), 1249 (m), 1211 (m), 1187 (w), 1119 (s), 1088 (s), 1043 (m), 1021 (m), 951 (w), 925 (w), 907 (w), 862 (m), 791 (w), 738 (w), 678 (w).
(R,Z)-2,5,5-trimethyl-2-(4,8,12-trimethyltridec-3-en-1-yl)-1,3-dioxane (*R-Z-THFA-neo)*
   ¹H NMR (300 MHz, CDCl₃): δ 0.84 (d, J = 6.4 Hz, 3 H), superimposed by 0.86 (d, J = 6.6 Hz, 6 H), 0.93 (s, 3 H), 0.97 (s, 3 H), 1.00-1.42 (m, 11 H), superimposed by 1.36 (s, 3 H), 1.52 (qqt, J = 6.7, 6.7, 6.7 Hz, 1 H), 1.63-1.76 (m, 2 H), 1.67 (s, 3 H), 1.94-2.15 (m, 4 H), AB signal (δ_{A} = 3.45, δ_{B} = 3.51, J_{AB} = 11.1 Hz, 4 H), 5.12 (t, J = 7.1 Hz, 1 H) ppm.
   ¹³C NMR (75 MHz, CDCl₃): δ 19.6 (1 C), 21.1 (1 C), 21.9 (1 C), 22.60 (2 C), 22.67 (2 C), 22.69 (1 C), 23.4 (1 C), 24.8 (1 C), 25.4 (1 C), 27.9 (1 C), 29.9 (1 C), 32.0 (1 C), 32.7 (1 C), 36.9 (1 C), 37.3 (1 C), 39.3 (1 C), 70.3 (2 C), 98.8 (1 C), 124.6 (1 C), 135.7 (1 C) ppm.
   MS (EI, m/z): 352 (M⁺, 3), 337 [(M-CH₃)⁺, 9), 265 (6), 129 (100), 95 (10), 69 (24), 43 (25).
   IR (cm⁻¹): 2953 (s), 2926 (s), 2860 (m), 1463 (m), 1394 (w), 1371 (m), 1270 (w), 1250 (w), 1211 (m), 1188 (w), 1117 (s), 1086 (s), 1043 (m), 1022 (w), 951 (w), 925 (w), 907 (w), 855 (m), 792 (w), 737 (w), 667 (w).

### Experiment E6b: Asymmetric hydrogenations of ketals of (R,E)-6,10,14-trimethylpentadec-5-en-2-one and (R,Z)-6,10,14-trimethylpentadec-5-en-2-one

An autoclave was charged with 0.5 mmol of ketals of (R,E)-6,10,14-trimethylpentadec-5-en-2-one and (R,Z)-6,10,14-trimethylpentadec-5-en-2-one as indicated in tables 3b and 3c, and with 4g of the solvent as indicated in tables 3b and 3c and a solution of the chiral iridium complex of formula (III-F) having the chirality given in tables 3b and 3c at the centre indicated by * in said formula in the amount indicated in tables 3b and 3c. The autoclave was closed and a pressure of 30 bar of molecular hydrogen was applied. The reaction mixture was stirred for 16 hours at room temperature. Afterwards the pressure was released and the solvent was removed.

Hydrogenation of (R,E)-6,10,14-trimethyl-2,2-bis(2,2,2-trifluoroethoxy)pentadec-5-ene yielded (6R,10R)-6,10,14-trimethyl-2,2-bis(2,2,2-trifluoroethoxy)pentadecane using the Ir complex of formula (III-F) of the S configuration at the chiral centre marked by *.

The characterization of the hydrogenated ketals is given hereafter.

**Tab. 3b: Asymmetric hydrogenation of different ketals of (R,E)-6,10,14-trimethylpentadec-5-en-2-one leading to (6R,10R)-6,10,14-trimethyl-pentadecan-2-one.**

| | ***8*** | ***9*** | ***10*** |
|---|---|---|---|
| Ketal to be hydrogenated | *R-E-THFA-DM* | *R-E-THFA-DM* | *R-E-THFA-neo* |
| Formula of Ir complex | *III-F* | *III-F* | *III-F* |
| Configuration of chiral Ir complex at * | (S) | (S) | (S) |
| Amount of chiral Ir complex [mol-%] | 0.25 | 0.25 | 0.5 |
| Solvent¹ | DCM | TFE | DCM |
| Conversion [%] | >99 | >99 | >99 |

| Isomer-Distribution^{2,3} | | | |
|---|---|---|---|
| (RR) [%] | 90.0 | 88.7 | 90.6 |
| ((SS)+(RS)) [%] | 8.0 | 8.7 | 9.4 |
| (SR) [%] | 2.0 | 2.6 | 0.0 |

| | | | |
|---|---|---|---|
| Conditions: 0.5 mmol ketal, 4 g solvent, pressure p(H₂)=30 bar, 16 h stirring at room temperature ¹ TFE = 2,2,2-trifluoroethanol; DCM = dichloromethane ² (SS) stands for the (6S,10S)-isomer, (RR) stands for the (6R,10R)-isomer, (SR) stands for the (6S,10R)-isomer, (RS) stands for the (6R,10S)-isomer of the corresponding ketal of 6,10,14-trimethyl-pentadecan-2-one ³ is determined as ketone after hydrolysis of the ketal | | | |

**Tab. 3c: Asymmetric hydrogenation of different ketals of (R,Z)-6,10,14-trimethylpentadec-5-en-2-one leading to (6R,10R)-6,10,14-trimethyl-pentadecan-2-one.**

| | ***11*** | ***12*** | ***13*** | ***14*** |
|---|---|---|---|---|
| Ketal to be hydrogenated | *R-Z-THFA-DM* | *R-Z-THFA-DM* | *R-Z-THFA-neo* | *R-Z-THFA-neo* |
| Formula of Ir complex | *III-F* | *III-F* | *III-F* | *III-F* |
| Configuration of chiral Ir complex at * | (R) | (R) | (R) | (R) |
| Amount of chiral Ir complex [mol-%] | 0.5 | 0.5 | 0.5 | 0.5 |
| Solvent¹ | DCM | TFE | DCM | TFE |
| Conversion [%] | >99 | >99 | >99 | >99 |

| Isomer-Distribution^{2,3} | | | | |
|---|---|---|---|---|
| (RR) [%] | 86.3 | 87.4 | 86.8 | 85.5 |
| ((SS)+(RS)) [%] | 8.2 | 7.5 | 8.2 | 9.4 |
| (SR) [%] | 5.5 | 5.1 | 5.0 | 5.1 |

| | | | | |
|---|---|---|---|---|
| Conditions: 0.5 mmol ketal, 4 g solvent, pressure p(H₂)=30 bar, 16 h stirring at room temperature ¹ TFE = 2,2,2-trifluoroethanol; DCM = dichloromethane ² (SS) stands for the (6S,10S)-isomer, (RR) stands for the (6R,10R)-isomer, (SR) stands for the (6S,10R)-isomer, (RS) stands for the (6R,10S)-isomer of the corresponding ketal of 6,10,14-trimethyl-pentadecan-2-one ³ is determined as ketone after hydrolysis of the ketal. | | | | |

Characterization data:
(6R,10R)-2,2-dimethoxy-6,10,14-trimethylpentadecane (*RR18-DM*)
   ¹H NMR (300 MHz, CDCl₃): δ 0.83-0.89 (m, 12 H), 0.98-1.45 (m, 21 H), 1.46-1.65 (m, 3 H), 3.18 (s, 6 H).
   ¹³C NMR (75 MHz, CDCl₃): δ 19.68 (1 C), 19.73 (1 C), 21.0 (1 C), 21.7 (1 C), 22.6 (1 C), 22.7 (1 C), 24.5 (1 C), 24.8 (1 C), 28.0 (1 C), 32.72 (1 C), 32.78 (1 C), 36.8 (1 C), 37.28 (1 C), 37.33 (1 C), 37.36 (1 C), 37.41 (1 C), 39.4 (1 C), 48.0 (2 C), 101.7 (1 C) ppm.
   IR (cm⁻¹): 2951 (s), 2926 (s), 2869 (s), 2828 (m), 1734 (w), 1723 (w), 1216 (w), 1463 (s), 1377 (s), 1308 (w), 1255 (m), 1215 (m), 1172 (s), 1105 (s), 1090 (s), 1054 (s), 971 (w), 933 (w), 860 (s), 815 (m), 736 (w) 618 (w).
2,5,5-trimethyl-2-((4R,8R)-4,8,12-trimethyltridecyl)-1,3-dioxane (*RR18-neo*)
   ¹H NMR (300 MHz, CDCl₃): δ 0.78-0.95 (m, 15 H), 0.95-1.61 (m, 19 H), superimposed by 1.01 (s, 3H), 1.36 (s, 3H), 1.63-1.74 (m, 2 H), AB signal (δ_{A} = 3.44, δ_{B} = 3.55, J_{AB} = 11.7 Hz, 4 H) ppm.
   ¹³C NMR (75 MHz, CDCl₃): δ 19.72 (1 C), 19.74 (1 C), 20.4 (1 C), 20.9 (1 C), 22.56 (1 C), 22.62 (1 C), 22.72 (1 C), 22.77 (1 C), 24.5 (1 C), 24.8 (1 C), 28.0 (1 C), 30.0 (1 C), 32.8 (1 C), 32.8 (1 C), 37.28 (1 C), 37.35 (1 C), 37.42 (2 C), 38.2 (1 C), 39.4 (1 C), 70.3 (2 C), 99.1 (1 C) ppm.
   MS (EI, m/z): 339 [(M-CH₃)⁺, 83], 269 (5), 129 (100), 69 (21), 43 (18).
   IR (cm⁻¹): 2952 (s), 2925 (s), 2867 (m), 1463 (m), 1394 (m), 1372 (m), 1258 (m), 1211 (m), 1189 (w), 1141 (w), 1100 (s), 1043 (m), 1020 (m), 951 (w), 925 (w), 907 (m), 858 (m), 792 (w), 737 (w), 677 (w).

### Experiment E6c: Hydrolysis of hydrogenated ketals of (R,E)-6,10,14-trimethylpentadec-5-en-2-one and (R,Z)-6,10,14-trimethylpentadec-5-en-2-one

After the asymmetric hydrogenation of the corresponding ketals of (R,E)-6,10,14-trimethylpentadec-5-en-2-one and (R,Z)-6,10,14-trimethylpentadec-5-en-2-one, the hydrogenated ketals obtained were hydrolyzed to the ketone and yielded (6R,10R)-6,10,14-trimethylpentadecan-2-one.

The hydrogenated ketals have been hydrolyzed according to the following methods:

### Method 1 - Neopentyl ketals, dimethyl ketals from asymmetric hydrogenation reactions in dichloromethane

A sample of the reaction mixture from the asymmetric hydrogenation reaction (1-2 ml) was stirred with an equal volume of 1M aqueous solution of hydrochloric acid at room temperature for 1 hour. Dichloromethane (2 ml) was added and the layers were separated. The aqueous layer was washed with dichloromethane (2ml) twice. The combined organic layers were evaporated under reduced pressure to yield the ketone as a colourless to pale-yellow oil. The crude ketone was then analysed for purity and isomer ratio and identified to be (6R,10R)-6,10,14-trimethylpentadecan-2-one.

### Method 2 - Ethylene glycol ketals and dimethyl ketals from asymmetric hydrogenation reactions in trifluoroethanol

A sample of the reaction mixture from the asymmetric hydrogenation reaction (1-2 ml) was stirred with 0.5 ml of a solution of 9:1:0.2 (by volume) methanol:water:trifluoroacetic acid at 40 °C for 1 hour. Dichloromethane (2 ml) and water (2ml) were added and the layers were separated. The aqueous layer was washed with dichloromethane (2ml) twice. The combined organic layers were evaporated under reduced pressure to yield the ketone as a colourless to pale-yellow oil. The crude ketone was then analysed for purity and isomer ratio and identified to be (6R,10R)-6,10,14-trimethylpentadecan-2-one.

### Formation of (R,R)-isophytol

### Experiment E6-I: Ethynylation of (6R,10R)-6,10,14-trimethylpentadecan-2-one (step g)

(6R,10R)-6,10,14-trimethylpentadecan-2-one (35.0 g, 129 mmol, 1.0 eq., 98.8%) was added to an autoclave equipped with thermostat, dosing pump, acetylene inlet and ammonia inlet. The reactor was sealed, evacuated, then flushed with nitrogen and cooled to 15 °C. Ammonia (715 g, 45.0 mol, 326 eq., 99.8%) was condensed into the reactor and cooled to 15 °C, resulting in a pressure of 8-9 bar. Acetylene was introduced until 12 bar was reached, followed by a dosed addition of KOH (40 wt% in water, 5.0 g, 35.6 mmol, 28 mol%) at 15 °C. The reaction progress was monitored by GC. At the desired conversion (after approx. 2 h), the reaction mixture was neutralized with acetic acid, and the reactor was subsequently vented at 25 °C. The reaction mixture was washed and concentrated in vacuo and purified by distillation in vacuo furnishing 26.9 g (7R,11R)-3,7,11,15-tetramethylhexadec-1-yn-3-ol with a purity of 98.8 area% (70% yield).

### Experiment E6-II Hydrogenation of (6R,10R)-6,10,14-trimethylpentadecan-2-one in the presence of a Lindlar catalyst (step h)

(7R,11R)-3,7,11,15-tetramethylhexadec-1-yn-3-ol (10 g, 33.4 mmol, 98.4 % purity), dissolved in heptane (40 g) and Lindlar catalyst (850 mg) were placed into an autoclave. The reactor was sealed, flushed with nitrogen and subsequently heated to 85 °C. When the desired temperature was reached, the reaction was pressurized with 2 bar hydrogen. The reaction was stirred for approximately 22 hours at this temperature until the required amount of hydrogen gas was consumed. After filtration, the crude product was combined with a second reaction batch. 11.9 g of the crude material was purified by distillation, furnishing 11.1 g of (R,R)-isophytol (97.6% purity by GC, 88% overall yield).

### Experiment E6-III:Vinylation of (6R,10R)-6,10,14-trimethylpentadecan-2-one (step h')

A dried 100 mL four-necked flask equipped with overhead stirrer, thermometer, condenser and argon inlet was evacuated and purged with argon. Vinylmagnesium chloride (18.3 mL of a 1.6 M solution in THF, 29.0 mmol, 1.59 eq.) was added at room temperature. A solution of (6R,10R)-6,10,14-trimethylpentadecan-2-one (5.00 g, 18.3 mmol, 98.2%, 1.0 eq.) in dry THF (20 mL) was added slowly within 25 min. The exothermic reaction was maintained between 25 and 30 °C internal temperature by cooling with an ice bath. After complete addition the reaction was allowed to stir at room temperature for 1 h. Saturated NH₄Cl solution (10 mL) was added carefully to quench excess Grignard reagent. Pentane (150 mL), water (150 mL) and brine (150 mL) was added. The organic phase was extracted with brine (2 x 150 mL) and the aqueous phase was back-extracted with pentane (2 x 150 mL). The combined organic phases were dried (MgSO₄) and concentrated in vacuo, resulting in a colorless oil (5.58 g). The crude product was purified by vacuum distillation in a Kugelrohr apparatus. The main fraction distilled at 143 °C/3.5x10⁻² mbar, furnishing (R,R)-isophytol (= (7R,11R)-3,7,11,15-tetramethylhexadec-1-en-3-ol) as colorless oil with a purity of 99.3% (5.271 g, 96% yield).

### Experiment E7: Formation of (2-ambo)-α-tocopherol (step m)

(R,R)-isophytol (= (7R,11R)-3,7,11,15-tetramethylhexadec-1-en-3-ol) was condensed with 2,3,5-trimethylbenzene-1,4-diol (= 2,3,5-trimethylhydoquinone) in the presence of a condensation catalyst to (2-*ambo*)-α-tocopherol according to the procedure disclosed in WO 2005/121115 A1.

### Experiment E8: Formation of (2R,4'R,8'R)-α-tocopherol (step n)

(2-*ambo*)-α-tocopherol was separated by means of chromatographic separation using a chiral phase. The preparative chromatography yielded (2R,4'R,8'R)-α-tocopherol and (2S,4'R,8'R)-α-tocopherol:
The (2-*ambo*)-α-tocopherol of experiment E7 was analyzed by HPLC (Column: Daicel Chiracel® OD-H, 250 mm x 4.6 mm; eluent 0.5% ethanol in n-heptane; flow 1 ml/min; detection 220 nm, 2 µl injection. Figure 7b) shows this chromatogram (Retention time 7.2 resp. 8.2 min, 50.2 : 49.2).
A solution of 140 mg (2-*ambo*)-α-tocopherol in heptane was injected and two peaks with retention time at maximum of 13.4 min (1) (50.1%) and 15.0 min (2) (49.9%) were separated by the preparative HPLC separation. Figure 7a) shows the chromatogram of the preparative HPLC separation.

After evaporation to dryness and dissolution the two collected fractions have been reanalysed on an analytical column (Daicel Chiracel® OD-H, 250 mm x 4.6 mm; eluent 0.5% ethanol in n-heptane; flow 1 ml/min; detection 220 nm, 2 µl injection). Figure 7c), respectively Figure 7d), show the chromatogram of the first fraction, respectively the second fraction. The isomeric ratios of the two isomers (Retention time 7.2 min, resp. 8.2 min) in said fractions are 99.5 : 0.5 (Figure 7c)) and 0.8 : 99.2 (Figure 7d), respectively. Hence, the two isomers have been separation by preparative chromatography almost completely.
The isomers have been identified to be (2R,4'R,8'R)-α-tocopherol (retention time 7.2 min) and (2S,4'R,8'R)-α-tocopherol (retention time 8.2 min).

### Experimental details for chromatography of experiment E8:

Preparative separations were performed on an Agilent 1100 series HPLC system consisting of an Agilent 1100 degasser, Agilent 1100 preparative pump, Agilent 1100 diode array detector, Agilent 1100 MPS G2250A autosampler/fraction collector controlled by chemstation/CC-mode software package.

### HPLC conditions for preparative separation:

Column: Daicel Chiracel® OD-H, 250 mm x 20 mm; eluent 0.5% isopropanol, 0.2 % acetic acid in n-heptane; flow 13 ml/min; detection 220 nm, 400 µl injection.

## Claims

1. A process of manufacturing (6R,10R)-6,10,14-trimethylpentadecan-2-one in a multistep synthesis from 3,7-dimethyloct-2-enal or 3,7-dimethylocta-2,6-dienal comprising the steps
a) providing a mixture of (E)-3,7-dimethyloct-2-enal and (Z)-3,7-dimethyloct-2-enal or a mixture of (E)-3,7-dimethylocta-2,6-dienal and (Z)-3,7-dimethylocta-2,6-dienal;
b) separating one isomer of 3,7-dimethyloct-2-enal or 3,7-dimethylocta-2,6-dienal from the mixture of step a)
c) asymmetric hydrogenation using molecular hydrogen in the presence of a chiral iridium complex and yielding (R)-3,7-dimethyloctanal ;
d) chemically transforming (R)-3,7-dimethyloctanal to a mixture of (R,E)-6,10,14-trimethylpentadec-5-en-2-one and (R,Z)-6,10,14-trimethylpentadec-5-en-2-one ;
e) separating one isomer of (R)-6,10,14-trimethylpentadec-5-en-2-one from the mixture obtained in step d)
f) asymmetric hydrogenation using molecular hydrogen in the presence of a chiral iridium complex and yielding (6R,10R)-6,10,14-trimethylpentadecan-2-one ;
wherein the steps a)-f) are in the order a,b,c,d,e,f.

2. The process according to claim 1 **characterized in that** before the step c) a step cₒ) takes place
cₒ) forming an acetal of the isomer of 3,7-dimethyloct-2-enal or of the acetal of the isomer of 3,7-dimethylocta-2,6-dienal separated in step b)
and that in step c) the acetal of 3,7-dimethyloct-2-enal or the acetal of 3,7-dimethylocta-2,6-dienal is asymmetrically hydrogenated and after the asymmetric hydrogenation the hydrogenated acetal is hydrolysed to the ketone and yielding (R)-3,7-dimethyloctanal .

3. The process according to claim 1 or 2 **characterized in that** before the step f) a step fₒ) takes place
fₒ) forming an acetal of the isomer of 6,10,14-trimethylpentadec-5-en-2-one separated in step e)
and that in step f) the ketal of 6,10,14-trimethylpentadec-5-en-2-one is asymmetrically hydrogenated and after the asymmetric hydrogenation the hydrogenated ketal is hydrolysed to the ketone and yielding (6R,10R)-6,10,14-trimethylpentadecan-2-one .

4. The process according to any one of the preceding claims **characterized in that** the asymmetric hydrogenation in step c) and/or in step f) takes place in the presence of an additive which is selected from the group consisting of organic sulfonic acids, transition metal salts of organic sulfonic acids, metal alkoxides, aluminoxanes, alkyl aluminoxanes and B(R)₍₃₋ᵥ₎(OZ)ᵥ;
wherein v stands for 0, 1, 2 or 3 and
R stands for F, a C₁₋₆-alkyl, a halogenated C₁₋₆-alkyl, an aryl or halogenated aryl group; and
Z stands a C₁₋₆-alkyl, a halogenated C₁₋₆-alkyl, an aryl or halogenated aryl group.

5. The process according to any one of the preceding claims **characterized in that** in step d) the chemical transformation of (R)-3,7-dimethyloctanal to a mixture of (R,E)-6,10,14-trimethylpentadec-5-en-2-one and (R,Z)-6,10,14-trimethylpentadec-5-en-2-one is done by the steps
d1) reacting with acetone to yield (R)-6,10-dimethylundec-3-en-2-one;
d2) hydrogenation of (R)-6,10-dimethylundec-3-en-2-one with molecular hydrogen in the presence of a hydrogenation catalyst to yield (R)-6,10-dimethylundecan-2-one;
followed by
either
d3) ethynylation of (R)-6,10-dimethylundecan-2-one using ethyne in the presence of a basic substance to yield (7R)-3,7,11-trimethyldodec-1-yn-3-ol;
d4) hydrogenation of (7R)-3,7,11-trimethyldodec-1-yn-3-ol with molecular hydrogen in the presence of a Lindlar catalyst to yield (7R)-3,7,11-trimethyldodec-1-en-3-ol;
or
d3')vinylation of (R)-6,10-dimethylundecan-2-one by addition of a vinyl Grignard reagent to yield (7R)-3,7,11-trimethyldodec-1-en-3-ol;
followed by
either
d5) reacting (7R)-3,7,11-trimethyldodec-1-en-3-ol with 2-methoxyprop-1-ene to yield a mixture of (R,E)-6,10,14-trimethylpentadec-5-en-2-one and (R,Z)-6,10,14-trimethylpentadec-5-en-2-one;
or
d5')reacting (7R)-3,7,11-trimethyldodec-1-en-3-ol with an alkyl acetoacetate or diketene in the presence of a base and/or an acid yield a mixture of (R,E)-6,10,14-trimethylpentadec-5-en-2-one and (R,Z)-6,10,14-trimethylpentadec-5-en-2-one.

6. The process according to any one of the preceding claims **characterized in that** the separation of isomers in step b) and/or e) is done by distillation.

7. The process according to claim 6 **characterized in that** the distillation is done in the presence of a cis/trans isomerization catalyst.

8. The process according to any one of the preceding claims 1-6 **characterized in that** the residual isomer is isomerized by means of a cis/trans isomerization catalyst and added to the corresponding mixture of isomers provided by steps a) resp. d).

9. The process according to any one of the preceding claims **characterized in that** the chiral iridium complex in steps c) and/or f) is a chiral iridium complex of formula (III-0) 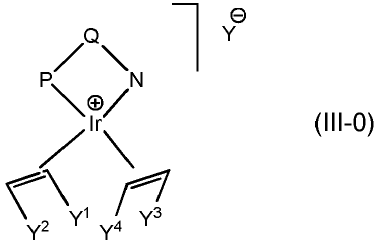 wherein
P-Q-N stands for a chelating organic ligand comprising a stereogenic centre or has planar or axial chirality and has a nitrogen and phosphorous atom as binding site to the iridium centre of the complex;
Y¹, Y², Y³ and Y⁴ independently from each other are hydrogen atoms, C₁₋₁₂-alkyl, C₅₋₁₀-cycloalkyl, or aromatic group; or at least two of them form together at least a two-valent bridged group of at least 2 carbon atoms; and
Y^{⊖} is an anion, particularly selected from the group consisting of halide, PF₆⁻, SbF₆⁻, tetra(3,5-bis(trifluoromethyl)phenyl)borate (BAr_{F}⁻), BF₄⁻, perfluorinated sulfonates, preferably F₃C-SO₃⁻ or F₉C₄-SO₃⁻; ClO₄⁻, Al(OC₆F₅)₄⁻, Al(OC(CF₃)₃)₄⁻, N(SO₂CF₃)₂⁻N(SO₂C₄F₉)₂⁻ and B(C₆F₅)₄⁻.

10. The process according to any one of the preceding claims 1-8 **characterized in that** the chiral iridium complex in steps c) and/or f) is a chiral iridium complex of formula (III) 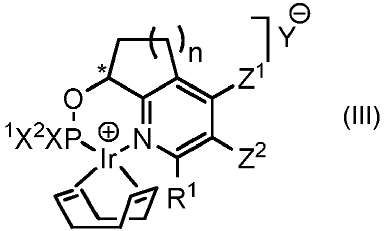 wherein
n is 1 or 2 or 3, preferred 1 or 2;
X¹ and X² are independently from each other hydrogen atoms, C₁₋₄-alkyl, C₅₋₇-cycloalkyl, adamantyl, phenyl (optionally substituted with one to three C₁₋₅-alkyl, C₁₋₄-alkoxy, C₁₋₄-perfluoroalkyl groups and/or one to five halogen atoms)), benzyl, 1-naphthyl, 2-naphthyl, 2-furyl or ferrocenyl;
Z¹ and Z² are independently from each other hydrogen atoms, C₁₋₅-alkyl or C₁₋₅-alkoxy groups;
or Z¹ and Z² stand together for a bridging group forming a 5 or 6 membered ring;
Y^{⊖} is an anion, particularly selected from the group consisting of halide, PF₆⁻, SbF₆⁻, tetra(3,5-bis(trifluoromethyl)phenyl)borate(BAr_{F}⁻), BF₄⁻, perfluorinated sulfonates, preferably F₃C-SO₃⁻ or F₉C₄-SO₃⁻; ClO₄⁻, Al(OC₆F₅)₄⁻, Al(OC(CF₃)₃)₄⁻, N(SO₂CF₃)₂⁻N(SO₂C₄F₉)₂⁻ and B(C₆F₅)₄⁻;
R¹ represents either phenyl or o-tolyl or m-tolyl or p-tolyl or a group of formula (IVa) or (IVb) or (IVc) 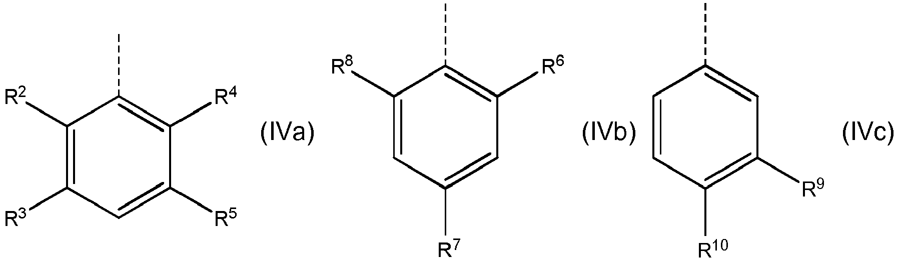
wherein R² and R³ represent either both H or a C₁-C₄-alkyl group or a halogenated C₁-C₄-alkyl group or represent a divalent group forming together a 6-membered cycloaliphatic or an aromatic ring which optionally is substituted by halogens atoms or by C₁-C₄-alkyl groups or by C₁-C₄-alkoxy groups
R⁴ and R⁵ represent either both H or a C₁-C₄-alkyl group or a halogenated C₁-C₄-alkyl group or a divalent group forming together a 6-membered cycloaliphatic or an aromatic ring which optionally is substituted by halogens atoms or by C₁-C₄-alkyl groups or by C₁-C₄-alkoxy groups;
R⁶ and R⁷ and R⁸ represent each a C₁-C₄-alkyl group or a halogenated C₁-C₄-alkyl group;
R⁹ and R¹⁰ represent either both H or a C₁-C₄-alkyl group or a halogenated C₁-C₄-alkyl group or a divalent group forming together a 6-membered cycloaliphatic or an aromatic ring which optionally is substituted by halogens atoms or by C₁-C₄-alkyl groups or by C₁-C₄-alkoxy groups;
and wherein * represents a stereogenic centre of the complex of formula (III).

11. The process according to claim 10 **characterized in that** the chiral iridium complex of formula (III) used in step c) and/or f) used for the asymmetric hydrogenation has the
S-configuration at the stereogenic centre indicated by * in case (E)-3,7-dimethyloct-2-enal or (E)-3,7-dimethylocta-2,6-dienal, or acetals thereof, or (R,E)-6,10,14-trimethylpentadec-5-en-2-one, or ketals thereof, is to be hydrogenated;
or has the
R-configuration at the stereogenic centre indicated by * in case (Z)-3,7-dimethyloct-2-enal or (Z)-3,7-dimethylocta-2,6-dienal, or acetals thereof, or (R,Z)-6,10,14-trimethylpentadec-5-en-2-one, or ketals thereof, is to be hydrogenated.

12. A process of manufacturing (R,R)-isophytol ((3RS,7R,11R)-3,7,11,15-tetramethylhexadec-1-en-3-ol) comprising
a process of manufacturing (6R,10R)-6,10,14-trimethylpentadecan-2-one as described in the process according any one of the preceding claims 1-11; followed by the steps
either
g) ethynylation of (6R,10R)-6,10,14-trimethylpentadecan-2-one using ethyne in the presence of a basic substance to yield (7R,11R)-3,7,11,15-tetramethylhexadec-1-yn-3-ol;
h) hydrogenation of (7R,11R)-3,7,11,15-tetramethylhexadec-1-yn-3-ol with molecular hydrogen in the presence of a Lindlar catalyst to yield (R,R)-isophytol;
or
h') vinylation of (6R,10R)-6,10,14-trimethylpentadecan-2-one by addition of a vinyl Grignard reagent to yield (R,R)-isophytol.

13. A process of manufacturing compound of formula (V) comprising
a process of manufacturing (6R,10R)-6,10,14-trimethylpentadecan-2-one as described in the process according any one of the preceding claims 1-11;
followed by the steps
either
g) ethynylation of (6R,10R)-6,10,14-trimethylpentadecan-2-one using ethyne in the presence of a basic substance to yield (7R,11R)-3,7,11,15-tetramethylhexadec-1-yn-3-ol;
h) hydrogenation of (7R,11R)-3,7,11,15-tetramethylhexadec-1-yn-3-ol with molecular hydrogen in the presence of a Lindlar catalyst to yield (R,R)-isophytol;
or
h') vinylation of (6R,10R)-6,10,14-trimethylpentadecan-2-one by addition of a vinyl Grignard reagent to yield (R,R)-isophytol;
followed by the steps
m) condensing (R,R)-isophytol with compound of formula (VI) to yield compound of formula (V) being an isomeric mixture in view of the chirality at the centre indicated by #; 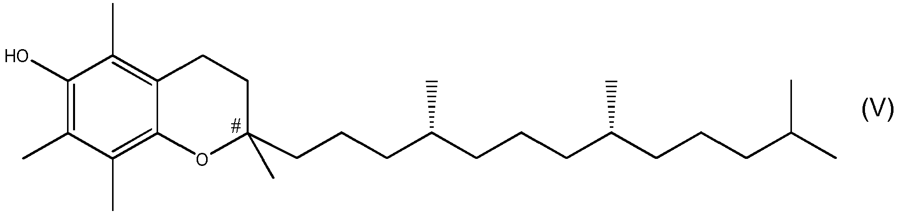 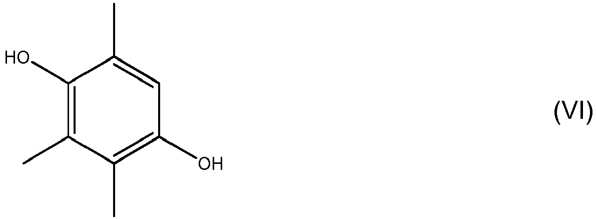 wherein # represents a stereogenic centre.

14. A process of manufacturing compound of formula (V-A) comprising
a process of manufacturing (6R,10R)-6,10,14-trimethylpentadecan-2-one as described in the process according any one of the preceding claims 1-11;
followed by the steps
either
g) ethynylation of (6R,10R)-6,10,14-trimethylpentadecan-2-one using ethyne in the presence of a basic substance to yield (7R,11R)-3,7,11,15-tetramethylhexadec-1-yn-3-ol;
h) hydrogenation of (7R,11R)-3,7,11,15-tetramethylhexadec-1-yn-3-ol with molecular hydrogen in the presence of a Lindlar catalyst to yield (R,R)-isophytol;
or
h') vinylation of (6R,10R)-6,10,14-trimethylpentadecan-2-one by addition of a vinyl Grignard reagent to yield (R,R)-isophytol; followed by the steps
m) condensing (R,R)-isophytol with compound of formula (VI) to yield compound of formula (V) being an isomeric mixture in view of the chirality at the centre indicated by #; 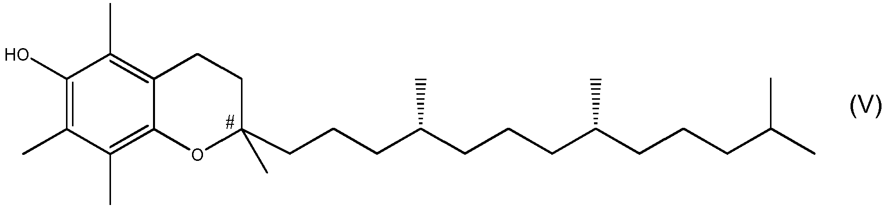 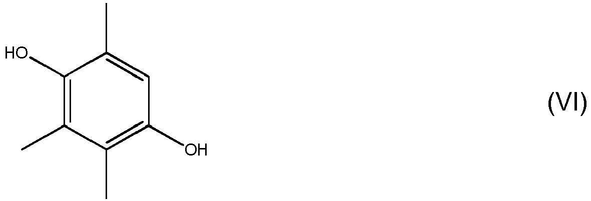 wherein # represents a stereogenic chiral centre;
and
n) isolating compound of formula (V-A) form the from the isomeric mixture of formula (V)

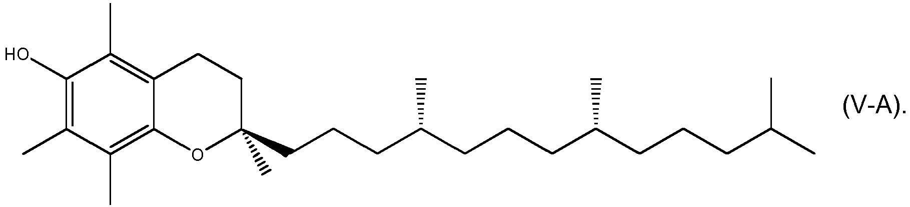

15. Composition comprising
- at least one acetal or ketal of formula (XI) or (XII) and
- at least one chiral iridium complex 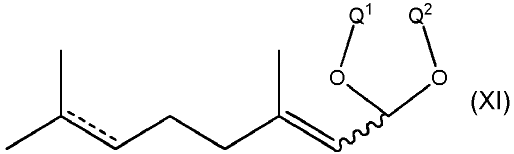 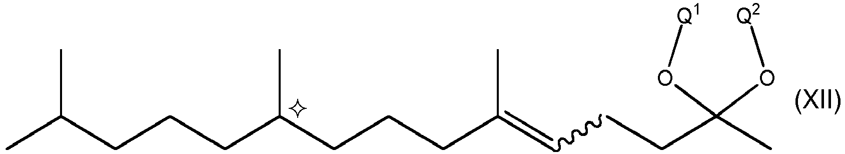
wherein a wavy line represents a carbon-carbon bond which is linked to the adjacent carbon-carbon double bond so as to have said carbon-carbon double bond either in the Z or in the E-configuration;
and wherein the double bond having dotted lines (------) in formula represent either a single carbon-carbon bond or a double carbon-carbon bond;
and wherein ✧ represents a stereogenic centre which has the R-configuration;
and wherein
Q¹ and Q²
stand either individually or both for a C₁-C₁₀ alkyl group or a halogenated C₁-C₁₀ alkyl group;
or form together a C₂-C₆ alkylene group or a C₆-C₈ cycloalkylene group.

16. Acetal or ketal of formula (XX-A) or (XX-B) or (XX-C) or (XX-D) or (XX-E) 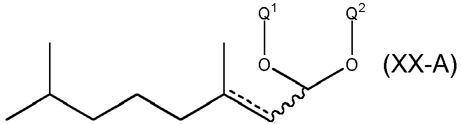 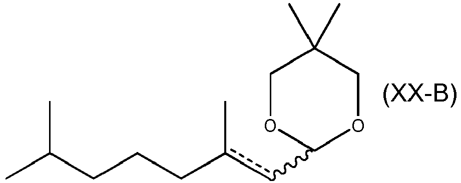 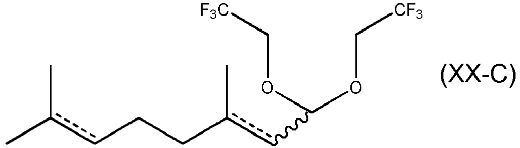 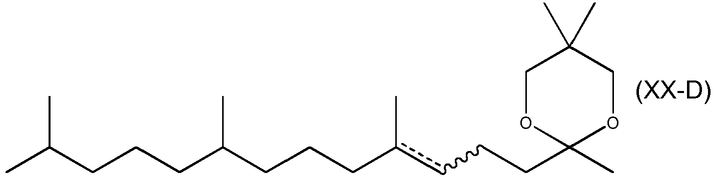 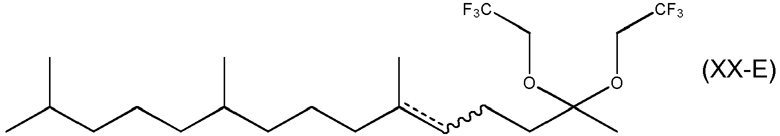
wherein the double bond having dotted lines (------) in the above formulae represents either a single carbon-carbon bond or a double carbon-carbon bond;and
wherein a wavy line represents a carbon-carbon bond which is linked to an adjacent single carbon bond (------ representing 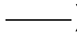 ) or to an adjacent carbon-carbon double bond (------ representing 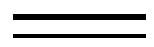 ) so as to have said carbon-carbon double bond either in the Z or in the E-configuration.
and wherein
Q¹ and Q²
stand either individually or both for a C₁-C₁₀ alkyl group or a halogenated C₁-C₁₀ alkyl group;
or form together a C₂-C₆ alkylene group or a C₆-C₈ cycloalkylene group;
with the exception of 1,1-dimethoxy-3,7-dimethyloct-2-ene;
and wherein
the acetal or ketal of formula (XX-D) or (XX-E) is
(R)-2,5,5-trimethyl-2-(4,8,12-trimethyltridec-3-en-1 -yl)-1,3-dioxane
or
(R)-6,10,14-trimethyl-2,2-bis(2,2,2-trifluoroethoxy)pentadec-5-ene
or
2,5,5-trimethyl-2-((4R,8R)-4,8,12-trimethyltridecyl)-1,3-dioxane
or
(6R,10R)-6,10,14-trimethyl-2,2-bis(2,2,2-trifluoroethoxy)pentadecane.

## Patentansprüche

1. Verfahren für die Herstellung von (6R,10R)-6,10,14-Trimethylpentadecan-2-on durch eine Mehrschrittsynthese von 3,7-Dimethyloct-2-enal oder 3,7-Dimethylocta-2,6-dienal, umfassend die Schritte
a) Bereitstellen einer Mischung von (E)-3,7-Dimethyloct-6-enal und (Z)-3,7-Dimethyloct-2-enal oder einer Mischung von (E)-3,7-Dimethylocta-2,6-dienal und (Z)-3,7-Dimethylocta-2,6-dienal;
b) Trennen eines Isomers von 3,7-Dimethyloct-2-enal oder 3,7-Dimethylocta-2,6-dienal von der Mischung aus Schritt a)
c) asymmetrische Hydrierung unter Anwendung von molekularem Wasserstoff in Gegenwart eines chiralen Iridiumkomplexes und Ergeben von (R)-3,7-Dimethyloctanal;
d) chemisches Umwandeln von (R)-3,7-Dimethyloctanal in eine Mischung von (R,E)-6,10,14-Trimethylpentadec-5-en-2-on und (R,Z)-6,10,14-Trimethylpenta-dec-5-en-2-on;
e) Trennen eines Isomers von (R)-6,10,14-Trimethylpentadec-5-en-2-on von der Mischung, die in Schritt d) erhalten worden ist
f) asymmetrische Hydrierung unter Anwendung von molekularem Wasserstoff in Gegenwart eines chiralen Iridiumkomplexes und Ergeben von (6R,10R)-6,10,14-Trimethylpentadecan-2-on;
wobei die Schritte a)-f) in der Reihenfolge 8 a,b,c,d,e,f stattfinden.

2. Verfahren nach Anspruch 1, **dadurch gekennzeichnet, dass** vor Schritt c) ein Schritt cₒ) stattfindet
cₒ) Bilden eines Acetals des Isomers von 3,7-Dimethyloct-2-enal oder des Acetals des Isomers von 3,7-Dimethylocta-2,6-dienals, das in Schritt b) abgetrennt worden ist
und dass in Schritt c) das Acetal von 3,7-Dimethyloct-2-enal oder das Acetal von 3,7-Dimethylocta-2,6-dienal asymmetrisch hydriert wird und nach der asymmetrischen Hydrierung das hydrierte Acetal zu dem Keton hydrolysiert wird und (R)-3,7-Dimethyloctanal ergibt.

3. Verfahren nach Anspruch 1 oder 2, **dadurch gekennzeichnet, dass** vor Schritt f) ein Schritt fₒ) stattfindet
fₒ) Bilden eines Acetals des Isomers von 6,10,14-Trimethylpentadec-5-en-2-on, das in Schritt e) abgetrennt worden ist
und dass in Schritt f) das Ketal von 6,10,14-Trimethylpentadec-5-en-2-on asymmetrisch hydriert wird und nach der asymmetrischen Hydrierung das hydrierte Ketal zu dem Keton hydrolysiert wird, wodurch (6R,10R)-6,10,14-Trimethylpentadecan-2-on ergeben wird.

4. Verfahren nach irgendeinem der vorhergehenden Ansprüche, **dadurch gekennzeichnet, dass** die asymmetrische Hydrierung in Schritt c) und/oder in Schritt f) in Gegenwart eines Zusatzmittels stattfindet, das aus der Gruppe ausgewählt ist bestehend aus organischen Sulfonsäuren, Übergangsmetallsalzen organischer Sulfonsäuren, Metallalkoxiden, Aluminoxanen, Alkylaluminoxanen und B(R)_{(3-v}}(OZ)ᵥ;
wobei v für 0, 1, 2 oder 3 steht und
R für F, eine C₁₋₆-Alkyl-, eine halogenierte C₁₋₆-Alkyl-, eine Aryl- oder halogenierte Arylgruppe steht; und
Z für eine C₁₋₆-Alkyl-, eine halogenierte C₁₋₆-Alkyl-, eine Aryl- oder halogenierte Arylgruppe steht.

5. Verfahren nach irgendeinem der vorhergehenden Ansprüche, **dadurch gekennzeichnet, dass** in Schritt d) die chemische Umwandlung von (R)-3,7-Dimethyloctanal zu einer Mischung von (R,E)-6,10,14-Trimethylpentadec-5-en-2-on und (R,Z)-6,10,14-Trimethylpentadec-5-en-2-on durch die Schritte erfolgt
d1) Umsetzen mit Aceton, um (R)-6,10-Dimethylundec-3-en-2-on zu ergeben;
d2) Hydrierung von (R)-6,10-Dimethylundec-3-en-2-on mit molekularem Wasserstoff in Gegenwart eines Hydrierungskatalysators, um (R)-6,10-Dimethylundecan-2-on zu ergeben;
gefolgt von
entweder
d3) Ethinylierung von (R)-6,10-Dimethylundecan-2-on unter Anwendung von Ethin in Gegenwart einer basischen Substanz, um (7R)-3,7,11-Trimethyldodec-1-yn-3-ol zu ergeben;
d4) Hydrierung von (7R)-3,7,11-Trimethyldodec-1-yn-3-ol mit molekularem Wasserstoff in Gegenwart eines Lindlar-Katalysators, um (7R)-3,7,11-Trimethyldodec-1-en-3-ol zu ergeben;
oder
d3') Vinylierung von (R)-6,10-Dimethylundecan-2-on durch Zugabe eines Vinyl-Grignard-Reagens, um (7R)-3,7,11-trimethyldodec-1-en-3-ol zu ergeben;
gefolgt von
entweder
d5) Umsetzen von (7R)-3,7,11-Trimethyldodec-1-en-3-ol mit 2-Methoxyprop-1-en, um eine Mischung von (R,E)-6,10,14-Trimethylpentadec-5-en-2-on und (R,Z)-6,10,14-trimethylpentadec-5-en-2-on zu ergeben;
oder
d5') Umsetzen von (7R)-3,7,11-Trimethyldodec-1-en-3-ol mit einem Alkylacetoacetat oder Diketen in Gegenwart einer Base und/oder einer Säure, um eine Mischung von (R,E)-6,10,14-Trimethylpentadec-5-en-2-on und (R,Z)-6,10,14-Trimethylpentadec-5-en-2-on zu ergeben.

6. Verfahren nach irgendeinem der vorhergehenden Ansprüche, **dadurch gekennzeichnet, dass** die Trennung von Isomeren in Schritt b) und oder e) durch Destillation ausgeführt wird.

7. Verfahren nach Anspruch 6, **dadurch gekennzeichnet, dass** die Destillation in Gegenwart eines cis/trans-Isomerisationskatalysators ausgeführt wird.

8. Verfahren nach irgendeinem der vorhergehenden Ansprüche 1-6, **dadurch gekennzeichnet, dass** das restliche Isomer durch einen cis/trans-Isomerisationskatalysator isomerisiert und der entsprechenden Mischung von Isomeren, die in Schritt a) bzw. d) bereitgestellt worden ist, zugegeben wird.

9. Verfahren nach irgendeinem der vorhergehenden Ansprüche, **dadurch gekennzeichnet, dass** der chirale Iridiumkomplex in Schritt c) und/oder f) ein chiraler Iridiumkomplex der Formel (III-0) ist 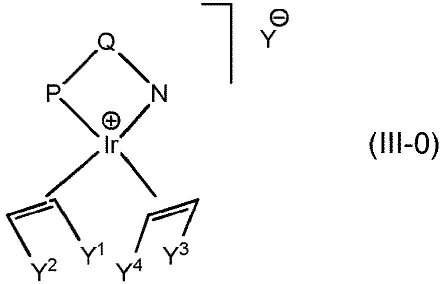 worin
P-Q-N für einen chelatbildenden organischen Liganden steht, der ein stereogenes Zentrum oder eine axiale Chiralität aufweist und ein Stickstoff- und Phosphoratom als Bindungsstelle in das Iridiumzentrum des Komplexes aufweist;
Y¹, Y², Y³ und Y⁴ unabhängig voneinander Wasserstoffatome, eine C₁₋₁₂-Alkyl-, C₅₋₁₀-Cycloalkyl- oder aromatische Gruppe sind; oder mindestens zwei davon zusammen mindestens eine zweiwertige gebrückte Gruppe von mindestens 2 Kohlenstoffatomen bilden; und
Y^{⊖} ein Anion ist, das insbesondere aus der Gruppe ausgewählt ist bestehend aus Halogenid, PF₆⁻, SbF₆⁻, tetra(3,5-Bis(trifluormethyl)phenyl)borat (BAr_{F}⁻), BF₄⁻, perfluorierten Sulfonaten, bevorzugt F₃C-SO₃⁻ oder F₉C₄-SO₃⁻; ClO₄⁻, Al(OC₆F₅)₄⁻, Al(OC(CF₃)₃)₄⁻, N(SO₂CF₃)₂⁻N(SO₂C₄F₉)₂⁻ und B(C₆F₅)₄⁻.

10. Verfahren nach irgendeinem der vorhergehenden Ansprüche 1-8, **dadurch gekennzeichnet, dass** der chirale Iridiumkomplex in Schritt c) und/oder f) ein chiraler Iridiumkomplex der Formel (III) ist 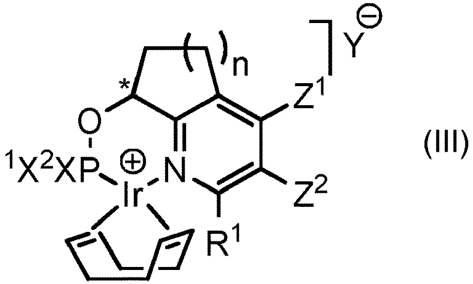 worin
n 1 oder 2 oder 3, bevorzugt 1 oder 2 beträgt;
X¹ und X² unabhängig voneinander Wasserstoffatome, C₁₋₄-Alkyl, C₅₋₇-Cycloalkyl, Adamantyl, Phenyl(das wahlweise mit einer bis drei C₁₋₅-Alkyl-, C₁₋₄-Alkoxy-, C₁₋₄-Perfluoroalkylgruppen und/oder einem bis fünf Halogenatomen substituiert ist), Benzyl, 1-Naphthyl, 2-Naphthyl, 2-Furyl oder Ferrocenyl sind;
Z¹ und Z² unabhängig voneinander Wasserstoffatome, C₁₋₅-Alkyl- oder C₅₋₁₀-Alkoxygruppen sind;
oder Z¹ und Z² zusammen für eine Überbrückungsgruppe stehen, die einen 5- oder 6-gliedrigen Ring bildet;
Y^{⊖} ein Anion ist, das insbesondere aus der Gruppe ausgewählt ist bestehend aus Halogenid, PF₆⁻, SbF₆⁻, tetra(3,5-Bis(trifluormethyl)phenyl)borat (BAr_{F}⁻), BF₄⁻, perfluorierten Sulfonaten, bevorzugt F₃C-SO₃⁻ oder F₉C₄-SO₃⁻; ClO₄⁻, Al(OC₆F₅)₄⁻, Al(OC(CF₃)₃)₄⁻, N(SO₂CF₃)₂⁻N(SO₂C₄F₉)₂⁻ und B(C₆F₅)₄⁻;
R¹ entweder Phenyl oder o-Tolyl oder m-Tolyl oder p-Tolyl oder eine Gruppe der Formel (IVa) oder (IVb) oder (IVc) 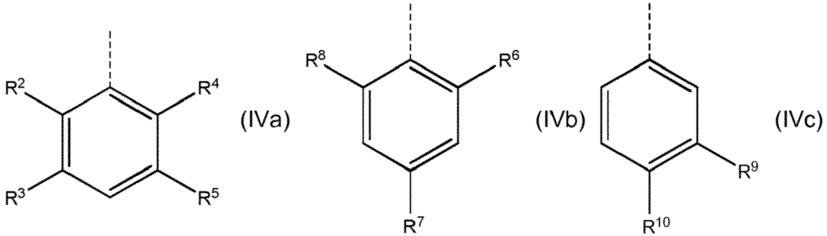
darstellt
worin R² und R³ entweder beide H oder eine C₁₋₄-Alkylgruppe oder eine halogenierte C₁₋₄-Alkylgruppe darstellen oder eine zweiwertige Gruppe darstellen, die zusammen einen 6-gliedrigen cycloaliphatischen oder aromatischen Ring bilden, der wahlweise durch Halogenatome oder durch C₁₋₄-Alkylgruppen oder durch C₁₋₄-Alkoxygruppen substituiert ist
R⁴ und R⁵ entweder beide H oder eine C₁₋₄-Alkylgruppe oder eine halogenierte C₁₋₄-Alkylgruppe oder eine zweiwertige Gruppe darstellen, die zusammen einen 6-gliedrigen cycloaliphatischen oder aromatischen Ring bilden, der wahlweise durch Halogenatome oder durch C₁₋₄-Alkylgruppen oder durch C₁₋₄-Alkoxygruppen substituiert ist;
R⁶ und R⁷ und R⁸ jeweils eine C₁₋₄-Alkylgruppe oder eine halogenierte C₁₋₄-Alkylgruppe darstellen;
R⁹ und R¹⁰ entweder beide H oder eine C₁₋₄-Alkylgruppe oder eine halogenierte C₁₋₄-Alkylgruppe oder eine zweiwertige Gruppe darstellen, die zusammen einen 6-gliedrigen cycloaliphatischen oder aromatischen Ring bilden, der wahlweise durch Halogenatome oder durch C₁₋₄-Alkylgruppen oder durch C₁₋₄-Alkoxygruppen substituiert ist;
und wobei * ein stereogenes Zentrum des Komplexes der Formel (III) darstellt.

11. Verfahren nach Anspruch 10, **dadurch gekennzeichnet, dass** der chirale Iridiumkomplex der Formel (III), der in Schritt c) und/oder f) verwendet wird und der für die asymmetrische Hydrierung verwendet wird, die
S-Konfiguration am stereogenen Zentrum aufweist, die durch * im Falle von (E)-3,7-Dimethyloct-2-enal oder (E)-3,7-Dimethylocta-2,6-dienal oder Acetale davon oder (R,E)-6,10,14- Trimethylpentadec-5-en-2-on oder Ketale davon angegeben ist, hydriert werden soll;
oder die
R-Konfiguration am stereogenen Zentrum aufweist, die durch * im Falle von (Z)-3,7-Dimethyloct-2-enal oder (Z)-3,7-Dimethylocta-2,6-dienal oder Acetalen davon oder (R,Z)-6,10,14-Trimethylpentadec-5-en-2-on oder Ketalen davon angegeben ist, hydriert werden soll.

12. Verfahren für die Herstellung von (R,R)-Isophytol ((3RS,7R,11R)-3,7,11,15-tetramethylhexadec-1-en-3-ol), umfassend
ein Verfahren für die Herstellung von (6R,10R)-6,10,14-Trimethylpentadecan-2-on, wie in dem Verfahren nach einem der vorhergehenden Ansprüche 1-11 beschrieben;
gefolgt von den Schritten
entweder
g) Ethinylierung von (6R,10R)-6,10,14-Trimethylpentadecan-2-on unter Anwendung von Ethin in Gegenwart einer basischen Substanz, um (7R,11R)-3,7,11,15-Tetramethylhexadec-1-yn-3-ol zu ergeben;
h) Hydrierung von (7R,11R)-3,7,11,15-Tetramethylhexadec-1-yn-3-ol mit molekularem Wasserstoff in Gegenwart eines Lindlar-Katalysators, um (R,R)-Isophytol zu ergeben;
oder
h') Vinylierung von (6R,10R)-6,10,14-Trimethylpentadecan-2-on durch Zugabe eines Vinyl-Grignard-Reagens, um (R,R)-Isophytol zu ergeben.

13. Verfahren für die Herstellung der Verbindung der Formel (V), umfassend
ein Verfahren für die Herstellung von (6R,10R)-6,10,14-Trimethylpentadecan-2-on, wie in dem Verfahren nach irgendeinem der vorhergehenden Ansprüche 1-11 beschrieben;
gefolgt von den Schritten
entweder
g) Ethinylierung von (6R,10R)-6,10,14-Trimethylpentadecan-2-on unter Anwendung von Ethin in Gegenwart einer basischen Substanz, um (7R,11R)-3,7,11,15-Tetramethylhexadec-1-yn-3-ol zu ergeben;
h) Hydrierung von (7R,11R)-3,7,11,15-Tetramethylhexadec-1-yn-3-ol mit molekularem Wasserstoff in Gegenwart eines Lindlar-Katalysators, um (R,R)-Isophytol zu ergeben;
oder
h') Vinylierung von (6R,10R)-6,10,14-Trimethylpentadecan-2-on durch Zugabe eines Vinyl-Grignard-Reagens, um (R,R)-Isophytol zu ergeben;
gefolgt von den Schritten
m) Kondensieren von (R,R)-Isophytol mit der Verbindung der Formel (VI), um die Verbindung der Formel (V) zu ergeben, die angesichts der Chiralität am durch # angegebenen Zentrum eine isomere Mischung ist; 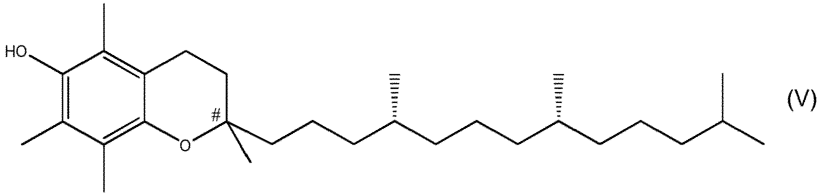 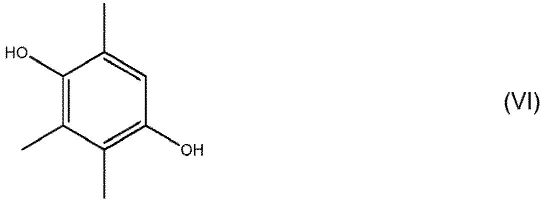 wobei # ein stereogenes Zentrum darstellt.

14. Verfahren für die Herstellung der Verbindung der Formel (V-A), umfassend
ein Verfahren für die Herstellung von (6R,10R)-6,10,14-Trimethylpentadecan-2-on, wie in dem Verfahren nach irgendeinem der vorhergehenden Ansprüche 1-11 beschrieben;
gefolgt von den Schritten
entweder
g) Ethinylierung von (6R,10R)-6,10,14-Trimethylpentadecan-2-on unter Anwendung von Ethin in Gegenwart einer basischen Substanz, um (7R,11R)-3,7,11,15-Tetramethylhexadec-1-yn-3-ol zu ergeben;
h) Hydrierung von (7R,11R)-3,7,11,15-Tetramethylhexadec-1-yn-3-ol mit molekularem Wasserstoff in Gegenwart eines Lindlar-Katalysators, um (R,R)-Isophytol zu ergeben;
oder
h') Vinylierung von (6R,10R)-6,10,14-Trimethylpentadecan-2-on durch Zugabe eines Vinyl-Grignard-Reagens, um (R,R)-Isophytol zu ergeben;
gefolgt von den Schritten
m) Kondensieren von (R,R)-Isophytol mit der Verbindung der Formel (VI), um die Verbindung der Formel (V) zu ergeben, die angesichts der Chiralität am durch # angegebenen Zentrum eine isomere Mischung ist; 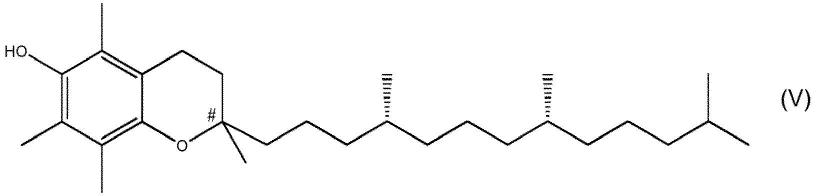 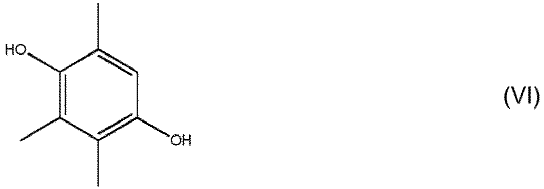 wobei # ein stereogenes chirales Zentrum darstellt;
und
n) Isolieren der Verbindung der Formel (V-A) von der isomeren Mischung der Formel (V)

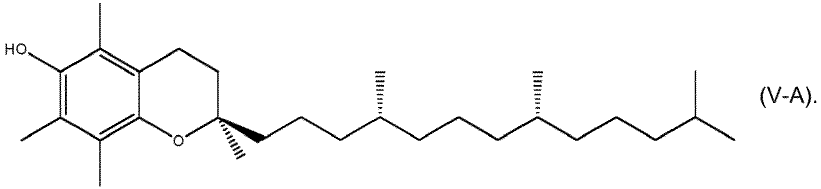

15. Zusammensetzung umfassend
- mindestens ein Acetal oder Ketal der Formel (XI) oder(XII) und
- mindestens einen chiralen Iridiumkomplex 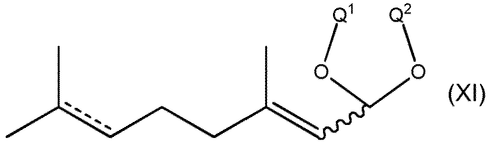 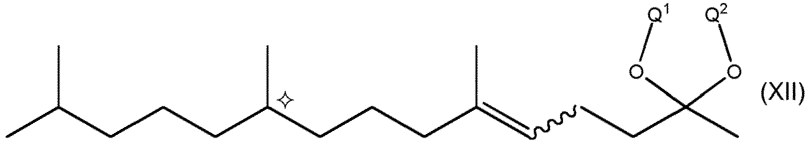
worin eine Wellenlinie eine Kohlenstoff-Kohlenstoff-Bindung darstellt, die an die anliegende Kohlenstoff-Kohlenstoff-Doppelbindung angeknüpft ist, sodass die Kohlenstoff-Kohlenstoff-Doppelbindung entweder in der Z- oder E-Konfiguration vorliegt;
und wobei die Doppelbindung, die in der Formel gestrichelte Linien (------) aufweist, entweder eine Kohlenstoff-Kohlenstoff-Einzelbindung oder eine Kohlenstoff-Kohlenstoff-Doppelbindung darstellt;
und worin ◊ ein stereogenes Zentrum darstellt, das die R-Konfiguration aufweist;
und worin
Q¹ und Q²
entweder einzeln oder beide für eine C₁₋₁₀-Alkylgruppe oder eine halogenierte C₁₋₁₀-Alkylgruppe stehen;
oder zusammen eine C₂₋₆-Alkylengruppe oder eine C₆₋₈-Cycloalkylengruppe bilden.

16. Acetal oder Ketal der Formel (XX-A) oder (XX-B) oder (XX-C) oder (XX-D) oder (XX-E) 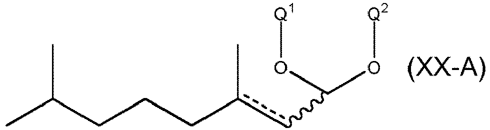 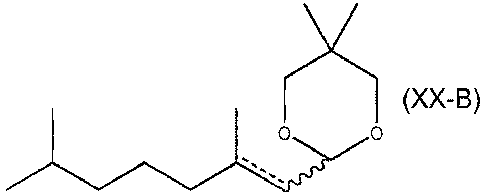 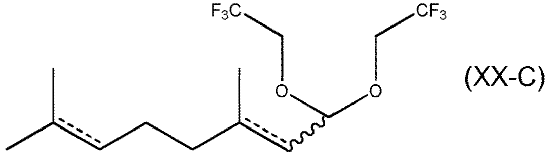 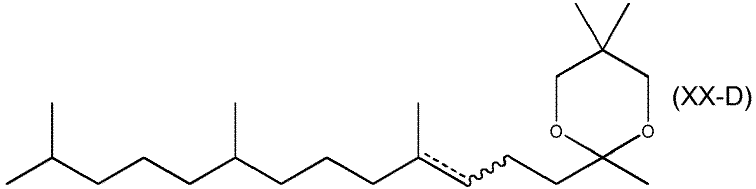 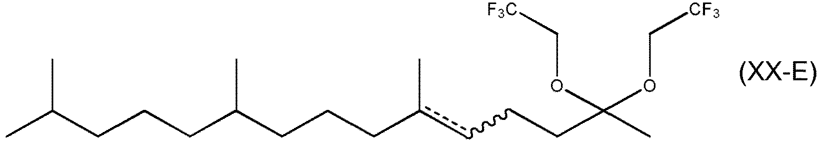
worin die Doppelbindung, die gestrichelte Linien (------) in den obigen Formeln aufweist, entweder eine Kohlenstoff-Kohlenstoff-Einzelbindung oder eine Kohlenstoff-Kohlenstoff- Doppelbindung darstellt; und
worin eine Wellenlinie eine Kohlenstoff-Kohlenstoffbindung darstellt, die an eine anliegende Kohlenstoffbindung (wobei ------ 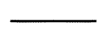 darstellt) oder an eine anliegende Kohlenstoff-Kohlenstoff-Doppeleinzelbindung(wobei ------ 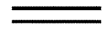 darstellt) angeknüpft ist, so dass die Kohlenstoff-Kohlenstoff-Doppelbindung entweder in der Z- oder der E-Konfiguration vorliegt,
und worin Q¹ und Q²
entweder einzeln oder beide für eine C₁₋₁₀-Alkylgruppe oder eine halogenierte C₁₋₁₀-Alkylgruppe stehen;
oder zusammen eine C₂₋₆-Alkylengruppe oder eine C₆₋₈-Cycloalkylengruppe bilden;
mit Ausnahme von 1,1-Dimethoxy-3,7-dimethyloct-2-en;
und worin
das Acetal oder Ketal der Formel (XX-D) oder (XX-E)
(R)-2,5,5-Trimethyl-2-(4,8,12-trimethyltridec-3-en-1-yl)-1,3-dioxan
oder
(R)-6,10,14-Trimethyl-2,2-bis(2,2,2-trifluorethoxy)pentadec-5-en
oder
2,5,5-Trimethyl-2-((4R,8R)-4,8,12-trimethyltridecyl)-1,3-dioxan
oder
(6R,10R)-6,10,14-Trimethyl-2,2-bis(2,2,2-trifluorethoxy)pentadecan ist.

## Revendications

1. Procédé de fabrication de (6R,10R)-6,10,14-triméthylpentadécan-2-one dans une synthèse à étapes multiples à partir de 3,7-diméthyloct-2-énal ou 3,7-diméthylocta-2,6-diénal comprenant les étapes de
a) fourniture d'un mélange de (E)-3,7-diméthyloct-2-énal et (Z)-3,7-diméthyloct-2-énal ou un mélange de (E)-3,7-diméthylocta-2,6-diénal et (Z)-3,7-diméthylocta-2,6-diénal ;
b) séparation d'un isomère de 3,7-diméthyloct-2-énal ou 3,7-diméthylocta-2,6-diénal à partir du mélange de l'étape a)
c) hydrogénation asymétrique au moyen d'hydrogène moléculaire en présence d'un complexe d'iridium chiral et obtention de (R)-3,7-diméthyloctanal ;
d) transformation chimique de (R)-3,7-diméthyloctanal en mélange de (R,E)-6,10,14-triméthylpentadéc-5-én-2-one et (R,Z)-6,10,14-triméthylpenta-déc-5-én-2-one ;
e) séparation d'un isomère de (R)-6,10,14-triméthylpentadéc-5-én-2-one à partir du mélange obtenu dans l'étape d)
f) hydrogénation asymétrique au moyen d'hydrogène moléculaire en présence d'un complexe d'iridium chiral et obtention de (6R,10R)-6,10,14-triméthylpentadécan-2-one ;
dans lequel les étapes a) à f) sont dans l'ordre a,b,c,d,e,f.

2. Procédé selon la revendication 1 **caractérisé en ce que**, avant l'étape c) une étape cₒ) est conduite
cₒ) formation d'un acétal de l'isomère de 3,7-diméthyloct-2-énal ou de l'acétal de l'isomère de 3,7-diméthylocta-2,6-diénal séparés dans l'étape b)
et que, dans l'étape c), l'acétal de 3,7-diméthyloct-2-énal ou l'acétal de 3,7-diméthylocta-2,6-diénal est hydrogéné de façon asymétrique et, après l'hydrogénation asymétrique, l'acétal hydrogéné est hydrolysé en cétone et produit le (R)-3,7-diméthyloctanal.

3. Procédé selon la revendication 1 ou 2 **caractérisé en ce que**, avant l'étape f), une étape fₒ) est conduite fₒ) formation d'un acétal de l'isomère de 6,10,14-triméthylpentadéc-5-én-2-one séparé dans l'étape e)
et que, dans l'étape f) le cétal de 6,10,14-triméthyl-pentadéc-5-én-2-one est hydrogéné de façon asymétrique et, après l'hydrogénation asymétrique, le cétal hydrogéné est hydrolysé en cétone et produit la (6R,10R)-6,10,14-triméthylpentadécan-2-one.

4. Procédé selon l'une quelconque des revendications précédentes **caractérisé en ce que** l'hydrogénation asymétrique dans l'étape c) et/ou dans l'étape f) est conduite en présence d'un additif qui est choisi dans le groupe constitué d'acides sulfoniques organiques, sels de métal de transition d'acides sulfoniques organiques, alcoxydes métalliques, aluminoxanes, alkylaluminoxanes et B(R)(_{3-V})(OZ)ᵥ ;
dans lequel v désigne 0, 1, 2 ou 3 et
R représente F, un groupe alkyle en C₁₋₆, alkyle en C₁₋₆ halogéné, aryle ou aryle halogéné ; et
Z représente un groupe alkyle en C₁₋₆, alkyle en C₁₋₆ halogéné, aryle ou aryle halogéné.

5. Procédé selon l'une quelconque des revendications précédentes **caractérisé en ce que**, dans l'étape d), la transformation chimique de (R)-3,7-diméthyloctanal en mélange de (R,E)-6,10,14-triméthylpentadéc-5-én-2-one et (R,Z)-6,10,14-triméthylpentadéc-5-én-2-one est effectuée par les étapes de
d1) réaction avec de l'acétone pour obtenir la (R)-6,10-diméthylundéc-3-én-2-one ;
d2) hydrogénation de (R)-6,10-diméthylundéc-3-én-2-one avec de l'hydrogène moléculaire en présence d'un catalyseur d'hydrogénation pour obtenir la (R)-6,10-diméthylundécan-2-one ;
suivies de
soit
d3) éthynylation de (R)-6,10-diméthylundécan-2-one en utilisant l'éthyne en présence d'une substance basique pour obtenir le (7R)-3,7,11-triméthyldodéc-1-yn-3-ol ;
d4) hydrogénation de (7R)-3,7,11-triméthyldodéc-1-yn-3-ol avec de l'hydrogène moléculaire en présence d'un catalyseur de Lindlar pour obtenir le (7R)-3,7,11-triméthyldodéc-1-én-3-ol ; ou
d3') vinylation de (R)-6,10-diméthylundécan-2-one par ajout d'un réactif de Grignard vinylique pour obtenir le (7R)-3,7,11-triméthyldodéc-1-én-3-ol ;
suivies de
d5) réaction de (7R)-3,7,11-triméthyldodéc-1-én-3-ol avec du 2-méthoxyprop-1-ène pour obtenir un mélange de (R,E)-6,10,14-triméthylpentadéc-5-én-2-one et (R,Z)-6,10,14-triméthylpentadéc-5-én-2-one ; ou
d5') réaction de (7R)-3,7,11-triméthyldodéc-1-én-3-ol avec un acétoacétate d'alkyle ou un dicétène en présence d'une base et/ou un acide pour obtenir un mélange de (R,E)-6,10,14-triméthylpentadéc-5-én-2-one et (R,Z)-6,10,14-triméthylpentadéc-5-én-2-one.

6. Procédé selon l'une quelconque des revendications précédentes **caractérisé en ce que** la séparation d'isomères dans l'étape b) et/ou e) est effectuée par distillation.

7. Procédé selon la revendication 6 **caractérisé en ce que** la distillation est effectuée en présence d'un catalyseur d'isomérisation cis/trans.

8. Procédé selon l'une quelconque des revendications précédentes 1 à 6 **caractérisé en ce que** l'isomère résiduel est isomérisé au moyen d'un catalyseur d'isomérisation cis/trans et ajouté au mélange d'isomères correspondant produit par les étapes a) et respectivement d) .

9. Procédé selon l'une quelconque des revendications précédentes **caractérisé en ce que** le complexe d'iridium chiral dans les étapes c) et/ou f) est un complexe d'iridium chiral de formule (III-0) 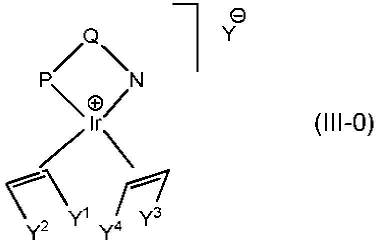 dans lequel
P-Q-N représente un ligand organique chélateur comprenant un centre stéréogénique ou présente une chiralité plane ou axiale et comporte un atome d'azote et de phosphore en tant que site de liaison au centre iridium du complexe ;
Y¹, Y², Y³ et Y⁴, indépendamment les uns des autres, sont des atomes d'hydrogène, un groupe alkyle en C₁₋₁₂, cycloalkyle en C₅₋₁₀ ou aromatique ; ou au moins deux d'entre eux forment conjointement au moins un groupe ponté divalent d'au moins 2 atomes de carbone ; et
Y^{⊖} est an anion, en particulier choisi dans le groupe constitué d'un halogénure, PF₆⁻, SbF₆⁻, tétra(3,5-bis(trifluorométhyl)phényl)borate (BAr_{F}⁻), BF₄⁻, des sulfonates perfluorés, de préférence F₃C-SO₃⁻ ou F₉C₄-SO₃⁻ ; ClO₄⁻, Al(OC₆F₅)₄⁻, Al(OC(CF₃)₃)₄⁻, N(SO₂CF₃)₂⁻, N(SO₂C₄F₉)₂⁻ et B(C₆F₅)₄⁻.

10. Procédé selon l'une quelconque des revendications précédentes 1 à 8 **caractérisé en ce que** le complexe d'iridium chiral dans l'étape c) et/ou f) est un complexe d'iridium chiral de formule (III) 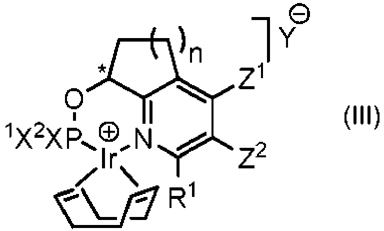 dans lequel
n est 1 ou 2 ou 3, de préférence 1 ou 2 ;
X¹ et X² sont, indépendamment l'un de l'autre, des atomes d'hydrogène, alkyle en C₁₋₄, cycloalkyle en C₅₋₇, adamantyle, phényle (facultativement substitué par un à trois groupes alkyle en C₁₋₅, alcoxy en C₁₋₄, perfluoroalkyle en C₁₋₄ et/ou un à cinq atomes d'halogène)), benzyle, 1-naphtyle, 2-naphtyle, 2-furyle ou ferrocényle ;
Z¹ et Z² sont, indépendamment l'un de l'autre, des atomes d'hydrogène, des groupes alkyle en C₁₋₅ ou alcoxy en C₁₋₅ ; ou Z¹ et Z² représentent conjointement un groupe de pontage formant un cycle de 5 ou 6 chaînons ;
Y^{⊖} est an anion, en particulier choisi dans le groupe constitué d'un halogénure, PF₆⁻, SbF₆⁻, tétra(3,5-bis(trifluorométhyl)phényl)borate (BAr_{F}⁻), BF₄⁻, des sulfonates perfluorés, de préférence F₃C-SO₃⁻ ou F₉C₄-SO₃⁻ ; ClO₄⁻, Al(OC₆F₅)₄⁻, Al(OC(CF₃)₃)₄⁻, N(SO₂CF₃)₂⁻, N(SO₂C₄F₉)₂⁻ et B(C₆F₅)₄⁻ ;
R¹ représente phényle ou o-tolyle ou m-tolyle ou p-tolyle ou un groupe de formule (IVa) ou (IVb) ou (IVc) 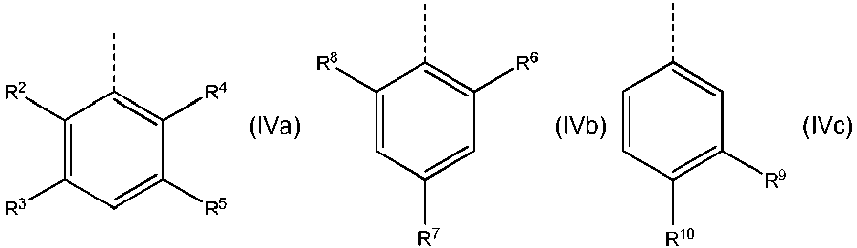
dans lequel R² et R³ représentent à la fois H ou un groupe alkyle en C₁-C₄ ou un groupe alkyle en C₁-C₄ halogéné ou représentent un groupe divalent formant conjointement un cycloaliphatique de 6 chaînons ou un cycle aromatique qui est facultativement substitué par des atomes d'halogène ou par des groupes alkyle en C₁-C₄ ou par des groupes alcoxy en C₁-C₄
R⁴ et R⁵ représentent à la fois H ou un groupe alkyle en C₁-C₄ ou un groupe alkyle en C₁-C₄ halogéné ou un groupe divalent formant conjointement un cycloaliphatique de 6 chaînons ou un cycle aromatique qui est facultativement substitué par des atomes d'halogène ou par des groupes alkyle en C₁-C₄ ou par des groupes alcoxy en C₁-C₄ ;
R⁶ et R⁷ et R⁸ représentent chacun un groupe alkyle en C₁-C₄ ou un groupe alkyle en C₁-C₄ halogéné ;
R⁹ et R¹⁰ représentent à la fois H ou un groupe alkyle en C₁-C₄ ou un groupe alkyle en C₁-C₄ halogéné ou un groupe divalent formant conjointement un cycloaliphatique à 6 chaînons ou un cycle aromatique qui est facultativement substitué par des atomes d'halogènes ou par des groupes alkyle en C₁-C₄ ou par des groupes alcoxy en C₁-C₄ ;
et dans lequel * représente un centre stéréogénique du complexe de formule (III).

11. Procédé selon la revendication 10 **caractérisé en ce que** le complexe d'iridium chiral de formule (III) utilisé dans l'étape c) et/ou f) utilisé pour l'hydrogénation asymétrique a la configuration S au centre stéréogénique indiqué par * dans le cas où le (E)-3,7-diméthyloct-2-énal ou le (E)-3,7-diméthylocta-2,6-diénal, ou des acétals de ceux-ci, ou la (R,E)-6,10,14-triméthylpentadéc-5-én-2-one, ou des cétals de celle-ci, doivent être hydrogénés ; ou a la configuration R au centre stéréogénique indiqué par * dans le cas où le (Z)-3,7-diméthyloct-2-énal ou le (Z)-3,7-diméthylocta-2,6-diénal, ou des acétals de ceux-ci, ou la (R,Z)-6,10,14-triméthylpentadéc-5-én-2-one, ou des cétals de celle-ci, doivent être hydrogénés.

12. Procédé de fabrication de (R,R)-isophytol ((3RS,7R,11R)-3,7,11,15-tétraméthylhexadéc-1-én-3-ol) comprenant
un procédé de fabrication de (6R,10R)-6,10,14-triméthylpentadécan-2-one comme décrit dans le procédé selon l'une quelconque des revendications précédentes 1 à 11 ;
suivi des étapes de
soit
g) éthynylation de (6R,10R)-6,10,14-triméthylpenta-décan-2-one en utilisant l'éthyne en présence d'une substance basique pour obtenir le (7R,11R)-3,7,11,15-tétraméthylhexadéc-1-yn-3-ol ;
h) hydrogénation de (7R,11R)-3,7,11,15-tétraméthyl-hexadéc-1-yn-3-ol avec de l'hydrogène moléculaire en présence d'un catalyseur de Lindlar pour obtenir le (R,R)-isophytol ;
ou
h') vinylation de (6R,10R)-6,10,14-triméthylpentadécan-2-one par ajout d'un réactif de Grignard vinylique pour obtenir le (R,R)-isophytol.

13. Procédé de fabrication de composé de formule (V) comprenant
un procédé de fabrication de (6R,10R)-6,10,14-triméthylpentadécan-2-one comme décrit dans le procédé selon l'une quelconque des revendications précédentes 1 à 11 ;
suivi des étapes de
soit
g) éthynylation de (6R,10R)-6,10,14-triméthyl-pentadécan-2-one en utilisant l'éthyne en présence d'une substance basique pour obtenir le (7R,11R)-3,7,11,15-tétraméthylhexadéc-1-yn-3-ol ;
h) hydrogénation de (7R,11R)-3,7,11,15-tétraméthyl-hexadéc-1-yn-3-ol avec de l'hydrogène moléculaire en présence d'un catalyseur de Lindlar pour obtenir le (R,R)-isophytol ;
ou
h') vinylation de (6R,10R)-6,10,14-triméthylpentadécan-2-one par ajout d'un réactif de Grignard vinylique pour obtenir le (R,R)-isophytol ; suivies des étapes de
m) condensation de (R,R)-isophytol avec un composé de formule (VI) pour obtenir un composé de formule (V) étant un mélange d'isomères compte tenu de la chiralité au centre indiqué par # ; 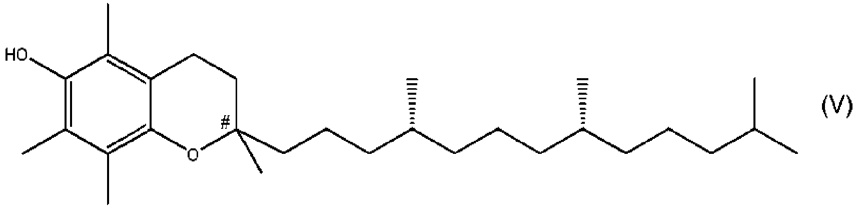 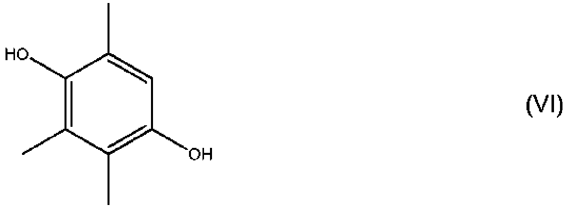 dans lequel # représente un centre stéréogénique.

14. Procédé de fabrication de composé de formule (V-A) comprenant
un procédé de fabrication de (6R,10R)-6,10,14-triméthylpentadécan-2-one comme décrit dans le procédé selon l'une quelconque des revendications précédentes 1 à 11 ;
suivi des étapes de
soit
g) éthynylation de (6R,10R)-6,10,14-triméthyl-pentadécan-2-one en utilisant l'éthyne en présence d'une substance basique pour obtenir le (7R,11R)-3,7,11,15-tétraméthylhexadéc-1-yn-3-ol ;
h) hydrogénation de (7R,11R)-3,7,11,15-tétraméthyl-hexadéc-1-yn-3-ol avec de l'hydrogène moléculaire en présence d'un catalyseur de Lindlar pour obtenir le (R,R)-isophytol ;
ou
h') vinylation de (6R,10R)-6,10,14-triméthylpentadécan-2-one par ajout d'un réactif de Grignard vinylique pour obtenir le (R,R)-isophytol ;
suivies des étapes de
m) condensation de (R,R)-isophytol avec un composé de formule (VI) pour obtenir un composé de formule (V) étant un mélange d'isomères compte tenu de la chiralité au centre indiqué par # ; 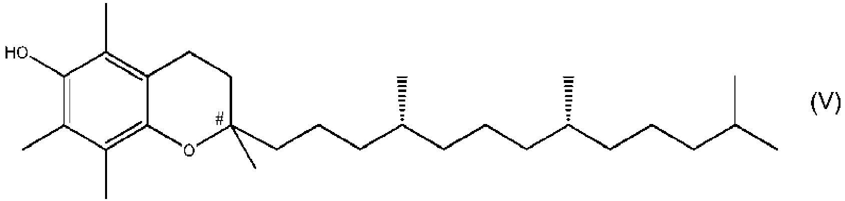 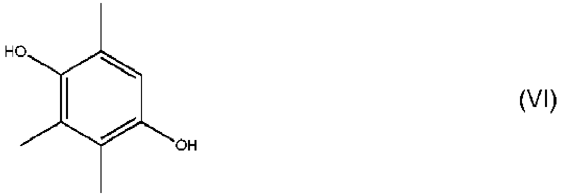 dans lequel # représente un centre chiral stéréogénique ; et
n) isolement d'un composé de formule (V-A) à partir du mélange d'isomères de formule (V)

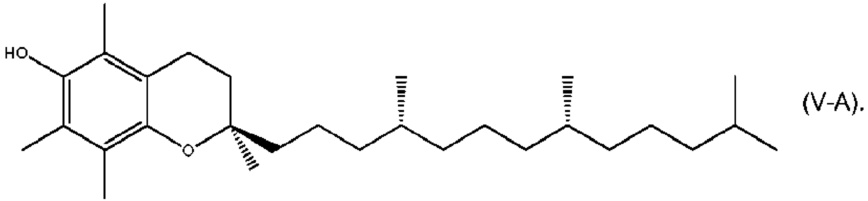

15. Composition comprenant
- au moins un acétal ou cétal de formule (XI) ou (XII) et
- au moins un complexe d'iridium chiral 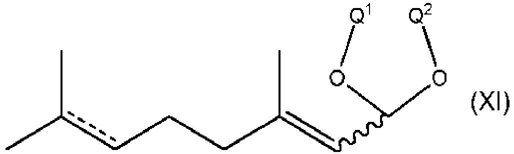 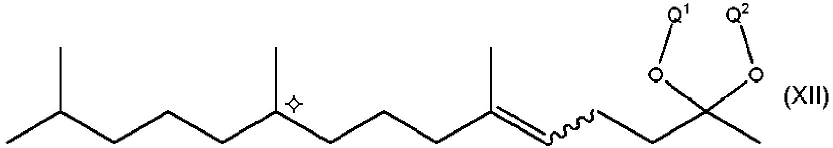
dans lequel un trait ondulé représente une liaison carbone-carbone qui est liée à la double liaison carbone-carbone adjacente de sorte que ladite double liaison carbone-carbone soit dans la configuration Z ou E ;
et dans lequel la double liaison ayant des traits pointillés (-----) dans la formule représente une simple liaison carbone-carbone ou une double liaison carbone-carbone ;
et dans lequel ✧ représente un centre stéréogénique qui a la configuration R ;
et dans lequel
Q¹ et Q²
représentent, individuellement ou conjointement, un groupe alkyle en C₁-C₁₀ ou un groupe alkyle en C₁-C₁₀ halogéné ;
ou forment conjointement un groupe alkylène en C₂-C₆ ou un groupe cycloalkylène en C₆-C₈.

16. Acétal ou cétal de formule (XX-A) ou (XX-B) ou (XX-C) ou (XX-D) ou (XX-E) 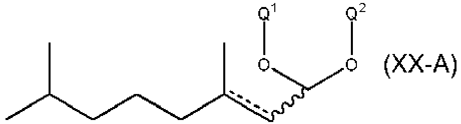 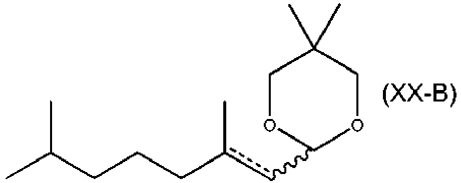 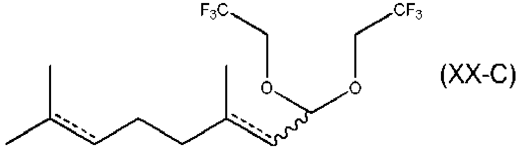 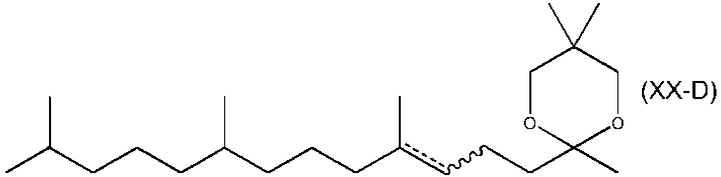 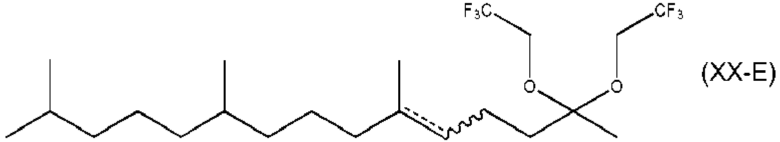
dans lequel la double liaison ayant des traits pointillés (-----) dans les formules ci-dessus représente une simple liaison carbone-carbone ou une double liaison carbone-carbone ; et
dans lequel un trait ondulé représente une liaison carbone-carbone qui est liée à la simple liaison carbone-carbone adjacente (----- représentant —) ou à une double liaison carbone-carbone adjacente (----- représentant 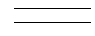 ) de sorte que ladite double liaison carbone-carbone soit dans la configuration Z ou E,
et dans lequel
Q¹ et Q²
représentent, individuellement ou conjointement, un groupe alkyle en C₁-C₁₀ ou un groupe alkyle en C₁-C₁₀ halogéné ;
ou forment conjointement un groupe alkylène en C₂-C₆ ou un groupe cycloalkylène en C₆-C₈ ;
à l'exception de 1,1-diméthoxy-3,7-diméthyloct-2-ène ; et dans lequel
l'acétal ou cétal de formule (XX-D) ou (XX-E) est (R)-2,5,5-triméthyl-2-(4,8,12-triméthyltridéc-3-én-1-yl)-1,3-dioxane
ou
(R)-6,10,14-triméthyl-2,2-bis(2,2,2-trifluoroéthoxy)pentadéc-5-ène
ou
2,5,5-triméthyl-2-((4R,8R)-4,8,12-triméthyltridécyl)-1,3-dioxane
ou
(6R,10R)-6,10,14-triméthyl-2,2-bis(2,2,2-trifluoroéthoxy)pentadécane.
